# EUROPÄISCHE PATENTSCHRIFT

(11) **EP 2 794 602 B1**
(45) Veröffentlichungstag und Bekanntmachung des Hinweises auf die Patenterteilung: **29.11.2017**
(21) Anmeldenummer: 12780423.5
(22) Anmeldetag: 30.10.2012
(51) Int. Cl.: C07D 471/04, A61K 31/437, A61P 35/00

(54) **3-CYANARYL-1H-PYRAZOLO-(2,3-B-)PYRIDINDERIVATE**
3-CYANARYL-1H-PYRAZOLO(2.3-B) PYRIDINE DERIVATIVES
DÉRIVÉS 3-CYANARYL-1H-PYRROLO[2,3-B]PYRIDINE

(30) Priorität: 22.11.2011 DE 102011119127
(43) Veröffentlichungstag der Anmeldung: 29.10.2014
(73) Patentinhaber: Merck Patent GmbH, 64293 Darmstadt (DE)
(72) Erfinder: DORSCH, Dieter, 64372 Ober-Ramstadt (DE); HOELZEMANN, Guenter, 64342 Seeheim-Jugenheim (DE); EGGENWEILER, Hans-Michael, 64291 Darmstadt (DE); CZODROWSKI, Paul, 61169 Friedberg (DE)
(86) Internationale Anmeldenummer: PCT/EP2012/004543
(87) Internationale Veröffentlichungsnummer: WO 2013/075785

(56) Entgegenhaltungen:
- WO-A1-2005/095400
- WO-A1-2006/015123
- WO-A1-2008/124848

## Beschreibung

### Hintergrund der Erfindung

Es war die Aufgabe der Erfindung, neue Verbindungen mit wertvollen Eigenschaften aufzufinden, insbesondere solche, die für die Herstellung von Arzneimitteln verwendet werden können.
Die vorliegende Erfindung betrifft Pyridinverbindungen, die in der Lage sind, eine oder mehrere Kinasen zu hemmen. Die Verbindungen finden Verwendung in der Behandlung einer Vielzahl von Störungen, darunter Krebs, septischer Schock, primäres Offenwinkelglaukom (Primary open Angle Glaucoma - POAG), Hyperplasie, rheumatoide Arthritis, Psoriasis, Atherosklerose, Retinopathie, Osteoarthritis, Endometriose, chronische Entzündung und/oder neurodegenerative Erkrankungen wie Morbus Alzheimer.

Die vorliegende Erfindung betrifft Verbindungen und die Verwendung von Verbindungen, bei denen die Hemmung, Regulierung und/oder Modulation der Signaltransduktion von Rezeptorkinasen eine Rolle spielt, ferner pharmazeutische Zusammensetzungen, die diese Verbindungen enthalten, sowie die Verwendung der Verbindungen zur Behandlung kinasebedingter Krankheiten.
Da Proteinkinasen nahezu jeden Zellprozeß, darunter Metabolismus, Zellproliferation, Zelldifferenzierung und Zellüberleben, regulieren, stellen sie attraktive Ziele für therapeutische Eingriffe bei verschiedenen Krankheitszuständen dar. Beispielsweise sind Zellzyklussteuerung und Angiogenese, in denen Proteinkinasen eine Schlüsselrolle spielen, Zellvorgänge, die mit zahlreichen Krankheitszuständen wie Krebs, Entzündungskrankheiten, abnormale Angiogenese und damit in Zusammenhang stehende Krankheiten, Atherosklerose, Makula-Degeneration, Diabetes, Fettsucht und Schmerz einhergehen, ohne hierauf beschränkt zu sein.

Die vorliegende Erfindung betrifft insbesondere Verbindungen und die Verwendung von Verbindungen, bei denen die Hemmung, Regulierung und/oder Modulation der Signaltransduktion von TBK1 und IKKε eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

IKKε und TBK1 sind Serin/Threonin Kinasen die hohe Homologien untereinander sowie zu anderen IkB-Kinasen aufweisen. Beide Kinasen spielen eine integrale Rolle für das angeborene, immanente Immunsystem. Doppelsträngige RNA-Viren werden durch die Toll-like Rezeptoren 3 und 4, sowie die RNA-Helicasen RIG-I und MDA-5 erkannt und führen zu einer Aktivierung derTRIF-TBK1/IKKε-IRF3 Signalkaskade, was zu einer Typ I Interferon-Antwort führt.

Boehm und Kollegen beschrieben 2007 IKKε als ein neuartiges Brustkrebsonkogen [J.S. Boehm et al., Cell 129, 1065-1079, 2007]. 354 Kinasen wurden auf Ihre Fähigkeit hin untersucht gemeinschaftlich mit einer aktivierten Form der MAPK Kinase Mek, den Ras-transformierenden Phänotyp zu rekapitulieren. IKKε wurde hierbei als ein kooperatives Onkogen identifiziert. Darüber hinaus konnten die Autoren zeigen, dass IKKε in zahlreichen Brustkrebszelllinien und Tumorproben amplifiziert und überexprimiert vorliegt. Die Verminderung der Genexpression mittels RNA interference in Brustkrebszellen induziert Apoptosis und beeinträchtigt deren Proliferation. Eddy und Kollegen kamen 2005 zu ähnlichen Befunden, was die Bedeutung von IKKε in Brustkrebserkrankungen unterstreicht [S.F.Eddy et al., Cancer Res. 2005; 65 (24), 11375-11383].

Über einen protumorigenen Effekt von TBK1 wurde erstmals 2006 berichtet. Korherr und Kollegen identifizierten in einem Screening einer 251000 cDNA umfassenden Genbibliothek mit TRIF, TBK1 und IRF3 gleich drei Gene, die typischerweise in der angeborenen Immunabwehr involviert sind, als proangiogene Faktoren [C.Korherr et al., PNAS, 103, 4240-4245, 2006]. Chien und Kollegen publizierten 2006 [Y.Chien et al., Cell 127, 157-170, 2006], dass TBK1-/- Zellen nur bedingt mit oncogenem Ras transformierbar sind, was eine Involvierung von TBK1 bei der Rasvermittelten Transformation nahelegt. Desweiteren konnten sie zeigen, dass ein RNAi vermittelter knock down von TBK1 Apoptose in MCF-7 und Panc-1 Zellen auslöst. Kürzlich publizierten Barbie und Kollegen, dass TBK1 in zahlreichen Krebszellinien mit mutierten K-Ras von essentieller Bedeutung ist, was nahelegt, dass eine TBK1 Intervention in entsprechenden Tumoren von therapeutischer Bedeutung sein könnte [D.A.Barbie et al., Nature Letters 1-5, 2009].

Durch Proteinkinasen hervorgerufene Erkrankungen sind durch eine anomale Aktivität oder Hyperaktivität solcher Proteinkinasen gekenn zeichnet. Anomale Aktivität betrifft entweder: (1) die Expression in Zellen, die gewöhnlich diese Proteinkinasen nicht exprimieren; (2) erhöhte Kinasen-Expression, die zu unerwünschter Zellproliferation, wie Krebs, führt; (3) erhöhte Kinasen-Aktivität, die zu unerwünschter Zellproliferation, wie Krebs, und/oder zu Hyperaktivität der entsprechenden Proteinkinasen führt. Hyperaktivität bezieht sich entweder auf eine Amplifikation des Gens, das eine bestimmte Proteinkinase codiert, oder die Erzeugung eines Aktivitäts-Spiegels, der mit einer Zellproliferationserkrankung korreliert werden kann (d.h. mit steigendem Kinase-Spiegel steigt die Schwere eines oder mehrerer Symptome der Zellproliferationserkrankung) die biologische Verfügbarkeit einer Proteinkinase kann auch durch das Vorhandensein oder Fehlen eines Satzes von Bindungsproteinen dieser Kinase beeinflusst werden.

IKKε und TBK1 sind hochgradig homologe Ser/Thr-Kinasen, die durch Induktion von Typ-1-Interferonen und anderen Zytokinen eine ausschlaggebende Rolle bei der angeborenen Immunantwort spielen. Diese Kinasen werden als Antwort auf eine virale/bakterielle Infektion stimuliert. Zur Immunantwort auf virale und bakterielle Infektionen gehört die Bindung von Antigenen wie bakteriellem Lipopolysaccharid (LPS), viraler doppelsträngiger RNA (dsRNA) an Toll-like Rezeptoren, daran anschließend Aktivierung des TBK1-Weges. Aktiviertes TBK1 und IKKε phosphorylieren IRF3 und IRF7, was die Dimerisierung und Kerntranslokation dieser Interferon regulierenden Transkriptionsfaktoren auslöst, was letztendlich eine Signalkaskade induziert, die zur IFN-Produktion führt.

Kürzlich wurden IKKε und TBK1 auch mit Krebs in Zusammenhang gebracht. Es wurde gezeigt, daß IKKε mit aktiviertem MEK zur Transformation menschlicher Zellen kooperiert. Außerdem wird IKKε häufig in Brustkrebs-Zellinien und von Patienten stammenden Tumoren amplifiziert/überexprimiert. TBK1 wird unter hypoxischen Bedingungen induziert und in vielen soliden Tumoren in signifikanter Höhe exprimiert. Des weiteren ist TBK1 erforderlich, um onkogene Ras-Transformation zu unterstützen, und TBK1-Kinaseaktivität ist in transformierten Zellen erhöht und für ihr Überleben in Kultur erforderlich. Ebenso wurde gefunden, daß TBK1- und NF-kB-Signalgebung in KRAS-mutierten Tumoren wesentlich sind. TBK1 wurde als ein synthetischer letaler Partner des onkogenen KRAS identifiziert.
Lit.:
Y.-H.Ou et al., Molecular Cell 41, 458-470, 2011;
D.A. Barbie et al., Nature, 1-5, 2009.

In der WO 2011/046970 A1 wird die Verwendung von TBK1- und/oder IKKε - Inhibitoren zur Behandlung von verschiedenen Krankheiten beschrieben, wie rheumatoide Arthritis (RA), systemischer Lupus erythematosus (SLE), Sjörgrens Syndrom, Aicardi-Goutieres Syndrom Lupus Chilblain, retinale Vasculopathie und cerebrale Leukodystrophie (RVCL), systemische Sclerosis, Myositis, Psoriasis, chronisch obstruktive pulmonare Krankheit (CPD), endzündliche Darmkrankheit (IBD), Fettsucht, Insulinresistenz, Typ 2 Diabetes (NIDDM), metabolisches Syndrom, Krebserkrankungen,

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation/ Vitalität wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, Zelltod zu induzieren oder Zellproliferation, Zellvitalität oder Migration zu inhibieren, gewöhnlich zwischen ungefähr einer Stunde und einer Woche. Zum Testen in vitro können kultivierte Zellen aus einer Biopsieprobe verwendet werden. Die Menge nach der Behandlung zurückbleibenden Zellen werden dann bestimmt.
Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Zudem können die erfindungsgemäßen Verbindungen verwendet werden, um bei gewissen existierenden Krebs-Chemotherapien und -bestrahlungen additive oder synergistische Effekte zu erzielen und/oder, um die Wirksamkeit gewisser existierender Krebs-Chemotherapien und -bestrahlungen wiederherzustellen.

Der Ausdruck "Methode" bezeichnet Arbeitsweisen, Mittel, Techniken und Prozeduren, um eine gegebene Aufgabe zu erfüllen, darunter diejenigen Arbeitsweisen, Mittel, Techniken und Prozeduren, die dem Fachmann auf chemischem, pharmakologischem, biologischem, biochemischem und medizinischem Gebiet entweder bekannt sind oder von ihm leicht aus bekannten Arbeitsweisen, Mitteln, Techniken und Prozeduren entwickelt werden können, ohne jedoch hierauf beschränkt zu sein.

Der Ausdruck "Verabreichung", wie er hier verwendet wird, bezeichnet eine Methode, um eine Verbindung der vorliegenden Erfindung und eine Zielkinase so zusammenzubringen, daß die Verbindung die Enzymaktivität der Kinase entweder direkt, d.h. durch Wechselwirkung mit der Kinase selber, oder indirekt, d.h. durch Wechselwirkung mit einem anderen Molekül, von dem die katalytische Aktivität der Kinase abhängt, beeinflussen kann. Wie hier verwendet, kann Verabreichung entweder in vitro, d.h. im Reagenzglas, oder in vivo, d.h. in Zellen oder Geweben eines lebenden Organismus, erfolgen.

Der Ausdruck "Behandeln" umfaßt hier Außerkraftsetzen, weitgehendes Hemmen, Verlangsamen oder Umkehren des Fortschreitens einer Krankheit oder Störung, weitgehendes Verbessern der klinischen Symptome einer Krankheit oder Störung oder weitgehendes Verhindern des Auftretens der klinischen Symptome einer Krankheit oder Störung.

Der Ausdruck "Verhindern" bezeichnet hier eine Methode, um einen Organismus dagegen zu blockieren, daß er eine Störung oder Krankheit überhaupt erwirbt.

Für eine beliebige in dieser Erfindung verwendete Verbindung kann eine therapeutisch wirksame Menge, die hier auch als therapeutisch wirksame Dosis bezeichnet wird, kann zunächst anhand von Zellkultur-Assays berechnet werden. Beispielsweise kann in Tiermodellen eine Dosis formuliert werden, um einen Kreislaufkonzentrationsbereich zu erreichen, der die IC50 oder die IC100 umfaßt, wie sie in Zellkulturen ermittelt wurden. Diese Information kann verwendet werden, um brauchbare Dosen für den Menschen genauer zu bestimmen. Anfangsdosierungen können auch aus In-vivo-Daten berechnet werden. Anhand dieser Anfangsrichtlinien könnte ein Durchschnittsfachmann eine wirksame Dosierung für den Menschen bestimmen.

Außerdem können die Toxizität und die therapeutische Wirksamkeit der hier beschriebenen Verbindungen nach pharmazeutischen Standardprozeduren an Zellkulturen oder Versuchstieren bestimmt werden, indem man z.B. die LD50 und die ED50 bestimmt. Das Dosisverhältnis zwischen toxischer und therapeutischer Wirkung ist der therapeutische Index, der als das Verhältnis zwischen LD50 und ED50 ausgedrückt werden kann. Verbindungen, die einen hohen therapeutischen Index aufweisen, sind bevorzugt. Die aus diesen Zellkultur-Assays und Tierversuchen erhaltenen Daten können verwendet werden, um einen Dosierungsbereich zu formulieren, der für die Verwendung am Menschen nicht toxisch ist. Die Dosierung solcher Verbindungen liegt vorzugsweise in Konzentrationsbereichen im Blutkreislauf, die die ED50 mit geringer oder keiner Toxizität umfassen. Innerhalb dieses Bereiches kann die Dosierung in Abhängigkeit von der eingesetzten Dosierungsform und dem verwendeten Verabreichungsweg variieren. Die genaue Formulierung, der Verabreichungsweg und die Dosierung können vom einzelnen Arzt unter Berücksichtigung des Zustandes des Patienten gewählt werden (siehe z.B. Fingl et al., 1975, in: The Pharmacological Basis of Therapeutics, Kapitel 1, Seite 1).

Dosierungsmenge und -interval können individuell eingestellt werden, um für Plasmaspiegel der Wirkverbindung zu sorgen, die ausreichen, um eine therapeutische Wirkung zu erhalten. Übliche Patientendosierungen für orale Verabreichung liegen im Bereich von etwa 50-2000 mg/kg/Tag, im allgemeinen von etwa 100-1000 mg/kg/Tag, vorzugsweise von etwa 150-700 mg/kg/Tag und insbesondere bevorzugt von etwa 250-500 mg/kg/Tag.

Vorzugsweise erreicht man therapeutisch wirksame Serumspiegel durch Verabreichen mehrerer Dosen pro Tag. Bei lokaler Verabreichung oder selektiver Aufnahme steht die wirksame lokale Konzentration des Medikamentes möglicherweise nicht mit der Plasmakonzentration in Beziehung. Der Fachmann wird in der Lage sein, therapeutisch wirksame lokale Dosierungen ohne übermäßiges Experimentieren zu optimieren.

Bevorzugte Krankheiten oder Störungen, für deren Vorbeugung, Behandlung und/oder Untersuchung die hier beschriebenen Verbindungen brauchbar sein können, sind zellproliferative Störungen, insbesondere Krebs, wie Papillom, Blastogliom, Kaposi-Sarkom, Melanom, Lungenkrebs, Eierstockkrebs, Prostatakrebs, Plattenepithelkarzinom, Astrozytom, Kopfkrebs, Halskrebs, Hautkrebs, Leberkrebs, Blasenkrebs, Brustkrebs, Lungenkrebs, Gebärmutterkrebs, Prostatakrebs, Hodenkarzinom, Kolorektalkrebs, Schilddrüsenkrebs, Bauchspeicheldrüsenkrebs, Magenkrebs, hepatozelluläres Karzinom, Leukämie, Lymphom, Morbus Hodgkin und Burkitt-Krankheit, ohne hierauf beschränkt zu sein.

### STAND DER TECHNIK

Andere Benzonitrilderivate sind in der WO 2011/046970 A1 als TBK1- und/oder IKKε-Inhibitoren beschrieben.

In der WO 2005/095400 sind andere Azaindol-Kinase-Inhibitoren beschrieben.

In der WO2006/015123 sind andere Pyrrolo-pyridin-Kinase-Modulatoren beschrieben.

Weitere heterocyclische Derivate und deren Verwendung als Antitumormittel wurden in der WO 2007/129044 beschrieben.

Weitere Pyridin- und Pyrazinderivate wurden in der Verwendung für die Behandlung von Krebs in der WO 2009/053737 und für die Behandlung von anderen Krankheiten in der WO 2004/055005 beschrieben.

Weitere heterocyclische Derivate wurden als IKKε-Hemmer in der WO 2009/122180 offenbart.

Pyrrolopyrimidine wurden als IKKε- und TBK1-Hemmer in der WO 2010/100431 beschrieben.

Pyrimidinderivate wurden als IKKε- und TBK1-Hemmer in der WO 2009/030890 beschrieben.

### ZUSAMMENFASSUNG DER ERFINDUNG

Gegenstand der Erfindung sind die Verbindungen "A4" und "A6" bis "A38" (erfindungsgemäße Verbindungen).

Es werden die Verbindungen der Formel I beschirieben: 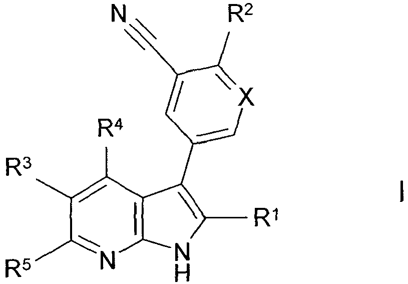 worin
- X: CH oder N,
- R¹: H, A oder Cyc,
- R²: O[C(R⁶)₂]ₙHet¹, NR⁶[C(R⁶)2]ₙHet¹, O[C(R⁶)₂]ₙCyc oder NR⁶[C(R⁶)₂]ₙCyc,
- R³: H, Hal, A, OR⁶, N(R⁶)₂, O[C(R⁶)₂]ₘN(R⁶)₂, O[C(R⁶)₂]ₙHet², NR⁶[C(R⁶)₂]ₘN(R⁶)₂, NR⁶[C(R⁶)₂]ₙHet², Ar oder Het²,
- R⁴: H oder OR⁶,
- R⁵: H oder OR⁶,
- R⁶: H oder unverzweigtes oder verzweigtes Alkyl mit 1-6 C-Atomen, worin 1-7 H-Atome durch F ersetzt sein können,
- Het¹: unsubstituiertes oder einfach durch Hal, CN, OH, OA, COOA, CONH₂, S(O)ₙA, S(O)ₙAr, COA, A oder =O substituiertes Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl oder Piperazinyl,
- Het²: unsubstituiertes oder ein- oder zweifach durch durch Hal, A, [C(R⁶)₂]ₚOR⁶, [C(R⁶)₂]ₚN(R⁶)₂, [C(R⁶)₂]ₚHet¹, NO₂, CN, [C(R⁶)₂]ₚCOOR⁶, CON(R⁶)₂, NR⁶COA, NR⁶SO₂A, SO₂N(R⁶)₂, S(O)ₙA, COHet¹, O[C(R⁶)₂]ₚN(R⁶)₂, O[C(R⁶)₂]ₚHet¹, NHCOOA, NHCON(R⁶)₂, NHCOO[C(R⁶)₂]ₚN(R⁶)₂, NHCOO[C(R⁶)₂]ₚHet¹, NHCONH[C(R⁶)₂]ₚN(R⁶)₂, NHCONH[C(R⁶)₂]ₚHet¹, OCONH[C(R⁶)₂]ₚN(R⁶)₂, OCONH[C(R⁶)₂]ₚHet¹, CHO, COA, =S, =NR⁶ und/oder =O substituiertes Dihydropyrrolyl, Pyrrolidinyl, Azetidinyl, Tetrahydroimidazolyl, Tetrahydrofuranyl, Dihydropyrazolyl, Tetrahydropyrazolyl, Dihydropyridyl, Tetrahydropyridyl, Dihydropyranyl, Tetrahydropyranyl, Piperidinyl, Morpholinyl, Hexahydropyridazinyl, Hexahydropyrimidinyl, [1,3]Dioxolanyl, Piperazinyl, Furyl, Thienyl, Pyrrolyl, Imidazolyl, Pyrazolyl, Oxazolyl, Isoxazolyl, Thiazolyl, Triazolyl, Tetrazolyl, Pyridyl, Pyrimidyl, Pyridazinyl, Indolyl, Isoindolyl, Benzimidazolyl, Indazolyl, Chinolyl, 1,3-Benzodioxolyl, Benzothiophenyl, Benzofuranyl, Imidazopyridyl oder Furo[3,2-b]pyridyl,
- Ar: unsubstituiertes oder ein-, zwei- oder dreifach durch Hal, A, [C(R⁶)₂]ₚOR⁶, [C(R⁶)₂]ₚN(R⁶)₂, [C(R⁶)₂]ₚHet¹, NO₂, CN, [C(R⁶)₂]ₚCOOR⁶, CON(R⁶)₂, NR⁶COA, NR⁶SO₂A, SO₂N(R⁶)₂, S(O)ₙA, COHet¹, O[C(R⁶)₂]ₚN(R⁶)₂, O[C(R⁶)₂]ₚHet¹, NHCOOA, NHCON(R⁶)₂, NHCOO[C(R⁶)₂]ₚN(R⁶)₂, NHCOO[C(R⁶)₂]ₚHet¹, NHCONH[C(R⁶)₂]ₚN(R⁶)₂, NHCONH[C(R⁶)₂]ₚHet¹, OCONH[C(R⁶)₂]ₚN(R⁶)₂, OCO-H[C(R⁶)₂]ₚHet¹, CHO und/oder COA substituiertes Phenyl oder Naphthyl,
- A: unverzweigtes oder verzweigtes Alkyl mit 1-10 C-Atomen, worin eine oder zwei nicht benachbarte CH- und/oder CH₂-Gruppen durch N-, O- und/oder S-Atomen ersetzt sein können und/oder auch 1-7 H-Atome durch F und/oder Cl ersetzt sein können,
- Cyc: unsubstituiertes oder einfach durch CN oder A substituiertes cyclisches Alkyl mit 3, 4, 5, 6 oder 7 C-Atomen,
- Hal: F, Cl, Br oder I,
- m: 1,2 oder 3,
- n: 0, 1 oder 2,
- p: 0, 1, 2, 3 oder 4
bedeuten,
sowie ihre pharmazeutisch verwendbaren Salze, Tautomere und Stereoisomere, einschließlich deren Mischungen in allen Verhältnissen.

Gegenstand der Erfindung sind auch die optisch aktiven Formen (Stereoisomeren), Salze, die Enantiomeren, die Racemate, die Diastereomeren sowie die Hydrate und Solvate der erfindungsgemäßen Verbindungen. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Gegenstand der Erfindung sind natürlich auch die Solvate der Salze.

Unter pharmazeutisch verwendbaren Derivaten versteht man z.B. die Salze der erfindungsgemäßen Verbindungen als auch sogenannte Prodrug-Verbindungen. Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden. Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen. Beschrieben werden die Verbindungen der Formel I und ihre Salze sowie ein Verfahren zur Herstellung von Verbindungen der Formel I sowie ihrer pharmazeutisch verwendbaren Salze, Tautomere und Stereoisomere, dadurch gekennzeichnet, daß man
a) eine Verbindung der Formel II 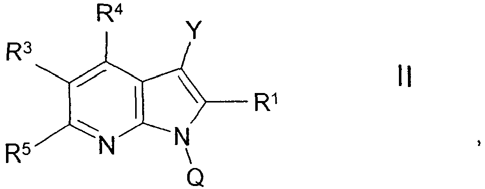
   worin R¹, R³, R⁴, R⁵ die in Anspruch 1 angegebenen Bedeutungen haben,
   Y Br oder I und
   Q eine Schutzgruppe bedeutet,
   mit einer Verbindung der Formel III 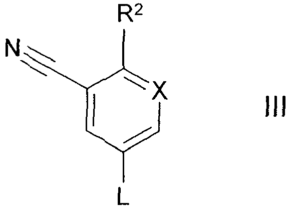
   worin X und R² die in Anspruch 1 angegebene Bedeutung hat und
   L einen Boronsäurerest oder eine Boronsäureestergruppe bedeutet, umsetzt,
   und anschließend Q abspaltet,
   oder
b) daß man sie aus einem ihrer funktionellen Derivate durch Behandeln mit einem solvolysierenden oder hydrogenolysierenden Mittel in Freiheit setzt,
und/oder eine Base oder Säure der Formel I in eines ihrer Salze umwandelt.

Vor- und nachstehend haben die Reste R¹, R², R³, R⁴, R⁵ und X die bei der Formel I angegebenen Bedeutungen, sofern nicht ausdrücklich etwas anderes angegeben ist.

A bedeutet Alkyl, ist unverzweigt (linear) oder verzweigt und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
In A können auch eine oder zwei CH- und/oder CH₂-Gruppen durch N, O- oder S-Atome ersetzt sein. So bedeutet A z.B. auch 2-Methoxyethyl.
Besonders bevorzugt bedeutet A unverzweigtes oder verzweigtes Alkyl mit 1-8 C-Atomen, worin eine oder zwei nicht benachbarte CH- und/oder CH₂-Gruppen durch N- und/oder O-Atome ersetzt sein können und/oder auch 1-7 H-Atome durch F ersetzt sein können.
Cycloalkyl (cyclisches Alkyl) bedeutet Cyclopropyl, Cyclobutyl, Cyclopentyl, Cyclohexyl oder Cycloheptyl.

R¹ bedeutet vorzugsweise H oder A.

R² bedeutet vorzugsweise O[C(R⁶)₂]ₙHet¹ oder O[C(R⁶)₂]ₙCyc.
R³ bedeutet vorzugsweise H, Hal, O[C(R⁶)₂]ₙHet², Ar oder Het².
R⁴ bedeutet besonders bevorzugt H.
R⁵ bedeutet besonders bevorzugt H.
R⁶ bedeutet vorzugsweise H oder Methyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-lodphenyl, 4-Fluor-3-chlorphenyl, 2-Fluor-4-bromphenyl, 2,5-Difluor-4-bromphenyl oder 2,5-Dimethyl-4-chlorphenyl. Ar bedeutet besonders bevorzugt unsubstituiertes oder einfach durch [C(R⁶)₂]ₚHet¹ oder CN substituiertes Phenyl oder Naphthyl.

Het¹ bedeutet vorzugsweise unsubstituiertes oder einfach durch S(O)ₙA, S(O)ₙAr oder A substituiertes Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl oder Piperazinyl.

Het¹ bedeutet besonders bevorzugt unsubstituiertes oder einfach durch S(O)ₙA, S(O)ₙAr oder A substituiertes Pyrrolidinyl, Oxetanyl, Piperidinyl, Morpholinyl, Tetrahydropyranyl oder Piperazinyl.

Het² bedeutet vorzugsweise unsubstituiertes oder einfach durch durch A, [C(R⁶)₂]ₚOR⁶ oder [C(R⁶)₂]ₚHet¹ substituiertes Dihydropyrrolyl, Pyrrolidinyl, Azetidinyl, Tetrahydroimidazolyl, Tetrahydrofuranyl, Dihydropyrazolyl, Tetrahydropyrazolyl, Dihydropyridyl, Tetrahydropyridyl, Dihydropyranyl, Tetrahydropyranyl, Piperidinyl, Morpholinyl, Hexahydropyridazinyl, Hexahydropyrimidinyl, [1,3]Dioxolanyl, Piperazinyl, Furyl, Thienyl, Pyrrolyl, Imidazolyl, Pyrazolyl, Oxazolyl, Isoxazolyl, Thiazolyl, Triazolyl, Tetrazolyl, Pyridyl, Pyrimidyl, Pyridazinyl, Indolyl, Isoindolyl, Benzimidazolyl, Indazolyl, Chinolyl, 1,3-Benzodioxolyl, Benzothiophenyl, Benzofuranyl, Imidazopyridyl oder Furo[3,2-b]pyridyl.

Het² bedeutet besonders bevorzugt unsubstituiertes oder einfach durch durch A, [C(R⁶)₂]ₚOR⁶ oder [C(R⁶)₂]ₚHet¹ substituiertes Piperidinyl, Morpholinyl, Piperazinyl, Pyrazolyl oder Triazolyl.

Hal bedeutet vorzugsweise F, Cl oder Br, aber auch I, besonders bevorzugt F oder Cl. Sämtliche Reste, die mehrfach auftreten, gleich oder verschieden sein können, d.h. unabhängig voneinander sind.
Die Verbindungen der Formel I können ein oder mehrere chirale Zentren besitzen und daher in verschiedenen stereoisomeren Formen vorkommen. Die Formel I umschließt alle diese Formen. Beschrieben werden insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ig ausgedrückt werden, die der Formel I entsprechen und worin die nicht näher bezeichneten Reste die bei der Formel I angegebene Bedeutung haben, worin jedoch

| | | |
|---|---|---|
| in Ia | R² | O[C(R⁶)₂]ₙHet¹ oder O[C(R⁶)₂]ₙCyc; |
| | | |
| in Ib | R³ | H, Hal, O[C(R⁶)₂]ₙHet², Ar oder Het²; |
| | | |
| in Ic | Het¹ | unsubstituiertes oder einfach durch S(O)ₙA, S(O)ₙAr oder A substituiertes Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl oder Piperazinyl; |
| | | |
| in Id | Het² | unsubstituiertes oder einfach durch durch A, [C(R⁶)₂]ₚOR⁶ oder [C(R⁶)₂]ₚHet₁ substituiertes Dihydropyrrolyl, Pyrrolidinyl, Azetidinyl, Tetrahydroimidazolyl, Tetrahydrofuranyl, Dihydropyrazolyl, Tetrahydropyrazolyl, Dihydropyridyl, Tetrahydropyridyl, Dihydropyranyl, Tetrahydropyranyl, Piperidinyl, Morpholinyl, Hexahydropyridazinyl, Hexahydropyrimidinyl, [1,3]Dioxolanyl, Piperazinyl, Furyl, Thienyl, Pyrrolyl, Imidazolyl, Pyrazolyl, Oxazolyl, Isoxazolyl, Thiazolyl, Triazolyl, Tetrazolyl, Pyridyl, Pyrimidyl, Pyridazinyl, Indolyl, Isoindolyl, Benzimidazolyl, Indazolyl, Chinolyl, 1,3-Benzodioxolyl, Benzothiophenyl, Benzofuranyl, Imidazopyridyl oder Furo[3,2-b]pyridyl; |
| | | |
| in Ie | Ar | unsubstituiertes oder einfach durch [C(R⁶)₂]ₚHet¹ oder CN substituiertes Phenyl oder Naphthyl; |
| | | |
| in If | A | unverzweigtes oder verzweigtes Alkyl mit 1-8 C-Atomen, worin eine oder zwei nicht benachbarte CH- und/oder CH₂-Gruppen durch N- und/oder O-Atome ersetzt sein können und/oder auch 1-7 H-Atome durch F ersetzt sein können; |
| | | |
| in Ig | X | CH oder N, |
| | R¹ | H, A oder Cyc, |
| | R² | O[C(R⁶)₂]ₙHet¹ oder O[C(R⁶)₂]ₙCyc, |
| | R³ | H, Hal, O[C(R⁶)₂]ₙHet², Ar oder Het², |
| | R⁴ | H oder OR⁶, |
| | R⁵ | H oder OR⁶, |
| | R⁶ | H oder Methyl, |
| | Het¹ | unsubstituiertes oder einfach durch S(O)ₙA, S(O)ₙAr oder A substituiertes Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl oder Piperazinyl, |
| | Het² | unsubstituiertes oder einfach durch durch A, [C(R⁶)₂]ₚOR⁶ oder [C(R⁶)₂]ₚHet¹ substituiertes Dihydropyrrolyl, Pyrrolidinyl, Azetidinyl, Tetrahydroimidazolyl, Tetrahydrofuranyl, Dihydropyrazolyl, Tetrahydropyrazolyl, Dihydropyridyl, Tetrahydropyridyl, Dihydropyranyl, Tetrahydropyranyl, Piperidinyl, Morpholinyl, Hexahydropyridazinyl, Hexahydropyrimidinyl, [1,3]Dioxolanyl, Piperazinyl, Furyl, Thienyl, Pyrrolyl, Imidazolyl, Pyrazolyl, Oxazolyl, Isoxazolyl, Thiazolyl, Triazolyl, Tetrazolyl, Pyridyl, Pyrimidyl, Pyridazinyl, Indolyl, Isoindolyl, Benzimidazolyl, Indazolyl, Chinolyl, 1,3-Benzodioxolyl, Benzothiophenyl, Benzofuranyl, Imidazopyridyl oder Furo[3,2-b]pyridyl, |
| | Ar | unsubstituiertes oder einfach durch [C(R⁶)₂]ₚHet¹ oder CN substituiertes Phenyl oder Naphthyl, |
| | A | unverzweigtes oder verzweigtes Alkyl mit 1-8 C-Atomen bedeutet, worin eine oder zwei nicht benachbarte CH- und/oder CH₂-Gruppen durch N- und/oder O-Atome ersetzt sein können und/oder auch 1-7 H-Atome durch F ersetzt sein können, |
| | Cyc | unsubstituiertes oder einfach durch CN oder A substituiertes cyclisches Alkyl mit 3, 4, 5, 6 oder 7 C-Atomen, |
| | Hal | F, Cl, Br oder I, |
| | m | 1, 2 oder 3, |
| | n | 0, 1 oder 2, |
| | p | 0, 1, 2, 3 oder 4 |

bedeuten, sowie ihre pharmazeutisch verwendbaren Salze, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II mit einer Verbindung der Formel III umsetzt.
Die Verbindungen der Formel II und der Formel III sind in der Regel bekannt. Sind sie neu, so können sie aber nach an sich bekannten Methoden hergestellt werden.

In den Verbindungen der Formel II bedeutet Y vorzugsweise I. Q bedeutet vorzugsweise BOC oder Phenylsulfonyl.

In den Verbindungen der Formel III, bedeutet L vorzugsweise

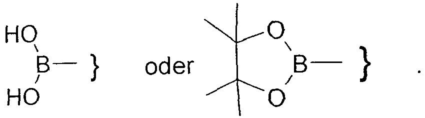

Die Umsetzung erfolgt unter Bedingungen, die dem Fachmann als Suzuki-Bedingungen oder Suzuki-Reaktion bekannt sind.

Die Reaktionszeit liegt je nach den angewendeten Bedingungen zwischen einigen Minuten und 14 Tagen, die Reaktionstemperatur zwischen etwa -10° und 160°, normalerweise zwischen 20° und 150°, besonders bevorzugt zwischen 40° und 100°C.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether, Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist DMF und Wasser.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.
Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden. Alkylierungen am Stickstoff erfolgen unter Standardbedingungen, wie sie dem Fachmann bekannt sind.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Phenylsulfonyl, Mtr, Pbf oder Pmc. Bevorzugte Aminoschutzgruppen sind BOC und Mtr, ferner CBZ, Fmoc, Benzyl und Acetyl.

Die NH-Gruppe des Pyrrolo[2,3-b]pyridin-Ringsystems wird während der Umsetzungen vorzugsweise durch eine Indolschutzgruppe geschützt. Bevorzugt sind BOC oder Phenylsulfonyl. Diese Schutzgruppe wird am Ende der Umsetzungen abgespalten.
Die Phenylsulfonylgruppe wird vorzugsweise mit Trifluorethanol oder Methanol in THF unter Zusatz einer organischen oder anorganischen Base abgespalten.
Als Basen eignen sich organische Basen, wie DIPEA, Triethylamin, Dimethylamin, Pyridin oder Chinolin. Bevorzugt ist auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist.

Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Tetrahydropyranyl, tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind.

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5 N HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°. Beschrieben werden weiterhin die Verbindungen der Formel II 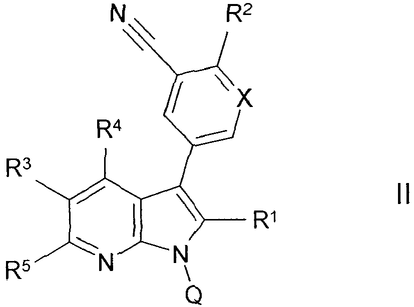 worin
- X: CH oder N,
- Q: tert.-Butoxycarbonyl oder Phenylsulfonyl,
- R¹: H, A oder Cyc,
- R²: O[C(R⁶)₂]ₙHet¹ oder O[C(R⁶)₂]ₙCyc,
- R³: H, Hal, O[C(R⁶)₂]ₙHet², Ar oder Het²,
- R⁴: H oder OR⁶,
- R⁵: H oder OR⁶,
- R⁶: H oder Methyl,
- Het¹: unsubstituiertes oder einfach durch S(O)ₙA, S(O)ₙAr oder A substituiertes Dihydropyrrolyl, Pyrrolidinyl, Azetidinyl, Oxetanyl, Tetrahydroimidazolyl, Dihydropyrazolyl, Tetrahydropyrazolyl, Tetrahydrofuranyl, Dihydropyridyl, Tetrahydropyridyl, Piperidinyl, Morpholinyl, Hexahydropyridazinyl, Hexahydropyrimidinyl, [1,3]Dioxolanyl, Tetrahydropyranyl oder Piperazinyl,
- Het²: unsubstituiertes oder einfach durch durch A, [C(R⁶)₂]ₚOR⁶ oder [C(R⁶)₂]ₚHet¹ substituiertes Dihydropyrrolyl, Pyrrolidinyl, Azetidinyl, Tetrahydroimidazolyl, Tetrahydrofuranyl, Dihydropyrazolyl, Tetrahydropyrazolyl, Dihydropyridyl, Tetrahydropyridyl, Dihydropyranyl, Tetrahydropyranyl, Piperidinyl, Morpholinyl, Hexahydropyridazinyl, Hexahydropyrimidinyl, [1,3]Dioxolanyl, Piperazinyl, Furyl, Thienyl, Pyrrolyl, Imidazolyl, Pyrazolyl, Oxazolyl, Isoxazolyl, Thiazolyl, Triazolyl, Tetrazolyl, Pyridyl, Pyrimidyl, Pyridazinyl, Indolyl, Isoindolyl, Benzimidazolyl, Indazolyl, Chinolyl, 1,3-Benzodioxolyl, Benzothiophenyl, Benzofuranyl, Imidazopyridyl oder Furo[3,2-b]pyridyl,
- Ar: unsubstituiertes oder einfach durch [C(R⁶)₂]ₚHet¹ oder CN substituiertes Phenyl oder Naphthyl,
- A: unverzweigtes oder verzweigtes Alkyl mit 1-8 C-Atomen bedeutet, worin eine oder zwei nicht benachbarte CH- und/oder CH₂-Gruppen durch N- und/oder O-Atome ersetzt sein können und/oder auch 1-7 H-Atome durch F ersetzt sein können,
- Cyc: unsubstituiertes oder einfach durch CN oder A substituiertes cyclisches Alkyl mit 3, 4, 5, 6 oder 7 C-Atomen,
- Hal: F, Cl, Br oder I,
- m: 1, 2 oder 3,
- n: 0, 1 oder 2,
- p: 0, 1, 2, 3 oder 4
bedeuten,
sowie ihre pharmazeutisch verwendbaren Salze, Tautomere und Stereoisomere, einschließlich deren Mischungen in allen Verhältnissen.

Die Bedeutungen und insbesondere die bevorzugten Bedeutungen sind die gleichen wie bei den Verbindungen der Formel I angegeben.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Kalziumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Kalzium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium, sowie die Erdalkalimetalsalze Kalzium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer lonenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C1₀₋C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Maleat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Kalzium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine erfindungsgemäße Verbindung und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie ihre pharmazeutisch verwendbaren Salze, Tautomere und Stereoisomere lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie ihre pharmazeutisch verwendbaren Salze, Tautomere und Stereoisomere können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Poly-epsilon-caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandeln den Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, oben erwähnten Krankheitszustände geeignet sind.

Gegenstand der Erfindung sind ferner Arzneimittel enthaltend mindestens eine erfindungsgemäße Verbindung und/oder sowie ihre pharmazeutisch verwendbaren Salze, Tautomere und Stereoisomere, einschließlich deren Mischungen in allen Verhältnissen, und mindestens einen weiteren Arzneimittelwirkstoff. Beschrieben wird auch ein Set (Kit), bestehend aus getrennten Packungen von
(a) einer wirksamen Menge an einer Verbindung der Formel I und/oder ihre pharmazeutisch verwendbaren Salze, Tautomere und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihre pharmazeutisch verwendbaren Salze, Tautomere und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die Erfindung betrifft die erfindungsgemäßen Verbindungen zur Verwendung für die Behandlung von Krebs, septischem Schock, primärem Offenwinkelglaukom (POAG), Hyperplasie, rheumatoider Arthritis, Psoriasis, Atherosklerose, Retinopathie, Osteoarthritis, Endometriose, chronischer Entzündung und/oder neurodegenerativen Erkrankungen wie Morbus Alzheimer. Beschrieben wird die Verwendung von Verbindungen der Formel I zur Herstellung eines Arzneimittels für die Behandlung von Krebs, septischem Schock, primärem Offenwinkelglaukom (POAG), Hyperplasie, rheumatoider Arthritis, Psoriasis, Atherosklerose, Retinopathie, Osteoarthritis, Endometriose, chronischer Entzündung und/oder neurodegenerativen Erkrankungen wie Morbus Alzheimer. Beschrieben wird eine Methode für die Behandlung eines Säugetiers, das an einer Krankheit leidet, die aus Krebs, septischem Schock, primärem Offenwinkelglaukom (POAG), Hyperplasie, rheumatoider Arthritis, Psoriasis, Atherosklerose, Retinopathie, Osteoarthritis, Endometriose, chronischer Entzündung und/oder neurodegenerativen Erkrankungen wie Morbus Alzheimer ausgewählt ist, wobei die Methode die Verabreichung einer therapeutisch wirksamen Menge einer Verbindung der Formel I an ein Säugetier umfaßt.

Die Erfindung betrifft weiterhin die erfindungsgemäßen Verbindungen zur Verwendung für die Behandlung von Krebs, septischem Schock, primärem Offenwinkelglaukom (POAG), Hyperplasie, Atherosklerose, Retinopathie, Osteoarthritis, Endometriose, chronischer Entzündung, neurodegenerativen Erkrankungen, rheumatoide Arthritis (RA), systemischer Lupus erythematosus (SLE), Sjörgrens Syndrom, Aicardi-Goutieres Syndrom Lupus Chilblain, retinale Vasculopathie, cerebrale Leukodystrophie (RVCL), systemische Sklerosis, Myositis, Psoriasis, chronisch obstruktive pulmonare Krankheit (CPD), endzündliche Darmkrankheit (IBD), Fettsucht, Insulinresistenz, Typ 2 Diabetes (NIDDM) und/oder metabolisches Syndrom

Die vorliegenden Verbindungen eignen sich als pharmazeutische Wirkstoffe für Säugetiere, insbesondere für den Menschen, bei der Behandlung und Bekämpfung von Krebserkrankungen und Entzündungserkrankungen.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln wie Anti-IgM zu ermöglichen, eine Zellantwort wie Expression eines Oberflächenmarkers zu induzieren, gewöhnlich zwischen ungefähr einer Stunde und einer Woche. Zum Testen in vitro können kultivierte Zellen aus Blut oder einer Biopsieprobe verwendet werden. Die Menge an exprimiertem Oberflächenmarker wird durch Durchflußzytometrie beurteilt, wobei spezielle Antikörper verwendet werden, die den Marker erkennen.
Die Dosis variiert abhängig von der verwendeten spezifischen Verbindung, der spezifischen Erkrankung, dem Patientenstatus usw. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z.B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B.

Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit ATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidase-konjugierten Anti-Schaf-Antikörper durch Chemilumineszenz nachweisbar (Ross et al., 2002, Biochem. J.). Beschrieben wird die Verwendung der Verbindungen der Formel I und/oder ihre physiologisch unbedenklichen Salze, Tautomere und Solvate zur Herstellung eines Arzneimittels zur Behandlung oder Vorbeugung von Krebs.

Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom Darmkrebs. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom. Beschrieben wird die Verwendung der Verbindungen der Formel I und/oder ihre physiologisch unbedenklichen Salze, Tautomere und Solvate zur Herstellung eines Arzneimittels zur Behandlung und/oder Bekämpfung einer durch Tumore bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt ist die Verwendung zur Behandlung einer Krankheit, wobei die Krebskrankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis stammt.

Die Verbindungen der Formel I können auch gemeinsam mit anderen gut bekannten Therapeutika, die aufgrund ihrer jeweiligen Eignung für das behandelte Leiden ausgewählt werden, verabreicht werden.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)-ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenyl-retinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(11)]bis[diamin-(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmern zählen zum Beispiel Paclitaxel, Vindesin-sulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.
Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]-chinolin-10,13(9H,15H)-dion, Lurtotecan, 7-[2-(N-Isopropylamino)ethyl]-(20S)-camptothecin, BNP1350, BNP11100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carbonsäureamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo-[g]isochinofin-5,10-dion, 5-(3-Aminopropylamino)-7,10-dihydroxy-2-(2-hydroxyethyl-aminomethyl)-6H-pyrazolo[4,5,1-de]acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carbonsäureamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxy-methyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### Test für die Hemmung von IKKε

### IKKε - Kinase-Assay (IKKepsilon)

### Zusammenfassung

Der Kinase-Assay wird als 384-Well-Flashplate-Assay durchgeführt (z.B. für Topcount-Messung).

1 nM IKKε, 800 nM biotinyliertes IκBα(19-42) Peptid (Biotin-C6-C6-GLKKERLLDDRHDSGLDSMKDEE) und 10 µM ATP (gespikt mit 0,3 µCi ³³P-ATP/Well) werden in einem Gesamtvolumen von 50 µl (10 mM MOPS, 10 mM Mg-acetat, 0,1 mM EGTA, 1 mM Dithiothreitol, 0,02 % Brij35, 0,1 % BSA, 0,1 % BioStab, pH 7,5) mit oder ohne Testverbindung für 2 Stunden bei 30°C inkubiert. Die Reaktion wird mit 25 µl 200 mM EDTA gestoppt. Nach 30 Min bei Raumtemperatur wird die Flüssigkeit entfernt und jeder Well dreimal mit 100 µl 0,9%iger Natriumchloridlösung gewaschen. Unspezifische Reaktion wird in Gegenwart von 3 µM MSC2119074 (BX-795) bestimmt. Die Radioaktivität wird mit einem Topcount (PerkinElmer) gemessen. Die Ergebnisse (z.B. IC₅₀-Werte) werden mit durch die IT-Abteilung breitgestellten Programm-Tools (z.B. AssayExplorer, Symyx) berechnet.

### Test für die Hemmung von TBK1

### Enzymtest

### Zusammenfassung

Der Kinase-Assay wird als 384-Well-Flashplate-Assay durchgeführt (z.B. für Topcount-Messung).

0,6 nM TANK Bindungskinase (TBK1), 800 nM biotinyliertes von MELK abgeleitetes Peptid (Biotin-Ah-Ah-AKPKGNKDYHLQTCCGSLAYRRR) und 10 µM ATP (gespikt mit 0,25 µCi ³³P-ATP/Well) werden in einem Gesamtvolumen von 50 µl (10 mM MOPS, 10 mM Mg-acetat, 0,1 mM EGTA, 1 mM DTT, 0,02 % Brij35, 0,1 % BSA, pH 7,5) mit oder ohne Testverbindung 120 Min bei 30°C inkubiert. Die Reaktion wird mit 25 µl 200 mM EDTA gestoppt. Nach 30 Min bei Raumtemperatur wird die Flüssigkeit entfernt und jeder Well dreimal mit 100 µl 0,9%iger Natriumchloridlösung gewaschen. Unspezifische Reaktion wird in Gegenwart von 100 nM Staurosporin gemessen. Die Radioaktivität wird in einem Topcount (PerkinElmer) gemessen. Die Ergebnisse (z.B. IC₅₀-Werte) werden mit durch die IT-Abteilung bereitgestellten Programm-Tools (z.B. AssayExplorer, Symyx) berechnet.

### Zelltest

Dosis-Antwort Hemmung von Phospho-IRF3 @ Ser 386
cell/MDAMB468/INH/PHOS/IMAG/pIRF3

### 1. Umfang

Obwohl TBK1 und IKKε vor allem als Schlüsselsubstanzen in der angeborenen Immunantwort bekannt sind, weisen neuere Erkenntnisse auf eine Rolle für TBK1 und IKKε in der Ras-induzierten onkogenen Transformation hin. TBK1 wurde als RalB-Effektor im Weg des Ras-like (Ral)-Guanine Nucleotide Exchange Factor (GEF) identifiziert, der für die Ras-induzierte Transformation erforderlich ist. TBK1 aktiviert direkt IRF3, das bei Phosphorylierung homodimerisiert und sich zum Kern verlagert, wo es Prozesse, die mit Entzündung, Immunregulierung, Zellüberleben und Proliferation in Zusammenhang stehen, aktiviert.

Dieser Assay wurde entwickelt, um die Wirksamkeit/Stärke von TBK1/IKKε-Hemmer-Verbindungen auf der Basis der **immunozytochemischen Detektion** von kernlokalisiertem Phospho-IRF3, einem Ziel direkt nach TBK1, zu beurteilen. Behandlung mit Polyinosin-Polycytidylsäure (poly(I:C), einem synthetischen Analogon von doppelsträngiger RNA (dsRNA), ein Molekülmuster, das mit viraler Infektion in Zusammenhang steht und vom Toll-like Rezeptor 3 (TLR3) erkannt wird, wird verwendet, um TBK1/IKKε-Aktivität und IRF3-Phosphorylierung bei Ser386 zu induzieren.

### 2. ASSAY-ÜBERSICHT

1. Tag: MDA-MB-468-Zellen werden mit HyQ-Tase abgelöst, gezählt und auf einer 384-Well-Platte mit TC-Oberfläche und transparentem Boden mit einer Dichte von 10 000 Zellen pro Well in einem Gesamtvolumen von 35 µl Komplettmedium ausgesät. Alternativ werden die Zellen direkt aus tiefgefrorenen Glasfläschchen ausgesät.
2. Tag: Die Zellen werden vor der Poly(I:C)-Stimulierung 1 h mit Hemmer-Verbindungen vorbehandelt. Nach 2h Inkubation mit Poly(I:C) werden die Zellen in (Para)formaldehyd (PFA) fixiert und mit Methanol (MeOH) permeabilisiert. Die Zellen werden dann blockiert und mit einem anti-pIRF3-Antikörper bei 4°C über Nacht inkubiert.
3. Tag: Der primäre Antikörper wird weggewaschen, ein AlexaFluor488-konjugierter sekundärer zugegeben, die Zellen werden mit Propidiumiodid kontrastgefärbt, gefolgt durch Bildaufnahme auf einem IMX Ultra High Content Reader.

### 3. Reagenzien, Materialien

Zellen : ATCC HTB 132, Burger Lab (MP-CB 2010-327 oder MDA-MB-468 / 10)
Plattiermedium = Kulturmedium:
   RPMI 1640, Invitrogen # 31870
   10% FCS, Invitrogen # 10270-106
      2mM Glutamax, Invitrogen #35050-038
   1mM Natrium-Pyruvat, Invitrogen # 11360
      1% Pen / Strep
   37°C, 5% CO₂
Platten : 384-Weil-Boden-Zellkulturplatten mit schwarzem / transparentem Boden, Falcon #35 3962 oder Greiner #781090
Subkultivierung: HyQ-Tase, Thermo Scientific (HyClone) # SV30030.01 weitere Reagenzien:
   Poly(I:C) (LMW), Invitrogen # tlrl-picw (20mg/ml Stammlösung in steriler PBS herstellen, 30min 55°C im Wasserbad denaturieren, langsam auf RT abkühlen, in Aliquots bei -20°C lagern)
Referenzhemmer: MSC2119074A-4 = BX-795 (IC50 : 200-800nM)
   Hemmkontrolle: 10µM MSC2119074A-4 = BX-795
   neutrale Kontrolle: 0,5% DMSO
   eine 10Punkt-Dosis-Antwort-Kurve mit MSC2119074A-4 = BX-795 ist in jedem Versuch enthalten
Hepes, Merck #1.10110
PBS 1x DPBS , Invitrogen # 14190
Formaldehyd (methanolfrei, 16%, ultrareine EM-Qualität), Polysciences # 18814 (Lagerung RT), Endkonz.: 4%
Methanol, Merck # 1.06009.1011 (vorgekühlt -20°C)
Ziegenserum, PAA # B15-035 (Lagerung 4°C, langfristig -20°C), Endkonz.: 10%
BSA (IgG- und Protease-frei, 30%), US-Biological # A1317 (Lagerung 4°C, langfristig -20°C), Endkonz.: 2%
   Tween 20 Detergens, Calbiochem # 655204 (Lagerung RT), (10%ige Stammlösung in Wasser herstellen; Endkonz.: 0,1%)
anti-pIRF-3 Kaninchen MAK, Epitomics # 2526-B (Lagerung -20°C), Endkonz.: 1:2000 in PBS / 2% BSA
   Alexa Fluor Ziege-anti-Kaninchen-488, Invitrogen # A11034 oder # A11008 (Lagerung 4°C, dunkel), Endkonz.: 1:2000 in PBS / 2% BSA / 0,1% Tween
Propidiumiodid (PI), Fluka # 81845, 1mg/ml in H₂O (Lagerung 4°C, dunkel), Endkonz.: 0,2µg/ml

### 4. Ablauf

| |
|---|
| 10 000 Zellen/well/35µl komplettes RPMI + 10% FCS in 384-Well-Boden-Zellkulturplatten mit schwarzem / transparentem Boden aussähen |
| ↓ |
| 2 h bei Raumtemperatur auf der Bank inkubieren, gefolgt durch weitere Inkubation 22h bei 37°C, 5 % CO₂ und 90 % rF |
| ↓ |
| **Behandlung der Verbindung:** 5µl vorverdünnte Verbindungen, Standard oder Kontrollreagenzien zugeben (8fach konz.) Verb. Verdünnung von DMSO-Stammlösungen in 20mM Hepes pH 7,2; DMSO Endkonz.: 0,5% Reihenverdünnung der Verb. aus 10mM Stammlösung (Remp) 10 Schritte, 3,16fach in DMSO 30µM 9,49µM 3µM 0,95µM 0,3µM 0,095µM 0,03µM 0,0095µM 0,003µM 0,00095µM 60 Minuten bei 37°C, 5 % CO₂ und 90 % rF inkubieren |
| ↓ |
| **Stimulierungsbehandlung:** Zu allen Wells außer nicht stimulierten Kontrollen 10µl Poly(I:C) zugeben, so daß eine Endkonzentration von 100µg/ml erzielt wird (Stammlösung 20mg/ml →1:40 in PBS) (5fach konz.) 120 Minuten bei 37°C, 5 % CO₂ und 90 % rF inkubieren |
| ↓ |
| Überstand vollständig absaugen |
| ↓ |
| **Zellen fixieren:** 100 µl 4 %iges Paraformaldehyd in PBS zugeben 15 Minuten bei RT inkubieren |
| ↓ |
| 3x mit 80 µl PBS waschen (Tecan Powerwasher), Überstand vollständig absaugen Platte auf Eis legen |
| **Zellen permeabilisieren:** Schnell 100 µl -20°C kaltes MeOH zugeben (Vorratsbehälter vorkühlen) 10 Minuten bei RT oder 4°C inkubieren |
| ↓ |
| Einmal mit 80 µl PBS waschen (Tecan Powerwasher), Überstand vollständig absaugen |
| ↓ |
| **Unspezifische Bindung blockieren:** 30 µl 10 %iges Ziegenserum in PBS / 2 % BSA zugeben Auf dem Multidrop Combi schütteln (17 Sekunden) 60 Minuten bei 37°C inkubieren |
| ↓ |
| Überstand vollständig absaugen |
| ↓ |
| **Primäres Anfärben:** 25 µl primären Antikörper 1:2000 in PBS / 2 % BSA verdünnt zugeben Auf dem Multidrop Combi schütteln (17 Sekunden) Über Nacht bei 4°C inkubieren |
| ↓ |
| 3x mit 80 µl PBS waschen (Tecan Powerwasher), Überstand vollständig absaugen |
| ↓ |
| **Sekundäres Anfärben und Kernfärbung:** 25 µl sekundären Antikörper (1:2000) und 0,2 µg/ml Propidiumiodid in PBS / 2 % BSA / 0,1% Tween zugeben Auf dem Multidrop Combi schütteln (17 Sekunden) 75 Minuten bei 37°C inkubieren |
| ↓ |
| 3x mit 80 µl PBS (Tecan Powerwasher) waschen, Überstand vollständig absaugen |
| ↓ |
| 80 µl PBS in alle Wells geben |
| ↓ |
| Platten mit transparenter Klebeabdichtung abdichten |
| ↓ |
| Bildaufnahme am IMX Ultra (Metaexpress 3.1. Scan-Einstellungen TBK_10x_pin8) |
| Bildanalyse (Metaexpress 3.1. <cell scoring>, TBK1-Cellscoring) |
| ↓ |
| Datenanalyse und Rapportieren mit Assay-Explorer |

### HPLC/HPLC-MS Bedingungen

HPLC/MS Bedingungen A
Säule: Chromolith Performance ROD RP-18e, 100 x 3 mm²
Gradient: A:B = 99:1 auf 0:100 in 3.5 min
Flussrate: 2.0 ml/min
Eluent A: Wasser + 0.05 % Ameisensäure
Eluent B: Acetonitril + 0.04 % Ameisensäure
Wellenlänge: 220 nm
Massenspektroskopie: Positiv-Modus

HPLC/MS Bedingungen B
Säule: Chromolith Performance ROD RP-18e, 50 x 4.6 mm²
Gradient: A:B = 96:4 auf 0:100 in 2.8 min
Flussrate: 2.40 ml/min
Eluent A: Wasser + 0.05 % Ameisensäure
Eluent B: Acetonitril + 0.04 % Ameisensäure
Wellenlänge: 220 nm
Massenspektroskopie: Positiv-Modus

### HPLC/MS Bedingungen C

Säule: Chromolith Performance ROD RP-18e, 100 x 3 mm²
Gradient: A:B = 99:1 to 0:100 in 1.8 min
Flussrate: 2.0 ml/min
Eluent A: Wasser + 0.05 % Ameisensäure
Eluent B: Acetonitril + 0.04 % Ameisensäure
Wellenlänge: 220 nm
Massenspektroskopie: Positiv-Modus

### Referenzbeispiel 1

Die Herstellung von 5-(1H-Pyrrolo[2,3-b]pyridin-3-yl)-2-tetrahydropyran-4-yloxy-benzonitril ("A1") erfolgt analog nachstehendem Schema:

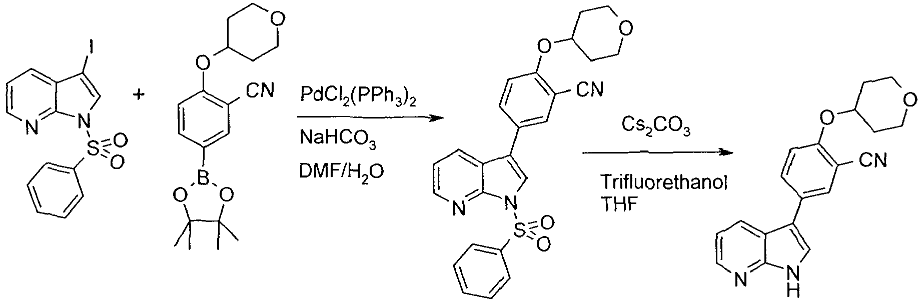

Eine unter Stickstoff gehaltene Suspension von 384 mg (1.00 mmol) 1-(Benzol-sulfonyl)-3-iod-pyrrolo[2,3-b]pyridin, 362 mg (1.10 mmol) 2-Tetrahydropyran-4-yloxy-5-(4,4,5,5-tetramethyl-1,3,2-dioxaborolan-2-yl)benzonitril (Herstellung beschrieben in WO 2011/046970) und 101 mg (1.2 mmol) Natriumhydrogencarbonat in 2 ml DMF und 1 ml Wasser wird unter Rühren auf 40° C erwärmt. Dann werden 14.0 mg (0.02 mmol) Bis(triphenylphosphin)-palladium(II)-chlorid zugegeben. Das Reaktionsgemisch wird auf 80° C erhitzt und bei dieser Temperatur 18 Stunden gerührt. Das Reaktionsgemisch wird auf Raumtemperatur gekühlt und mit Wasser versetzt. Der entstandene Niederschlag wird abgesaugt, mit Wasser gewaschen und im Vakuum getrocknet: 1-(Benzolsulfonyl)-3-(3-methyl-4-tetrahydropyran-4-yloxy-phenyl)pyrrolo[2,3-b]pyridin als graues Pulver; HPLC/MS (B): 2.47 min, [M+H] 460.

Zu einer Suspension von 436 mg (0.95 mmol) 1-(Benzolsulfonyl)-3-(3-methyl-4-tetrahydropyran-4-yloxy-phenyl)pyrrolo[2,3-b]pyridin in 8 ml THF werden 8 ml 2,2,2-Trifluorethanol und 928 mg (2.85 mmol) Caesiumcarbonat gegeben. Das Reaktionsgemisch wird 7 Stunden bei 80° C gerührt, auf Raumtemperatur gekühlt und zwischen THF und gesättigter Natriumchloridlösung verteilt. Die organische Phase wird über Natriumsulfat getrocknet und eingedampft. Der Rückstand wird an einer Kieselgelsäule mit Cyclohexan/Ethylacetat als Laufmittel chromatographiert: 5-(1H-Pyrrolo[2,3-b]pyridin-3-yl)-2-tetrahydropyran-4-yloxy-benzonitril als farblose Kristalle; HPLC/MS (B): 1.92 min, [M+H] 320;
¹H NMR (400 MHz, DMSO-d₆) δ [ppm] = 11.94 (s, 1H), 8.28 (m, 2H), 8.03 (d, *J*=2.3*,* 1H), 7.98 (dd, *J*=8.8, 2.4, 1H), 7.92 (d, *J*=2.2, 1H), 7.41 (d, *J*=8.9, 1H), 7.16 (dd, *J*=7.9*,* 4.8, 1H), 4.84 (tt, *J*=7.8, 3.8, 1H), 3.88 (m, 2H), 3.55 (ddd, *J*=11.5, 8.3, 3.1, 2H), 2.02 (m, 2H), 1.68 (m, 2H).

Analog erhält man:
5-(4-Methoxy-1H-pyrrolo[2,3-b]pyridin-3-yl)-2-tetrahydropyran-4-yloxy-benzonitril ("A2") 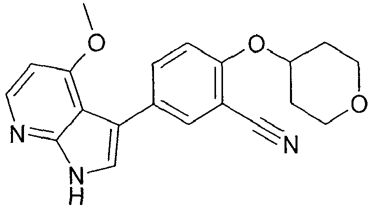 ausgehend von 1-(Benzolsulfonyl)-3-iod-4-methoxy-pyrrolo[2,3-b]pyridin (Herstellung beschrieben in WO2011/008915);
   HPLC/MS (A): 1.85 min, [M+H] 350;
5-(6-Methoxy-1H-pyrrolo[2,3-b]pyridin-3-yl)-2-tetrahydropyran-4-yloxy-benzonitril ("A3") 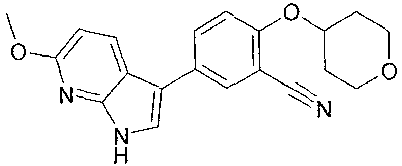 ausgehend von 1-(Benzolsulfonyl)-3-iod-6-methoxy-pyrrolo[2,3-b]pyridin; HPLC/MS (A): 2.71 min, [M+H] 350.

### Beispiel 2

Die Herstellung von 5-[5-(1-Methylpyrazol-4-yl)-1H-pyrrolo[2,3-b]pyridin-3-yl]-2-tetrahydropyran-4-yloxy-benzonitril ("A4") erfolgt analog nachstehendem Schema:

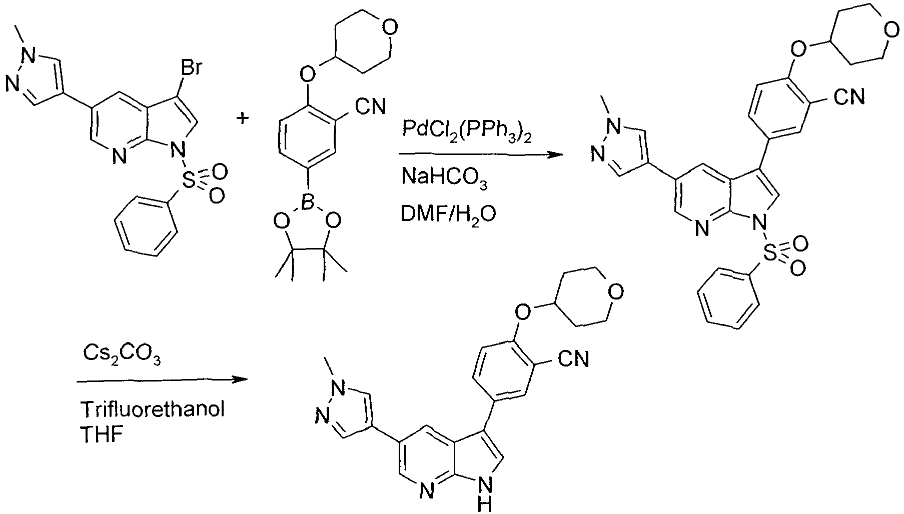

Ausgehend von 1-(Benzolsulfonyl)-3-brom-5-(1-methylpyrazol-4-yl)pyrrolo[2,3-b]pyridin (Synthese beschrieben in WO 2009/054941) wird analog zu Beispiel 1 die Verbindung 5-[5-(1-Methylpyrazol-4-yl)-1H-pyrrolo[2,3-b]pyridin-3-yl]-2-tetrahydropyran-4-yloxy-benzonitril hergestellt; leicht gelbe Kristalle; HPLC/MS (B): 1.92 min, [M+H] 400;
¹H NMR (500 MHz, DMSO-d₆) δ [ppm] = 11.91 (d, *J*=1.7, 1H), 8.53 (d, *J*=2.0, 1H), 8.35 (d, *J*=1.8, 1H), 8.23 (s, 1H), 8.06 (d, *J*=2.3, 1H), 8.03 (dd, *J*=8.8, 2.3, 1H), 7.97 (s, 1H), 7.90 (d, *J*=2.6, 1H), 7.41 (d, *J*=8.9, 1H), 4.85 (tt, *J*=7.7, 3.7, 1H), 3.89 (m, 5H), 3.56 (ddd, *J*=11.4, 8.3, 3.1, 2H), 2.03 (m, 2H), 1.70 (m, 2H).

### Referenzbeispiel 3

Die Herstellung von 5-(5-Brom-1H-pyrrolo[2,3-b]pyridin-3-yl)-2-tetrahydropyran-4-yloxy-benzonitril ("A5") erfolgt analog nachstehendem Schema:

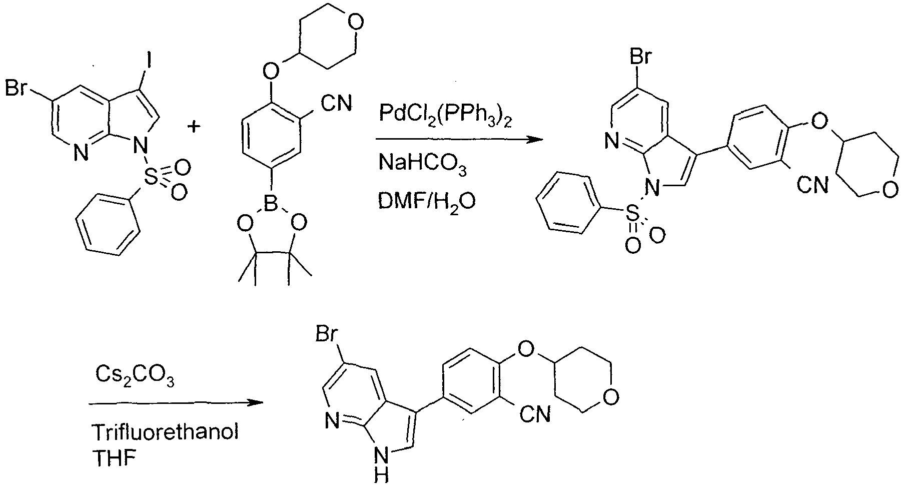

Eine unter Stickstoff gehaltene Suspension von 4.63 g (10.0 mmol) 1-(Benzol-sulfonyl)-5-brom-3-iod-pyrrolo[2,3-b]pyridin, 3.62 g (11.0 mmol) 2-Tetrahydropyran-4-yloxy-5-(4,4,5,5-tetramethyl-1,3,2-dioxaborolan-2-yl)benzonitril und 1.01 g (12.0 mmol) Natriumhydrogencarbonat in 20 ml DMF und 10 ml Wasser wird unter Rühren auf 40° C erwärmt. Dann werden 140 mg (0.20 mmol) Bis(triphenylphosphin)-palladium(II)-chlorid zugegeben. Das Reaktionsgemisch wird auf 80° C erhitzt und bei dieser Temperatur 18 Stunden gerührt. Das Reaktionsgemisch wird auf Raumtemperatur gekühlt und mit Wasser versetzt. Der entstandene Niederschlag wird abgesaugt, mit Wasser gewaschen und im Vakuum getrocknet: 5-[1-(Benzolsulfonyl)-5-brom-pyrrolo[2,3-b]pyridin-3-yl]-2-tetrahydropyran-4-yloxy-benzonitril als hellgraue Kristalle; HPLC/MS (A): 3.32 min, [M+H] 538/540;
¹H NMR (400 MHz, DMSO-d₆) 5 [ppm] = 8.57 (d, *J*=2.1, 1H), 8.53 (d, *J*=2.1, 1H), 8.37 (s, 1H), 8.19 (d, *J*=2.3, 1H), 8.16 (d, *J*=7.3, 2H), 8.05 (dd, *J*=8.8, 2.4, 1H), 7.75 (m, 1H), 7.65 (t, *J*=7.8, 2H), 7.44 (d, *J*=9.0, 1H), 4.90 (dt, *J*=11.7, 3.9, 1H), 3.88 (m, 2H), 3.56 (ddd, *J*=11.4, 8.2, 3.2, 3H), 2.03 (m, 2H), 1.69 (dtd, *J*=12.1, 8.1, 3.8, 2H).

Zu einer Suspension von 241 mg (0.45 mmol) 5-[1-(Benzolsulfonyl)-5-brom-pyrrolo[2,3-b]pyridin-3-yl]-2-tetrahydropyran-4-yloxy-benzonitril in 4 ml THF werden 4 ml 2,2,2-Trifluorethanol und 438 mg (1.35 mmol) Caesiumcarbonat gegeben. Das Reaktionsgemisch wird 3 Stunden bei 80° C gerührt, auf Raumtemperatur gekühlt und zwischen THF und gesättigter Natriumchloridlösung verteilt. Die organische Phase wird über Natriumsulfat getrocknet und eingedampft. Der Rückstand wird an einer Kieselgelsäule mit Cyclohexan/Ethylacetat als Laufmittel chromatographiert: 5-(5-Brom-1H-pyrrolo[2,3-b]pyridin-3-yl)-2-tetrahydropyran-4-yloxy-benzonitril als farblose Kristalle; HPLC/MS (B): 2.32 min, [M+H] 398/400;
¹H NMR (400 MHz, DMSO-d₆) δ [ppm] = 12.20 (s, 1H), 8.51 (d, *J*=2.1, 1H), 8.34 (d, *J*=2.1, 1H), 8.07 (d, *J*=2.3, 1H), 8.00 (s, 1H), 7.98 (dd, *J*=8.9, 2.4, 1H), 7.40 (d, *J*=8.9, 1H), 4.85 (tt, *J*=7.6, 3.7, 1H), 3.89 (m, 2H), 3.56 (ddd, *J*=11.4, 8.3, 3.1, 2H), 2.03 (m, 2H), 1.69 (dtd, *J*=12.2, 8.1, 3.8, 2H).

### Beispiel 4

Die Herstellung von 5-[5-(4-Cyanphenyl)-1H-pyrrolo[2,3-b]pyridin-3-yl]-2-tetrahydropyran-4-yloxy-benzonitril ("A6") erfolgt analog nachstehendem Schema:

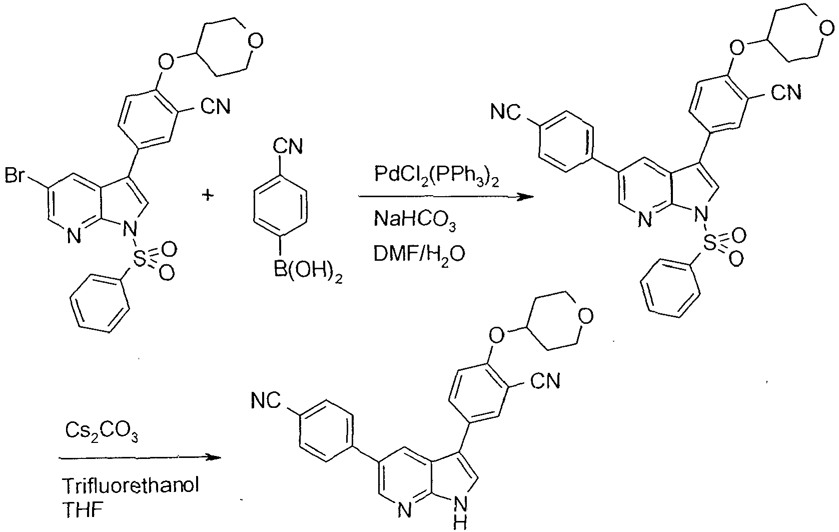

Eine unter Stickstoff gehaltene Suspension von 538 mg (1.00 mmol) 5-[1-(Benzol-sulfonyl)-5-brom-pyrrolo[2,3-b]pyridin-3-yl]-2-tetrahydropyran-4-yloxy-benzonitril, 162 mg (1.10 mmol) 4-Cyanphenylboronsäure und 101 mg (1.20 mmol) Natriumhydrogencarbonat in 2 ml DMF und 1 ml Wasser wird unter Rühren auf 40° C erwärmt. Dann werden 14 mg (0.02 mmol) Bis(triphenylphosphin)-palladium(II)-chlorid zugegeben. Das Reaktionsgemisch wird auf 80° C erhitzt und bei dieser Temperatur 4 Tage gerührt. Das Reaktionsgemisch wird auf Raumtemperatur gekühlt und zwischen Wasser und Dichlormethan verteilt. Die organische Phase wird über Natriumsulfat getrocknet und eingedampft. Der Rückstand wird an einer Kieselgelsäule mit Cyclohexan/Ethylacetat chromatographiert: 5-[1-(Benzol-sulfonyl)-5-(4-cyanphenyl)pyrrolo[2,3-b]pyridin-3-yl]-2-tetrahydropyran-4-yloxy-benzonitril als farblose Kristalle; HPLC/MS (A): 3.55 min, [M+H] 561.

Zu einer Suspension von 252 mg (0.45 mmol) 5-[1-(Benzolsulfonyl)-5-(4-cyan-phenyl)pyrrolo[2,3-b]pyridin-3-yl]-2-tetrahydropyran-4-yloxy-benzonitril in 4 ml THF werden 4 ml 2,2,2-Trifluorethanol und 439 mg (1.35 mmol) Caesiumcarbonat gegeben. Das Reaktionsgemisch wird 3 Stunden bei 80° C gerührt, auf Raumtemperatur gekühlt und zwischen THF und gesättigter Natriumchloridlösung verteilt. Die organische Phase wird über Natriumsulfat getrocknet und eingedampft. Der Rückstand wird an einer Kieselgelsäule mit Cyclohexan/Ethylacetat als Laufmittel chromatographiert: 5-[5-(4-Cyanphenyl)-1H-pyrrolo[2,3-b]pyridin-3-yl]-2-tetrahydropyran-4-yloxy-benzonitril als farblose Kristalle; HPLC/MS (B): 2.31 min, [M+H] 421;
¹H NMR (500 MHz, DMSO-d₆) δ [ppm] = 12.15 (s, 1H), 8.66 (d, *J*=2.1, 1H), 8.56 (d, *J*=2.1, 1H), 8.14 (d, *J*=2.3, 1H), 8.08 (dd, *J*=8.8, 2.3, 1H), 8.04 (d, *J*=8.5, 2H), 8.00 (s, 1H), 7.95 (d, *J*=8.5, 2H), 7.42 (d, *J*=8.9, 1H), 4.85 (tt, *J*=7.8, 3.8, 1H), 3.89 (m, 2H), 3.56 (ddd, *J*=11.4, 8.3, 3.1, 2H), 2.03 (m, 2H), 1.69 (dtd, *J*=12.2, 8.1, 3.8, 2H).

Analog werden hergestellt:
5-[5-[1-(2-Morpholinoethyl)pyrazol-4-yl]-1H-pyrrolo[2,3-b]pyridin-3-yl]-2-tetrahydropyran-4-yloxy-benzonitril ("A7") 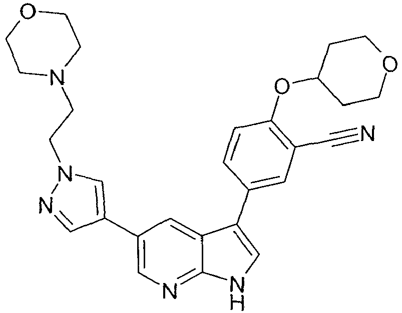 ausgehend von 4-[2-[4-(4,4,5,5-Tetramethyl-1,3,2-dioxaborolan-2-yl)pyrazol-1-yl]ethyl]morpholin;
   HPLC/MS (B): 1.55 min, [M+H] 499;
5-[5-(4-Morpholinophenyl)-1H-pyrrolo[2,3-b]pyridin-3-yl]-2-tetrahydropyan-4-yloxy-benzonitril ("A8") 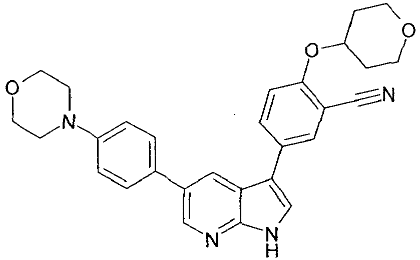 ausgehend von 4-[4-(4,4,5,5-Tetramethyl-[1,3,2]dioxaborolan-2-yl)-phenyl]-morpholin;
   HPLC/MS (B): 2.26 min, [M+H] 481;
5-[5-[1-(2-Pyrro)idin-1-ylethyl)pyrazol-4-yl]-1H-pyrrolo[2,3-b]pyridin-3-yl]-2-tetrahydropyran-4-yloxy-benzonitril ("A9") 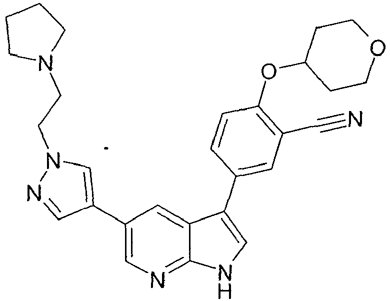 ausgehend von 1-(2-Pyrrolidin-1-yl-ethyl)-4-(4,4,5,5-tetramethyl-[1,3,2]dioxaborolan-2-yl)-1H-pyrazol;
   HPLC/MS (A): 1.83 min, [M+H] 483;
5-[5-[1-(2-Methoxyethyl)pyrazol-4-yl]-1H-pyrrolo[2,3-b]pyridin-3-yl]-2-tetrahydropyran-4-yloxy-benzonitril ("A10") 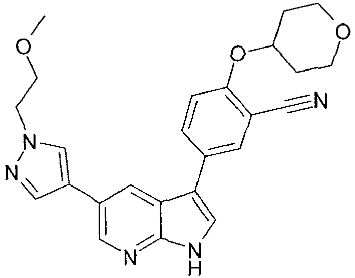 ausgehend von 1-(2-Methoxy-ethyl)-4-(4,4,5,5-tetramethyl-[1,3,2]dioxaborolan-2-yl)-1H-pyrazol;
   HPLC/MS (A): 2.30 min, [M+H] 444;
¹H NMR (400 MHz, DMSO-d₆) δ [ppm] 11.90 (s, 1H), 8.54 (d, *J*=2.0, 1H), 8.36 (d, *J*=2.0, 1H), 8.25 (s, 1H), 8.07 (d, *J*=2.3, 1H), 8.03 (dd, *J*=8.7, 2.3, 1H), 8.00 (s, 1H), 7.90 (s, 1H), 7.41 (d, *J*=8.9, 1H), 4.85 (tt, *J*=7.8, 3.8, 1H), 4.29 (t, *J*=5.4, 2H), 3.89 (m, 2H), 3.74 (t, *J*=5.4, 2H), 3.56 (ddd, *J*=11.4, 8.3, 3.1, 2H), 3.26 (s, 3H), 2.03 (m, 2H), 1.70 (dtd, *J*=12.3, 8.1, 3.9, 2H).

### Beispiel 5

Die Herstellung von 5-[2-Methyl-5-(1-methylpyrazol-4-yl)-1H-pyrrolo[2,3-b]pyridin-3-yl]-2-tetrahydropyran-4-yloxy-benzonitril ("A11") erfolgt analog nachstehendem Schema:

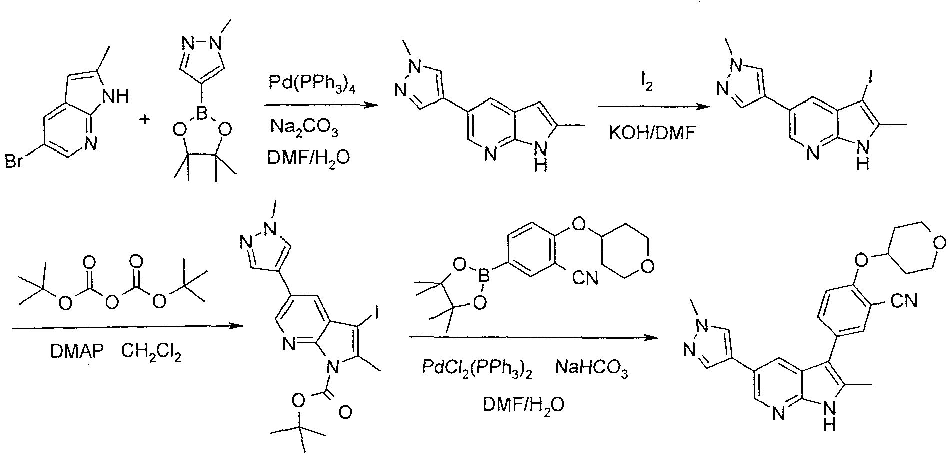

Zu einer unter Stickstoff gehaltenen Lösung von 2.11 g (10.0 mmol) 5-Brom-2-methyl-1H-pyrrolo[2,3-b]pyridin und 3.54 g (17.0 mmol) 1-Methyl-1H-pyrazol-4-boronsäure-pinacol-ester in 30 ml DMF wird eine Lösung von 3.18 g (30.0 mmol) Natriumcarbonat in 15 ml Wasser gegeben. Das Gemisch wird auf 80° C erhitzt, mit 462 mg (0.40 mmol) Tetrakis(triphenylphosphin)-palladium versetzt und 18 Stunden bei 80° C gerührt. Das Reaktionsgemisch wird auf Raumtemperatur gekühlt und 50 ml Wasser werden zugegeben. Der entstandene Niederschlag wird abgesaugt, mit Wasser gewaschen und im Vakuum getrocknet: 2-Methyl-5-(1-methylpyrazol-4-yl)-1H-pyrrolo[2,3-b]pyridin als grauer Feststoff; HPLC/MS (A): 1.68 min, [M+H] 213.

Zu einer Suspension von 1.70 g (8.00 mmol) 2-Methyl-5-(1-methylpyrazol-4-yl)-1H-pyrrolo[2,3-b]pyridin in 8 ml DMF werden 1.12 g (20.0 mmol) Kaliumhydroxid (als Pulver) gegeben und anschließend eine Lösung von 2.03 g (8.00 mmol) Iod in 8 ml DMF zugetropft. Das Reaktionsgemisch wird 30 Minuten bei Raumtemperatur gerührt und anschließend zwischen Ethylacetat und wässriger verdünnter Natriumhydrogensulfit-Lösung verteilt. Die unlöslichen Anteile werden abgesaugt und im Vakuum getrocknet: 3-Iod-2-methyl-5-(1-methylpyrazol-4-yl)-1H-pyrrolo[2,3-b]pyridin als graues Pulver; HPLC/MS (A): 2.45 min, [M+H] 339.
Aus der organischen Phase wird nach Trocknen über Natriumsulfat und Eindampfen weiteres Produkt isoliert.

Zu einer Suspension von 2.27 g (6.72 mmol) 3-lod-2-methyl-5-(1-methylpyrazol-4-yl)-1H-pyrrolo[2,3-b]pyridin in 14 ml Dichlormethan werden 82.1 mg (0.67 mmol) 4-(Dimethylamino)-pyridin und anschließend eine Lösung von 2.20 g (10.1 mmol) Ditert-butyldicarbonat in 7 ml Dichlormethan zugetropft. Das Reaktionsgemisch wird 30 Minuten bei Raumemperatur gerührt und anschließend zweimal mit gesättigter Natriumthiosulfat-Lösung und einmal mit Wasser gewaschen. Die organische Phase wird über Natriumsulfat getrocknet und eingedampft. Der Rückstand wird an einer Kieselgelsäule mit Cyclohexan/Ethylacetat als Laufmittel chromatographiert: tert-Butyl-3-iod-2-methyl-5-(1-methylpyrazol-4-yl)pyrrolo[2,3-b]pyridin-1-carboxylat als farblose Kristalle; HPLC/MS (A): 3.35 min, [M+H] 439.

Eine unter Stickstoff gehaltene Lösung von 285 mg (0.65 mmol) tert-Butyl-3-iod-2-methyl-5-(1-methylpyrazol-4-yl)pyrrolo[2,3-b]pyridin-1-carboxylat, 213 mg (0.65 mmol) 2-Tetrahydropyran-4-yloxy-5-(4,4,5,5-tetramethyl-1,3,2-dioxaborolan-2-yl)benzonitril und 4.56 mg (6.5 µmol) Bis(triphenylphosphin)-palladium(II)-chlorid in 2 ml DMF wird auf 80° C erhitzt. Nach Zugabe von 65.5 mg (0.78 mmol) Natriumhydrogencarbonat und 1 ml Wasser wird das Reaktionsgemisch 18 Stunden bei 80° C gerührt. Das Reaktionsgemisch wird auf Raumtemperatur gekühlt und zwischen Wasser und Dichlormethan verteilt. Die organische Phase wird über Natriumsulfat getrocknet und eingedampft. Der Rückstand wird an einer Kieselgelsäule mit Ethylacetat/Methanol als Laufmittel chromatographiert: 5-[2-Methyl-5-(1-methylpyrazol-4-yl)-1H-pyrroio[2,3-b]pyridin-3-yl]-2-tetrahydropyran-4-yloxy-benzonitril als blassgelbe Kristalle; HPLC/MS (A): 2.45 min, [M+H] 414.
¹H NMR (400 MHz, DMSO-d₆) δ [ppm] = 11.71 (s, 1H), 8.42 (d, *J*=2.0, 1H), 8.15 (s, 1H), 7.96 (d, *J*=2.0, 1H), 7.89 (d, *J*=0.7, 1H), 7.79 (d, *J*=2.2, 1H), 7.76 (dd, *J*=8.7, 2.3, 1H), 7.44 (d, *J*=8.8, 1H), 4.85 (tt, *J*=7.8, 3.8, 1H), 3.90 (m, 5H), 3.56 (ddd, *J*=11.5, 8.4, 3.1, 2H), 2.46 (s, 3H), 2.05 (m, 2H), 1.71 (dtd, *J*=12.3, 8.2, 3.8, 2H).

### Beispiel 6

Die Herstellung von 5-[5-(1-Piperidin-4-yl-1H-pyrazol-4-yl)-1H-pyrrolo[2,3-b]pyridin-3-yl]-2-(tetrahydro-pyran-4-yloxy)-benzonitril ("A12") erfolgt analog nachstehendem Schema:

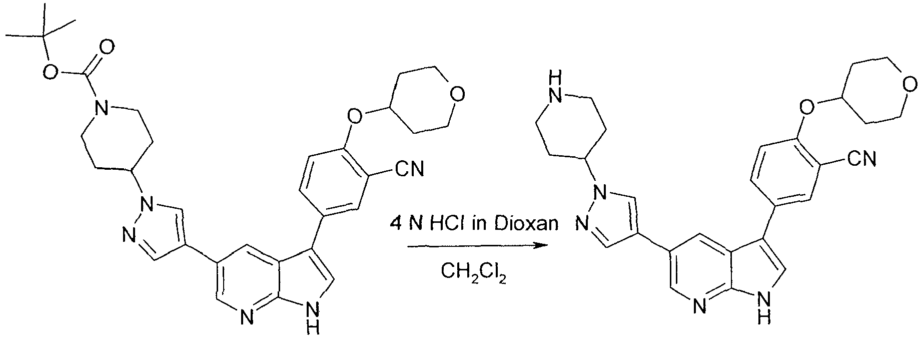

Eine Lösung von 96.7 mg (0.17 mmol) 4-(4-{3-[3-Cyan-4-(tetrahydro-pyran-4-yloxy)-phenyl]-1H-pyrrolo[2,3-b]pyridin-5-yl}-pyrazol-1-yl)-piperidin-1-carbonsäure-tert-butylester (hergestellt aus 4-[4-(4,4,5,5-Tetramethyl-[1,3,2]dioxaborolan-2-yl)-pyrazol-1-yl]-piperidin-1-carbonsäure-tert-butylester) in 1.6 ml Dichlormethan wird mit 0.4 ml einer 0.4 N Lösung von Chlorwasserstoff in Dioxan versetzt. Das Reaktionsgemisch wird 16 Stunden bei Raumtemperatur belassen und anschließend zwischen Dichlormethan und gesättigter Natriumcarbonat-Lösung verteilt. Die organische Phase wird über Natriumsulfat getrocknet und eingedampft: 5-[5-(1-Piperidin-4-yl-1H-pyrazol-4-yl)-1H-pyrrolo[2,3-b]pyridin-3-yl]-2-(tetrahydro-pyran-4-yloxy)-benzonitril als hellgelbes Pulver: HPLC/MS (B): 1.56 min, [M+H] 469;
¹H NMR (400 MHz, DMSO-d₆) δ [ppm] = 11.94 (s, 1H), 8.59 (d, *J*=2.0, 1H), 8.41 (d, *J*=2.0, 1H), 8.36 (s, 1H), 8.11 (d, *J*=2.2, 1H), 8.08 (dd, *J*=8.7, 2.3, 1H), 8.02 (s, 1H), 7.93 (s, 1H), 7.45 (d, *J*=8.9, 1H), 4.89 (m, 1H), 4.25 (ddd, *J*=11.6, 7.5, 4.1, 1H), 3.93 (m, 2H), 3.60 (m, 2H), 3.10 (d, *J*=12.3, 2H), 2.66 (m, 2H), 2.06 (m, 5H), 1.88 (m, 2H), 1.74 (dtd, *J*=12.3, 8.2, 3.8, 2H).

### Beispiel 7

Die Herstellung von 2-(1-Methyl-piperidin-4-yloxy)-5-[5-(1-methyl-1H-pyrazol-4-yl)-1H-pyrrolo[2,3-b]pyridin-3-yl]-benzonitril ("A13") und von 5-(5-Brom-1H-pyrroto[2,3-b]pyridin-3-yl)-2-(cyclopropylmethoxy)benzonitril ("A14") erfolgt analog nachstehendem Schema:

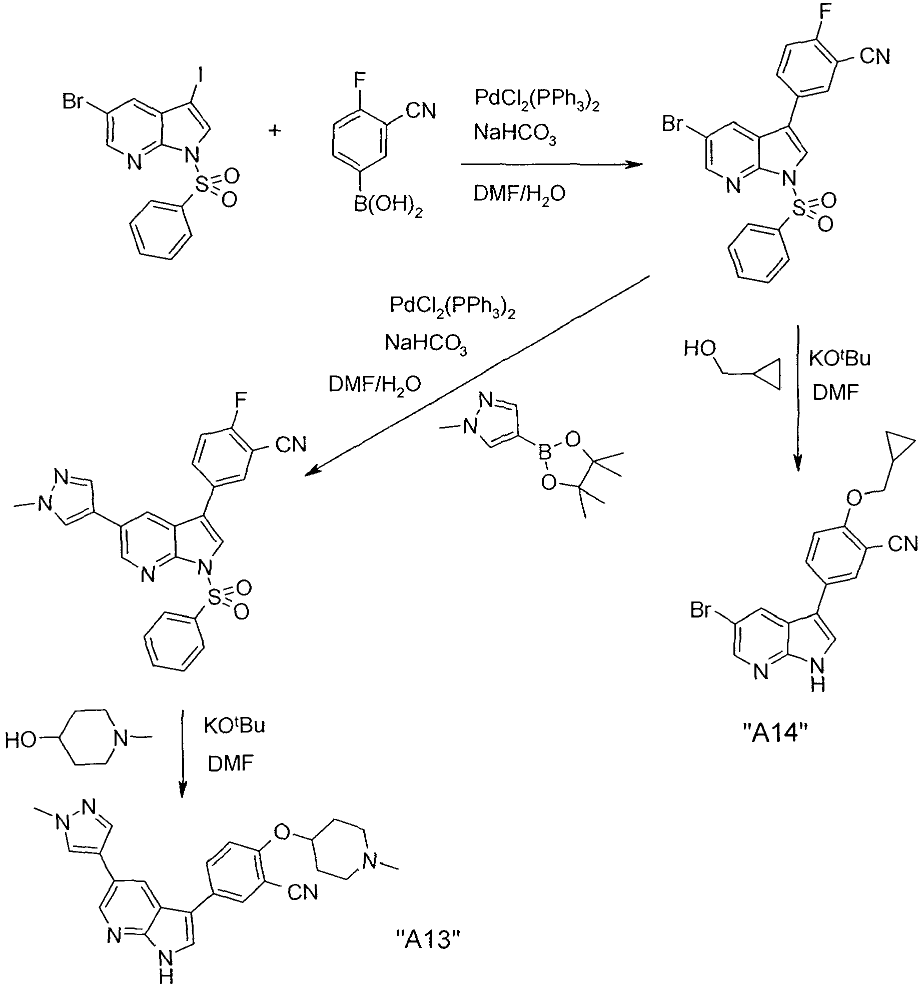

Eine unter Stickstoff gehaltene Suspension von 4.63 g (10.0 mmol) 1-(Benzol-sulfonyl)-5-brom-3-iod-pyrrolo[2,3-b]pyridin, 1.81 g (11.0 mmol) (3-Cyan-4-fluorphenyl)boronsäure und 1.01 g (12.0 mmol) Natriumhydrogencarbonat in 20 ml DMF und 10 ml Wasser wird unter Rühren auf 40° C erwärmt. Dann werden 140 mg (0.20 mmol) Bis(triphenylphosphin)-palladium(II)-chlorid zugegeben. Das Reaktionsgemisch wird auf 80° C erhitzt und bei dieser Temperatur 18 Stunden gerührt. Das Reaktionsgemisch wird auf Raumtemperatur gekühlt und mit Wasser versetzt. Der entstandene Niederschlag wird abgesaugt, mit Wasser gewaschen und im Vakuum getrocknet: 5-[1-(Benzolsulfonyl)-5-brom-pyrrolo[2,3-b]pyridin-3-yl]-2-fluor-benzonitril als hellbraunes Pulver; HPLC/MS (B): 2.77 min, [M+H] 456/458.

Zu einer unter Stickstoff gehaltenen Lösung von 212 mg (2.93 mmol) Cyclopropylmethanol in 0.8 ml DMF wirden unter externer Eiskühlung nacheinander 269 mg (2.39 mmol Kalium-tert-butanolat und eine Suspension von 299 mg (0.67 mmol) 5-[1-(Benzolsulfonyl)-5-brom-pyrrolo[2,3-b]pyridin-3-yl]-2-fluor-benzonitril in 1.6 ml DMF gegeben. Das Reaktionsgemisch wird 20 Stunden bei Raumtemperatur gerührt und anschließend zwischen Wasser und Dichlormethan verteilt. Die organische Phase wird über Natriumsulfat getrocknet und eingedampft. Der Rückstand wird an einer Kieselgelsäule mit Cyclohexan/Ethylacetat chromatographiert: 5-(5-Brom-1H-pyrrolo[2,3-b]pyridin-3-yl)-2-(cyclopropy)methoxy)benzonitril ("A14") als farblose Kristalle; HPLC/MS (B): 2.32 min, [M+H] 398/400.

Eine unter Stickstoff gehaltene Suspension von 2.28 g (5.00 mmol) 5-[1-(Benzol-sulfonyl)-5-brom-pyrrolo[2,3-b]pyridin-3-yl]-2-fluor-benzonitril, 1.14 g (5.50 mmol) 1-Methyl-4-(4,4,5,5-tetramethyl-[1,3,2]dioxaborolan-2-yl)-1H-pyrazol und 504 mg (6.00 mmol) Natriumhydrogencarbonat in 10 ml DMF und 5 ml Wasser wird unter Rühren auf 40° C erwärmt. Dann werden 70 mg (0.10 mmol) Bis(triphenylphosphin)-palladium(II)-chlorid zugegeben. Das Reaktionsgemisch wird auf 80° C erhitzt und bei dieser Temperatur 20 Stunden gerührt. Das Reaktionsgemisch wird auf Raumtemperatur gekühlt und mit Wasser versetzt. Der entstandene Niederschlag wird abgesaugt, mit Wasser gewaschen und an einer Kieselgelsäule mit Cyclohexan / Ethylacetat als Laufmittel chromatographiert: 5-[1-(Benzolsulfonyl)-5-(1-methyl-pyrazol-4-yl)pyrrolo[2,3-b]pyridin-3-yl]-2-fluor-benzonitril als hellgraue Kristalle; HPLC/MS (B): 2.45 min, [M+H] 458.

Eine Lösung von 705 µl (6.00 mmol) 1-Methyl-4-piperidinol in 2 ml DMF wird unter externer Eiskühlung nacheinander mit 449 mg (4.00 mmol) Kalium-tert-butanolat und 457 mg (1.00 mmol) 5-[1-(Benzolsulfonyl)-5-(1-methylpyrazol-4-yl)pyrrolo[2,3-b]pyridin-3-yl]-2-fluor-benzonitril versetzt. Das Reaktionsgemisch wird 4 Tage bei Raumtemperatur gerührt und anschließend zwischen Wasser und Ethylacetat verteilt. Die organische Phase wird über Natriumsulfat getrocknet und eingedampft. Der Rückstand wird an einer Kieselgelsäule mit Methanol/Dichlormethan als Laufmittel chromatographiert: 2-(1-Methyl-piperidin-4-yloxy)-5-[5-(1-methyl-1H-pyrazol-4-yl)-1H-pyrrolo[2,3-b]pyridin-3-yl]-benzonitril ("A13") als hellgelbe Kristalle; HPLC/MS (A): 1.64 min, [M+H]413;
¹H NMR (400 MHz, DMSO-d₆) δ [ppm] = 11.90 (d, *J*=1.9, 1H), 8.53 (d, *J*=2.0, 1H), 8.35 (d, *J*=1.9, 1H), 8.23 (s, 1H), 8.04 (m, 2H), 8.01 (d, *J*=2.4, 1H), 7.89 (d, *J*=2.6, 1H), 7.36 (d, *J*=8.8, 1H), 4.65 (m, 1H), 3.89 (s, 3H), 2.61 (m, 2H), 2.28 (m, 2H), 2.20 (s, 3H), 1.97 (m, 2H), 1.76 (m, 2H).

### Beispiel 8

Die Herstellung von 5-[5-[1-(2-Hydroxyethyl)pyrazol-4-yl]-1H-pyrrolo[2,3-b]pyridin-3-yl]-2-tetrahydropyran-4-yloxy-benzonitril ("A15") erfolgt analog nachstehendem Schema:

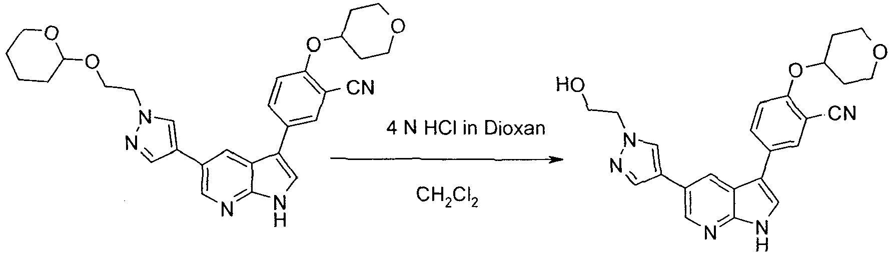

Eine Lösung von 208 mg (0.405 mmol) 2-(Tetrahydro-pyran-4-yloxy)-5-(5-{1-[2-(tetrahydro-pyran-2-yloxy)-ethyl]-1H-pyrazol-4-yl}-1H-pyrrolo[2,3-b]pyridin-3-yl)-benzonitril (hergestellt aus 1-[2-(Tetrahydro-pyran-2-yloxy)-ethyl]-4-(4,4,5,5-tetramethyl-[1,3,2]dioxaborolan-2-yl)-1H-pyrazol) in 7 ml Dichlormethan wird mit 0.5 ml einer 0.4 N Lösung von Chlorwasserstoff in Dioxan versetzt. Das Reaktionsgemisch wird 30 Minuten bei Raumtemperatur belassen und anschließend zwischen Dichlormethan und gesättigter Natriumcarbonat-Lösung verteilt. Die organische Phase wird über Natriumsulfat getrocknet und eingedampft: 5-[5-[1-(2-Hydroxyethyl)pyrazol-4-yl]-1H-pyrrolo[2,3-b]pyridin-3-yl]-2-tetrahydropyran-4-yloxy-benzonitril als gelbes Pulver: HPLC/MS (A): 1.83 min, [M+H] 430;
¹H NMR (400 MHz, DMSO-d₆) δ [ppm] = 11.90 (d, *J*=2.2, 1H), 8.54 (d, *J*=2.0, 1H), 8.36 (d, *J* = 1.9, 1H), 8.25 (s, 1H), 8.06 (d, *J*=2.2, 1H), 8.03 (dd, *J*=8.7, 2.3, 1H), 7.99 (s, 1H), 7.90 (d, *J*=2.7, 1H), 7.41 (d, *J*=8.9, 1H), 4.92 (s, 1H), 4.85 (ddd, *J*=11.8, 7.8, 3.9, 1H), 4.18 (t, *J*=5.7, 2H), 3.89 (m, 2H), 3.79 (t, *J*=5.6, 2H), 3.56 (m, 2H), 2.03 (m, 2H), 1.70 (dtd, *J*=12.3, 8.1, 3.8, 2H).

### Beispiel 9

Die Herstellung von 5-[5-[1-(1-Methyl-4-piperidyl)pyrazol-4-yl]-1H-pyrrolo[2,3-b]pyridin-3-yl]-2-tetrahydropyran-4-yloxy-benzonitril ("A16") erfolgt analog nachstehendem Schema:

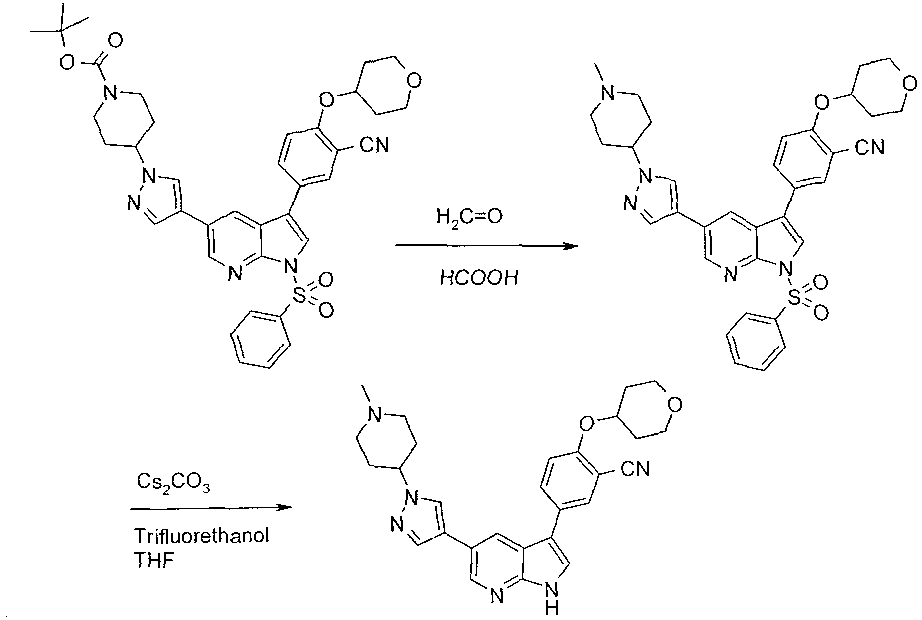

Zu einer Lösung von 201 mg (0.28 mmol) 4-(4-{1-Benzolsulfonyl-3-[3-cyano-4-(tetrahydro-pyran-4-yloxy)-phenyl]-1H-pyrrolo[2,3-b]pyridin-5-yl}-pyrazol-1-yl)-piperidin-1-carbonsäure-tert-butylester (hergestellt aus 4-[4-(4,4,5,5-Tetramethyl-[1,3,2]dioxaborolan-2-yl)-pyrazol-1-yl]-piperidin-1-carbonsäure-tert-butylester) in 1.1 ml Ameisensäure werden 0.07 ml einer 35%igen wässrigen Formaldehyd-Lösung gegeben. Das Reaktionsgemisch wird 4 Stunden bei 80° C gerührt und anschließend im Vakuum eingeengt. Der Rückstand wird zwischen 2 N NaOH und Dichlormethan verteilt. Die organische Phase wird über Natriumsulfat getrocknet und eingedampft. Der Rückstand wird an einer Kieselgelsäule mit Dichlormethan / Methanol als Laufmittel chromatographiert: 5-{1-Benzolsulfonyl-5-[1-(1-methyl-piperidin-4-yl)-1H-pyrazol-4-yl]-1H-pyrrolo[2,3-b]pyridin-3-yl}-2-(tetrahydro-pyran-4-yloxy)-benzonitril als weißes Pulver; HPLC/MS (A): 2.17 min, [M+H] 623.

Eine Lösung von 105 mg (0.168 mmol) 5-{1-Benzolsulfonyl-5-[1-(1-methyl-piperidin-4-yl)-1H-pyrazol-4-yl]-1H-pyrrolo[2,3-b]pyridin-3-yl}-2-(tetrahydro-pyran-4-yloxy)-benzonitril in 1.4 ml THF wird mit 1.4 ml 2,2,2-Trifluorethanol und 164 mg (0.51 mmol) Caesiumcarbonat versetzt und 3 Stunden bei 80° C gerührt. Das Reaktionsgemisch wird zwischen THF und gesättigter Natriumchlorid-Lösung verteilt. Die organische Phase wird über Natriumsulfat getrocknet, eingedampft und der Rückstand an einer Kieselgelsäule mit Dichlormethan/Methanol als Laufmittel chromatographiert: 5-[5-[1-(1-Methyl-4-piperidyl)pyrazol-4-yl]-1H-pyrrolo[2,3-b]pyridin-3-yl]-2-tetrahydropyran-4-yloxy-benzonitril als farblose Kristalle; HPLC/MS (A): 1.83 min, [M+H] 483;
¹H NMR (400 MHz, DMSO-d₆) δ [ppm] = 11.92 (d, *J*=1.7, 1H), 8.55 (d, *J*=2.0, 1H), 8.37 (d, *J*=1.9, 1H), 8.35 (s, 1H), 8.07 (d, *J*=2.3, 1H), 8.03 (dd, *J*=8.7, 2.4, 1H), 7.99 (d, *J*=0.5, 1H), 7.90 (d, *J*=2.5, 1H), 7.41 (d, *J*=8.9, 1H), 4.85 (tt, *J*=7.8, 3.8, 1H), 4.13 (td, *J*=10.0, 4.6, 1H), 3.89 (m, 2H), 3.56 (ddd, *J*=11.4, 8.3, 3.1, 2H), 2.87 (d, *J*=8.5, 2H), 2.22 (s, 3H), 2.03 (m, 8H), 1.69 (dtd, *J*=12.3, 8.2, 3.9, 2H).

### Beispiel 10

Die Herstellung von 5-[5-(4-Methylpiperazin-1-yl)-1H-pyrrolo[2,3-b]pyridin-3-yl]-2-tetrahydropyran-4-yloxy-benzonitril ("A17") erfolgt analog nachstehendem Schema: 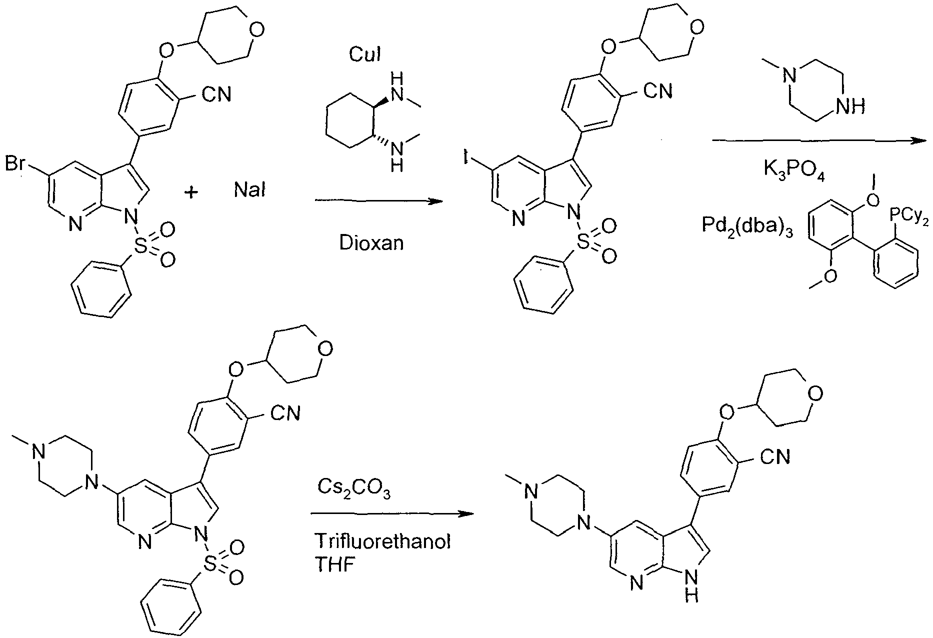 Zu einer unter Stickstoff gehaltenen Suspension von 538 mg (1.00 mmol) 5-[1-(Benzolsulfonyl)-5-brom-pyrrolo[2,3-b]pyridin-3-yl]-2-tetrahydropyran-4-yloxy-benzonitril, 315 mg (2.10 mmol) Natriumiodid und 9.5 mg (0.050) Kupfer(l)iodid in 2.0 ml Dioxan werden 15 µl trans-N,N'-Dimethyl-1,2-cyclohexandiamin gegeben. Das Reaktionsgemisch wird 20 Stunden bei 100° C gerührt. Das Reaktionsgemisch wird auf Raumtemperatur gekühlt und zwischen Wasser und Dichlormethan verteilt. Die organische Phase wird über Natriumsulfat getrocknet, eingedampft und der Rückstand an einer Kieselgelsäule mit Cyclohexan/Ethylacetat als Laufmittel chromatographiert: 5-[1-(Benzolsulfonyl)-5-iod-pyrrolo[2,3-b]pyridin-3-yl]-2-tetrahydropyran-4-yloxy-benzonitril als farbloses Pulver; HPLC/MS (A): 3.34 min, [M+H] 586.

Eine unter Stickstoff gehaltene Suspension von 385 mg (0.662 mmol) 5-[1-(Benzol-sulfonyl)-5-iod-pyrrolo[2,3-b]pyridin-3-yl]-2-tetrahydropyran-4-yloxy-benzonitril, 220 mg (1.04 mmol) Trikaliumphosphat, 0.124 ml (1.11 mmol) 1-Methylpiperazin, 24.3 mg (0.06 mmol) 2-Dicyclohexylphosphino-2',6'-dimethoxybiphenyl und 6.8 mg (0.007 mmol) Tris(dibenzylidenaceton)dipalladium in 1 ml Toluol wird auf 110° C erhitzt und bei dieser Temperatur 4 Tage gerührt. Das Reaktionsgemisch wird zwischen THF und gesättigter Natriumchlorid-Lösung verteilt. Die organische Phase wird über Natriumsulfat getrocknet und eingedampft. Der Rückstand wird an einer Kieselgelsäule mit Methanol/Ethylacetat als Laufmittel chromatographiert: 5-[1-(Benzolsulfonyl)-5-(4-methylpiperazin-1-yl)pyrrolo[2,3-b]pyridin-3-yl]-2-tetrahydropyran-4-yloxy-benzonitril als gelbes Pulver; HPLC/MS (A): 2.11 min, [M+H] 558.

Eine Lösung von 14 mg (0.025 mmol) 5-[1-(Benzolsulfonyl)-5-(4-methylpiperazin-1-yl)pyrrolo[2,3-b]pyridin-3-yl]-2-tetrahydropyran-4-yloxy-benzonitril in 0.3 ml THF wird mit 0.3 ml 2,2,2-Trifluorethanol und 25 mg (0.076 mmol) Caesiumcarbonat versetzt und 3 Stunden bei 80° C gerührt. Das Reaktionsgemisch wird zwischen THF und gesättigter Natriumchlorid-Lösung verteilt. Die organische Phase wird über Natriumsulfat getrocknet, eingedampft und der Rückstand an einer Kieselgelsäule mit Dichlormethan/Methanol als Laufmittel chromatographiert: 5-[5-(4-Methylpiperazin-1-yl)-1H-pyrrolo[2,3-b]pyridin-3-yl]-2-tetrahydropyran-4-yloxy-benzonitril als hellgelbes Pulver; HPLC/MS (A): 1.71 min, [M+H] 418;
¹H NMR (500 MHz, DMSO-d₆) δ[ppm] = 11.71 (s, 1H), 8.14 (d, *J*=2.4, 1H), 7.99 (d, *J*=2.3, 1H), 7.95 (dd, *J*=8.8, 2.3, 1H), 7.82 (d, *J*=2.6, 1H), 7.71 (s, 1H), 7.40 (d, *J*=8.9, 1H), 4.83 (tt, *J*=7.8, 3.8, 1H), 3.88 (m, 2H), 3.55 (ddd, *J*=11.4, 8.3, 3.1, 2H), 3.18 (bs, 3H), 2.6 (breites Signal, 8H), 2.02 (m, 2H), 1.69 (dtd, *J*=12.3, 8.2, 3.8, 2H).

### Beispiel 11

Die Herstellung von
5-[5-[1-(4-Piperidyl)pyrazol-4-yl]-1H-pyrrolo[2,3-b]pyridin-3-yl]-2-tetrahydropyran-4-yloxy-pyridin-3-carbonitril ("A18"),
5-[5-[1-[1-(Benzolsulfonyl)-4-piperidyl]pyrazol-4-yl]-1H-pyrrolo[2,3-b]pyridin-3-yl]-2-tetrahydropyran-4-yloxy-pyridin-3-carbonitril ("A19") und
5-[5-[1-(1-Methyl-4-piperidyl)pyrazol-4-yl]-1H-pyrrolo[2,3-b]pyridin-3-yl]-2-tetrahydropyran-4-yloxy-benzonitril ("A20")
erfolgt analog nachstehendem Schema:

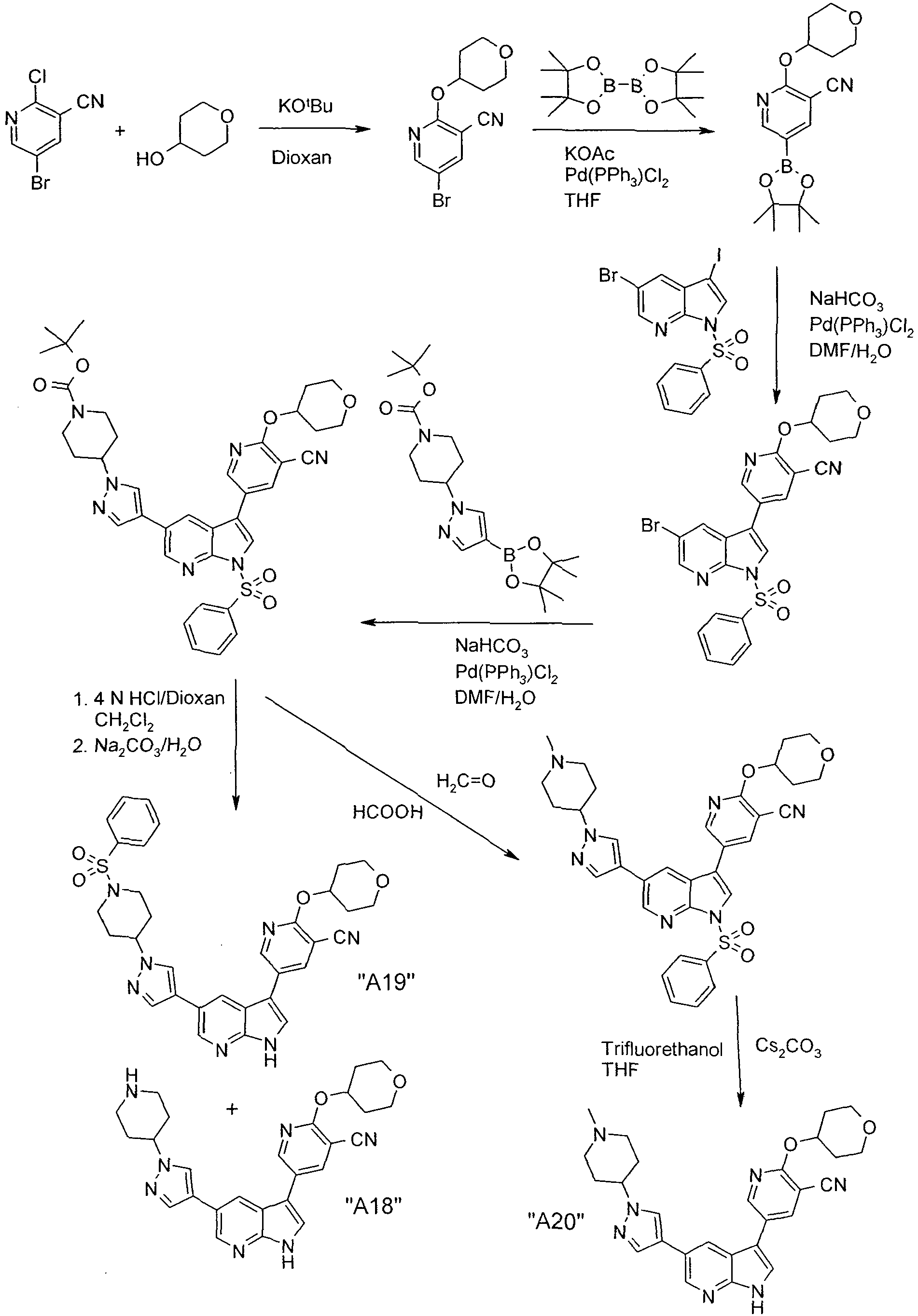

Zu einer unter Stickstoff gehaltenen Lösung von 2.32 g (22.7 mmol) Tetrahydropyran-4-ol in 10 ml Dioxan werden unter externer Eiskühlung 2.55 g (22.7 mmol) Kalium-tert-butanolat gegeben. Dann wird zu der enstandenen Suspension eine Lösung von 2.47 g (11.4 mmol) 5-Brom-2-chlornicotinsäurenitril in 10 ml Dioxan zugetropft und das Reaktionsgemisch wird 90 Minuten bei Raumtemperatur gerührt. Das Reaktionsgemisch wird zwischen Wasser und Dichlormethan verteilt. Die organische Phase wird über Natriumsulfat getrocknet und eingedampft. Der Rückstand wird an einer Kieselgelsäule mit Cyclohexan/Ethylacetat als Laufmittel chromatographiert: 5-Brom-2-(tetrahydro-pyran-4-yloxy)-nicotinsäurenitril als farblose Kristalle; HPLC/MS (B): 2.23 min, [M+H] 283/285.

Eine unter Stickstoff gehaltene Lösung von 1.50 g (5.30 mmol) 5-Brom-2-(tetrahydropyran-4-yloxy)-nicotinsäurenitril und 1.75 g (6.89 mmol) Bis(pinacoloto)dibor in 10 ml THF wird mit 1.56 g (15.9 mmol) trockenem Kaliumacetat versetzt und 40 Minuten bei 40° C gerührt. Anschließend werden 74 mg (0.11 mmol) Bis(triphenylphosphin)-palladium(II)-chlorid zugegeben und das Reaktionsgemisch 16 Stunden bei 80° C gerührt. Das Reaktionsgemisch wird zwischen Wasser und Dichlormethan verteilt. Die organische Phase wird über Natriumsulfat getrocknet und eingedampft. Der Rückstand wird an einer Kieselgelsäule mit Cyclohexan/Ethylacetat als Laufmittel chromatographiert: 2-Tetrahydropyran-4-yloxy-5-(4,4,5,5-tetramethyl-1,3,2-dioxaborolan-2-yl)-pyridin-3-carbonitril als gelbes Öl; HPLC/MS (A): 3.09 min, [M+H] 331.
¹H NMR (400 MHz, DMSO-d₆) δ [ppm] = 8.59 (d, *J*=1.9, 1H), 8.30 (d, *J*=1.9, 1H), 5.40 (tt, *J*=8.3, 4.0, 1H), 3.85 (m, 2H), 3.54 (ddd, *J*=11.7, 8.8, 3.0, 2H), 2.03 (ddd, *J*=7.8, 3.8, 2.1, 2H), 1.70 (m, 2H), 1.30 (s, 12H).

Eine unter Stickstoff gehaltene Suspension von 1.11 g (2.41 mmol) 1-(Benzolsulfonyl)-5-brom-3-iod-pyrrolo[2,3-b]pyridin, 864 mg (2.65 mmol) 2-Tetrahydropyran-4-yloxy-5-(4,4,5,5-tetramethyl-1,3,2-dioxaborolan-2-yl)-pyridin-3-carbonitril und 243 mg (2.89 mmol) Natriumhydrogencarbonat in 5 ml DMF und 2.5 ml Wasser wird unter Rühren auf 40° C erwärmt. Dann werden 34 mg (0.05 mmol) Bis(triphenylphosphin)-palladium(II)-chlorid zugegeben. Das Reaktionsgemisch wird auf 80° C erhitzt und bei dieser Temperatur 18 Stunden gerührt. Das Reaktionsgemisch wird auf Raumtemperatur gekühlt und mit Wasser versetzt. Der entstandene Niederschlag wird abgesaugt, mit Wasser gewaschen und an einer Kieselgelsäule mit Cyclohexan / Ethylacetat als Laufmittel chromatographiert: 5-[1-(Benzolsulfonyl)-5-brom-pyrrolo[2,3-b]pyridin-3-yl]-2-tetrahydropyran-4-yloxy-pyridin-3-carbonitril als beiges Pulver; HPLC/MS (A): 3.33 min, [M+H] 539/541.

Eine unter Stickstoff gehaltene Suspension von 696 mg (1.29 mmol) 5-[1-(Benzol-sulfonyl)-5-brom-pyrrolo[2,3-b]pyridin-3-yl]-2-tetrahydropyran-4-yloxy-pyridin-3-carbonitril, 535 mg (1.42 mmol) 4-[4-(4,4,5,5-Tetramethyl-[1,3,2]dioxaborolan-2-yl)-pyrazol-1-yl]-piperidin-1-carbonsäure-tert-butylester und 130 mg (1.55 mmol) Natriumhydrogencarbonat in 2.6 ml DMF und 1.3 ml Wasser wird unter Rühren auf 40° C erwärmt. Dann werden 18 mg (0.03 mmol) Bis(triphenylphosphin)-palladium(II)-chlorid zugegeben. Das Reaktionsgemisch wird auf 80° C erhitzt und bei dieser Temperatur 4 Tage gerührt. Das Reaktionsgemisch wird auf Raumtemperatur gekühlt und zwischen Wasser und Dichlormethan verteilt. Die organische Phase wird über Natriumsulfat getrocknet und eingedampft. Der Rückstand wird an einer Kieselgelsäule mit Cyclohexan/Ethylacetat chromatographiert: tert-Butyl-4-[4-[1-(benzol-sulfonyl)-3-(5-cyan-6-tetrahydropyran-4-yloxy-3-pyridyl)pyrrolo[2,3-b]pyridin-5-yl]pyrazol-1-yl]piperidin-1-carboxylat als gelblicher Schaum; HPLC/MS (A): 3.37 min, [M+H] 710.

Eine Lösung von 397 mg (0.56 mmol) tert-Butyl-4-[4-[1-(benzolsulfonyl)-3-(5-cyan-6-tetrahydropyran-4-yloxy-3-pyridyl)pyrrolo[2,3-b]pyridin-5-yl]pyrazol-1-yl]piperidin-1-carboxylat in 5 ml Dichlormethan wird mit einer 4 N Lösung von Chlorwasserstoff in Dioxan versetzt und 4 Tage bei Raumtemperatur gerührt. Das Reaktionsgemisch wird zwischen Dichlormethan und gesättigter Natriumcarbonat-Lösung verteilt. Die organische Phase wird über Natriumsulfat getrocknet und eingedampft. Der Rückstand wird durch präparative HPLC getrennt:
5-[5-[1-[1-(Benzolsulfonyl)-4-piperidyl]pyrazol-4-yl]-1H-pyrrolo[2,3-b]pyridin-3-yl]-2-tetrahydropyran-4-yloxy-pyridin-3-carbonitril ("A19") als gelbliches Pulver; HPLC/MS (A): 2.77 min, [M+H] 610;
   ¹H NMR (500 MHz, DMSO-d₆) δ [ppm] = 12.00 (d, *J*=2.2, 1H), 8.89 (d, *J*=2.5, 1H), 8.62 (d, *J*=2.5, 1H), 8.55 (d, *J*=1.9, 1H), 8.42 (d, *J*=1.7, 1H), 8.36 (s, 1H), 8.02 (s, 1H), 7.98 (d, *J*=2.7, 1H), 7.80 (m, 2H), 7.75 (m, 1H), 7.68 (t, *J*=7.5, 2H), 5.38 (tt, *J*=8.3, 3.9, 1H), 4.25 (tt, *J*=11.1, 4.0, 1H), 3.90 (m, 2H), 3.77 (d, *J*=12.1, 2H), 3.56 (ddd, *J*=11.6, 8.9, 2.9, 2H), 2.54 (m, 2H), 2.17 (m, 2H), 2.04 (m, 4H), 1.75 (m, 2H);
5-[5-[1-(4-Piperidyl)pyrazol-4-yl]-1H-pyrrolo[2,3-b]pyridin-3-yl]-2-tetrahydropyran-4-yloxy-pyridin-3-carbonitril Formiat ("A18") als farbloses Pulver; HPLC/MS (A): 1.81 min, [M+H] 470;

Zu einer Lösung von 396 mg (0.56 mmol) tert-Butyl-4-[4-[1-(benzolsulfonyl)-3-(5-cyan-6-tetrahydropyran-4-yloxy-3-pyridyl)pyrrolo[2,3-b]pyridin-5-yl]pyrazol-1-yl]piperidin-1-carboxylat in 2.2 ml Ameisensäure werden 0.13 ml einer 35%igen wässrigen Formaldehyd-Lösung gegeben. Das Reaktionsgemisch wird 4 Stunden bei 80° C gerührt und anschließend im Vakuum eingeengt. Der Rückstand wird zwischen 2 N NaOH und Dichlormethan verteilt. Die organische Phase wird über Natriumsulfat getrocknet und eingedampft. Der Rückstand wird an einer Kieselgelsäule mit Dichlormethan/Methanol als Laufmittel chromatographiert: 5-[1-(Benzolsulfonyl)-5-[1-(1-methyl-4-piperidyl)-pyrazol-4-yl]pyrrolo[2,3-b]pyridin-3-yl]-2-tetrahydropyran-4-yloxy-pyridin-3-carbonitril als weißes Pulver; HPLC/MS (*A*): 2.21 min, [M+H] 624.

Eine Lösung von 208 mg (0.334 mmol) 5-[1-(Benzolsulfonyl)-5-[1-(1-methyl-4-piperidyl)pyrazol-4-yl]pyrrolo[2,3-b]pyridin-3-yl]-2-tetrahydropyran-4-yloxy-pyridin-3-carbonitril in 2.8 ml THF wird mit 2.8 ml 2,2,2-Trifluorethanol und 326 mg (1.00 mmol) Caesiumcarbonat versetzt und 3 Stunden bei 80° C gerührt. Das Reaktionsgemisch wird zwischen THF und gesättigter Natriumchlorid-Lösung verteilt. Die organische Phase wird über Natriumsulfat getrocknet, eingedampft und der Rückstand an einer Kieselgelsäule mit Dichlormethan/Methanol als Laufmittel chromatographiert: 5-[5-[1-(1-Methyl-4-piperidyl)pyrazol-4-yl]-1H-pyrrolo[2,3-b]pyridin-3-yl]-2-tetrahydropyran-4-yloxy-benzonitril ("A20") als farblose Kristalle; HPLC/MS (B): 1.55 min, [M+H] 484;
¹H NMR (400 MHz, DMSO-d₆) δ [ppm] = 12.00 (d, *J*=2.4, 1H), 8.90 (d, *J*=2.5, 1H), 8.63 (d, *J*=2.5, 1H), 8.57 (d, *J*=2.0, 1H), 8.44 (d, *J*=1.9, 1H), 8.39 (s, 1H), 8.03 (s, 1H), 7.98 (d, *J*=2.7, 1H), 5.38 (tt, *J*=8.3, 4.0, 1H), 4.16 (m, 1H), 3.90 (m, 2H), 3.56 (ddd, *J*=11.6, 8.8, 3.0, 2H), 2.93 (d, J=9.0, 2H), 2.27 (s, 3H), 2.17 (s, 2H), 2.06 (m, 6H), 1.75 (m, 2H).

### Beispiel 12

Die Herstellung von 5-[5-(2-Morpholinoethoxy)-1H-pyrrolo[2,3-b]pyridin-3-yl]-2-tetrahydropyran-4-yloxy-benzonitril ("A21") erfolgt analog nachstehendem Schema:

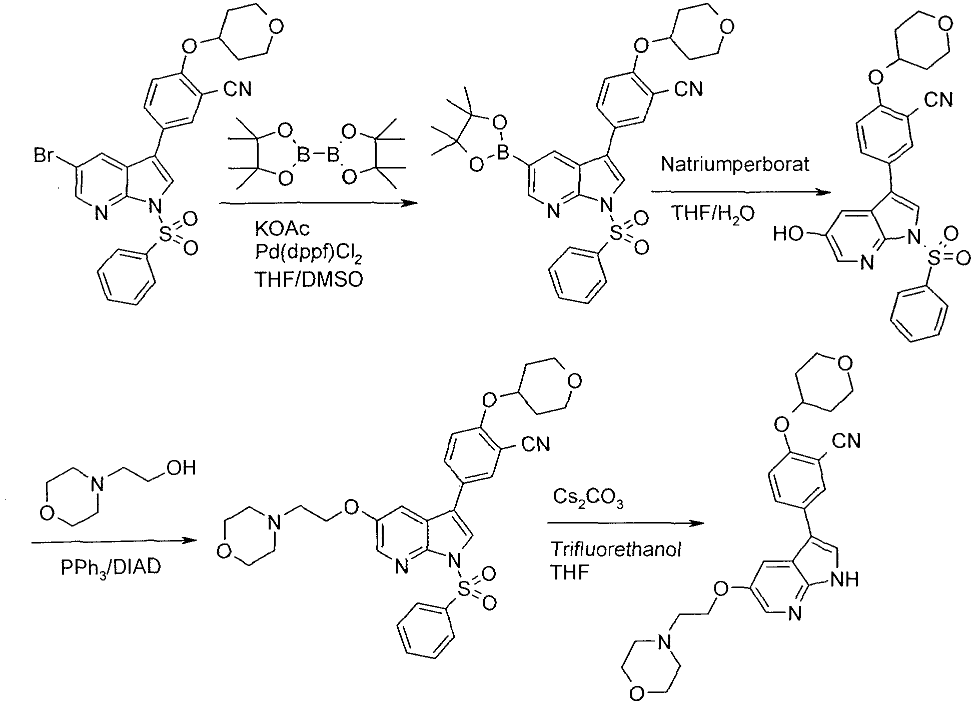

Eine unter Stickstoff gehaltene Lösung von 1.08 g (2.00 mmol) 5-[1-(Benzolsulfonyl)-5-brom-pyrrolo[2,3-b]pyridin-3-yl]-2-tetrahydropyran-4-yloxy-benzonitril und 559 mg (2.20 mmol) Bis(pinacoloto)dibor in 4 ml THF wird mit 2 ml DMSO und 393 mg (4.00 mmol trockenem Kaliumacetat versetzt. Anschließend werden 82 mg (0.10 mmol) [1,1'-bis(diphenylphosphino)ferrocen]palladium(II)dichlorid zugegeben und das Reaktionsgemisch 20 Stunden bei 80° C gerührt. Das Reaktionsgemisch wird zwischen Wasser und Dichlormethan verteilt. Die organische Phase wird über Natriumsulfat getrocknet und eingedampft. Der Rückstand wird an einer Kieselgelsäule mit Cyclohexan/Ethylacetat als Laufmittel chromatographiert: 5-[1-(Benzol-sulfonyl)-5-(4,4,5,5-tetramethyl-1,3,2-dioxaborolan-2-yl)pyrrolo[2,3-b]pyridin-3-yl]-2-tetrahydropyran-4-yloxy-benzonitril als braunes hochviskoses Öl; HPLC/MS (A): 3.50 min, [M+H] 586.

Eine Suspension von 274 mg (0.468 mmol) 5-[1-(Benzolsulfonyl)-5-(4,4,5,5-tetramethyl-1,3,2-dioxaborolan-2-yl)pyrrolo[2,3-b]pyridin-3-yl]-2-tetrahydropyran-4-yloxy-benzonitril in 1 ml THF wird mit 180 mg (1.17 mmol) Natriumperborat-Tetrahydrat und 1 ml Wasser versetzt. Das Gemisch wird 20 Stunden bei Raumtemperatur gerührt, anschließend mit THF verdünnt und filtriert. Das Filtrat wird im Vakuum eingedampft; der Rückstand wird in Wasser aufgenommen und mit 0.5 ml 1N HCl versetzt. Der entstandene Niederschlag wird abgesaugt, mit Wasser gewaschen, an der Luft getrocknet und dann an einer Kieselgelsäule mit Cyclohexan/Ethylacetat als Laufmittel chromatographiert: 5-[1-(Benzolsulfonyl)-5-hydroxy-pyrrolo[2,3-b]pyridin-3-yl]-2-tetrahydropyran-4-yloxy-benzonitril als leicht gelbes Pulver; HPLC/MS (A): 2.72 min, [M+H] 476.

Zu einer Lösung von 139 mg (0.292 mmol) 5-[1-(Benzolsulfonyl)-5-hydroxy-pyrrolo[2,3-b]pyridin-3-yl]-2-tetrahydropyran-4-yloxy-benzonitril, 57 mg (0.44 mmol) N-(2-Hydroxyethyl)-morpholin und 115 mg (0.44 mmol) Triphenylphosphin in 3 ml THF werden 88.5 mg (0.44 mmol) Diisopropylazodicarboxylat zugetropft. Das Reaktionsgemisch wird 2 Stunden bei Raumtemperatur gerührt, eingedampft und anschließend an einer Kieselgelsäule mit Ethylacetat/Methanol als Laufmittel chromatographiert: 5-[1-(Benzolsulfonyl)-5-(2-morpholinoethoxy)pyrrolo[2,3-b]pyridin-3-yl]-2-tetrahydro-pyran-4-yloxy-benzonitril als weißer Schaum; HPLC/MS (A): 2.13 min, [M+H] 589.

Eine Suspension von 125 mg (0.334 mmol) 5-[1-(Benzolsulfonyl)-5-(2-morpholino-ethoxy)pyrrolo[2,3-b]pyridin-3-yl]-2-tetrahydropyran-4-yloxy-benzonitril in 1 ml THF wird mit 1 ml 2,2,2-Trifluorethanol und 207 mg (0.64 mmol) Caesiumcarbonat versetzt und 16 Stunden bei 80° C gerührt. Das Reaktionsgemisch wird eingedampft und der Rückstand an einer Kieselgelsäule mit Dichlormethan/Methanol als Laufmittel chromatographiert: 5-[5-(2-Morpholinoethoxy)-1H-pyrrolo[2,3-b]pyridin-3-yl]-2-tetrahydropyran-4-yloxy-benzonitril als leicht gelbes Pulver; HPLC/MS (A): 1.75 min, [M+H] 449;
¹H NMR (500 MHz, DMSO-d₆) δ [ppm] = 11.79 (d, *J*=1.7, 1H), 8.03 (d, *J*=2.6, 1H), 8.00 (d, *J*=2.3, 1H), 7.97 (dd, *J*=8.8, 2.4, 1H), 7.86 (d, *J*=2.7, 1H), 7.79 (d, *J*=2.5, 1H), 7.39 (d, *J*=8.9, 1H), 4.83 (tt, *J*=7.8, 3.8, 1H), 4.21 (t, *J*=5.8, 2H), 3.88 (m, 2H), 3.56 (m, 6H), 2.73 (t, *J*=5.7, 2H), 2.5 (m, 4H), 2.02 (m, 2H), 1.69 (dtd, *J*=12.3, 8.1, 3.8, 2H).

### Beispiel 13

Die Herstellung von 5-[5-[1-(4-Piperidyl)triazol-4-yl]-1H-pyrrolo[2,3-b]pyridin-3-yl]-2-tetrahydropyran-4-yloxy-benzonitril ("A22") und 5-[5-[1-(1-Methyl-4-piperidyl)triazol-4-yl]-1H-pyrrolo[2,3-b]pyridin-3-yl]-2-tetrahydropyran-4-yloxy-benzonitril ("A23") erfolgt analog nachstehendem Schema:

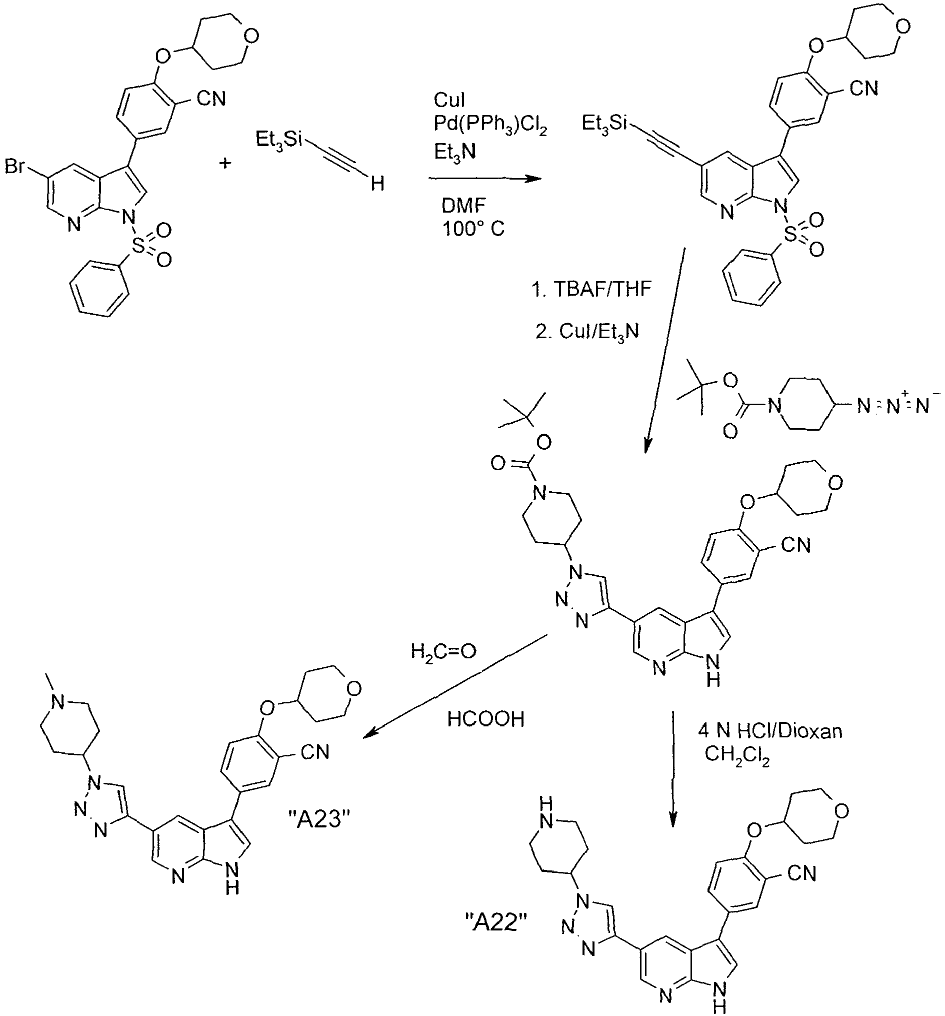

Zu einer unter Stickstoff gehaltenen Lösung von 538 mg (1.00 mmol) 5-[1-(Benzolsulfonyl)-5-brom-pyrrolo[2,3-b]pyridin-3-yl]-2-tetrahydropyran-4-yloxy-benzonitril in 5 ml DMF werden 70.1 mg (0.10 mmol) Bis(triphenylphosphin)-palladium(II)-chlorid, 5.7 mg (0.03 mmol) Kupfer(I)iodid, 416 µl (3.0 mmol) Triethylamin und 351 mg (2.50 mmol) Triethylsilylacetylen gegeben. Das Reaktionsgemisch wird 18 Stunden bei 100° C gerührt. Es wird auf Raumtemperatur gekühlt und zwischen Wasser und Dichlormethan verteilt. Die organische Phase wird mit 0.1 N HCl gewaschen, über Natriumsulfat getrocknet und eingedampft. Der Rückstand wird in tert-Butylmethylether aufgenommen, die Suspension wird erwärmt und rasch abgesaugt. Das Filtrat wird eingedampft und der Rückstand aus Cyclohexan kristallisiert: 5-[1-(Benzolsulfonyl)-5-(2-triethylsilyl-ethinyl)pyrrolo[2,3-b]pyridin-3-yl]-2-tetrahydropyran-4-yloxy-benzonitril als hellgraues Pulver; HPLC/MS (A): 4.08 min, [M+H] 598.

Zu einer unter Stickstoff gehaltenen Lösung von 466 mg (0.779 mmol) 5-[1-(Benzolsulfonyl)-5-(2-triethylsilylethinyl)pyrrolo[2,3-b]pyridin-3-yl]-2-tetrahydropyran-4-yloxy-benzonitril in 5 ml THF werden 1 ml (1 mmol) einer 1 M Lösung von Tetrabutylammoniumfluorid in THF gegeben und die Lösung 1 Stunde bei Raumtemperatur gerührt. Anschließend werden 216 µl (1.56 mmol) Triethylamin, 211 mg (0.934 mmol) tert-Butyl-4-azidopiperidin-1-carboxylat und 7.4 mg (0.04 mmol) Kupfer(I)iodid zugegeben. Das Reaktionsgemisch wird 18 Stunden bei 80° C gerührt und anschließend im Vakuum eingedampft. Der Rückstand wird an einer Kieselgelsäule mit Methanol/Ethylacetat als Laufmittel chromatographiert: tert-Butyl-4-[4-[3-(3-cyano-4-tetrahydropyran-4-ytoxy-phenyl)-1H-pyrrolo[2,3-b]pyridin-5-yl]triazol-1-yl]piperidin-1-carboxylat als cremefarbener Feststoff; HPLC/MS (A): 2.77 min, [M+H] 570.

Eine Lösung von 138 mg (0.24 mmol) tert-Butyl-4-[4-[3-(3-cyano-4-tetrahydropyran-4-yloxy-phenyl)-1H-pyrrolo[2,3-b]pyridin-5-yl]triazol-1-yl]piperidin-1-carboxylat in 1 ml Dichlormethan wird mit 0.5 ml einer 0.4 N Lösung von Chlorwasserstoff in Dioxan versetzt und 16 Stunden bei Raumtemperatur gerührt. Das Reaktionsgemisch wird mit Dichlormethan verdünnt. Der entstandene Niederschlag wird abgesaugt, mit Aceton gewaschen und im Vakuum getrocknet: 5-[5-[1-(4-Piperidyl)-triazol-4-yl]-1H-pyrrolo[2,3-b]pyridin-3-yl]-2-tetrahydropyran-4-yloxy-benzonitril Hydrochlorid als gelber Feststoff: HPLC/MS (A): 1.77 min, [M+H] 470;
¹H NMR (500 MHz, DMSO-d₆) δ [ppm] = 12.11 (d, *J*=1.8, 1H), 9.10 (d, *J*=9.9, 1H), 8.88 (d, *J*=9.8, 1H), 8.84 (s, 1H), 8.78 (s, 1H), 8.66 (d, *J*=1.6, 1H), 8.07 (d, *J*=2.3, 1H), 8.02 (dd, *J*=8.8, 2.3, 1H), 7.97 (d, *J*=2.6, 1H), 7.46 (d, *J*=9.0, 1H), 4.88 (m, 2H), 3.89 (m, 2H), 3.56 (ddd, *J*=11.4, 8.3, 3.1, 2H), 3.45 (d, *J*=13.0, 2H), 3.16 (q, *J*=12.3, 2H), 2.38 (m, 2H), 2.26 (m, 2H), 2.04 (m, 2H), 1.70 (dtd, *J*=12.3, 8.1, 3.8, 2H).

Zu einer Lösung von 138 mg (0.243 mmol) tert-Butyl-4-[4-[3-(3-cyan-4-tetrahydropyran-4-yloxy-phenyl)-1H-pyrrolo[2,3-b]pyridin-5-yl]triazol-1-yl]piperidin-1-carboxylat in 0.5 ml Ameisensäure werden 0.06 ml einer 35%igen wässrigen Formaldehyd-Lösung gegeben. Das Reaktionsgemisch wird 16 Stunden bei 80° C gerührt und anschließend eingedampft. Der Rückstand wird zwischen Dichlormethan und gesättiger Natriumhydrogencarbonat-Lösung verteilt. Die organische Phase wird über Natriumsulfat getrocknet und eingedampft. Der Rückstand wird an einer Kieselgelsäule mit Dichlormethan/Methanol als Laufmittel chromatographiert: 5-[5-[1-(1-Methyl-4-piperidyl)triazol-4-yl]-1H-pyrrolo[2,3-b]pyridin-3-yl]-2-tetrahydropyran-4-yloxy-benzonitril als farbloser Feststoff; HPLC/MS (A): 1.75 min, [M+H] 484;
¹H NMR (500 MHz, DMSO-d₆) δ [ppm] = 12.05 (d, *J*=2.1, 1H), 8.79 (d, *J*=1.9, 1H), 8.77 (s, 1H), 8.62 (d, *J*=1.7, 1H), 8.06 (d, *J*=2.3, 1H), 8.01 (dd, *J*=8.8, 2.3, 1H), 7.96 (d, *J*=2.6, 1H), 7.45 (d, *J*=8.9, 1H), 4.85 (tt, *J*=7.8, 3.8, 1H), 4.52 (m, 1H), 3.89 (m, 2H), 3.55 (ddd, *J*=11.4, 8.3, 3.1, 2H), 2.91 (d, *J*=9.3, 2H), 2.25 (s, 3H), 2.08 (m, 8H), 1.70 (dtd, *J*=12.3, 8.2, 3.8, 2H).

Analog werden die nachstehenden Verbindungen erhalten

| Verbindung Nr. | Struktur und/oder Name | Analog Synthesebeispiel |
|---|---|---|
| "A24" | 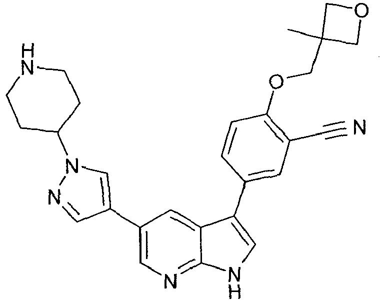 | 6 |
| | 2-[(3-Methyloxetan-3-yl)methoxy]-5-[5-[1-(4-piperidyl)pyrazol-4-yl]-1H-pyrrolo[2,3-b]pyridin-3-yl]benzonitril | |
| | HPLC/MS (A): 1.81 min, [M+H] 469 | |
| "A25" | 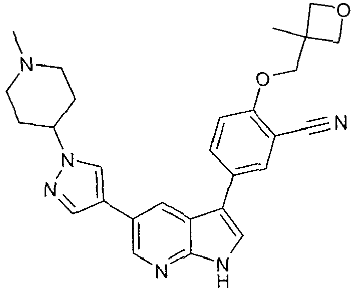 | 9 |
| | 2-[(3-Methyloxetan-3-yl)methoxy]-5-[5-[-(1-methyl-4-piperidyl)pyrazol-4-yl]-1H-pyrrolo[2,3-b]pyridin-3-yl]benzonitril | |
| "A26" | 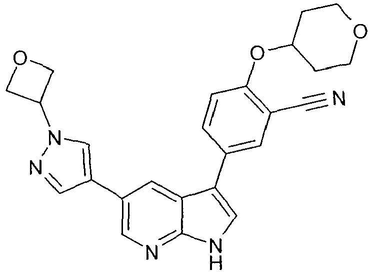 | 4 |
| | 5-[5-[1-(Oxetan-3-yl)pyrazol-4-yl]-1H-pyrrolo[2,3-b]pyridin-3-yl]-2-tetrahydropyran-4-yloxy-benzonitril | |
| | HPLC/MS (C): 1.65 min, [M+H] 442 | |
| "A27" | 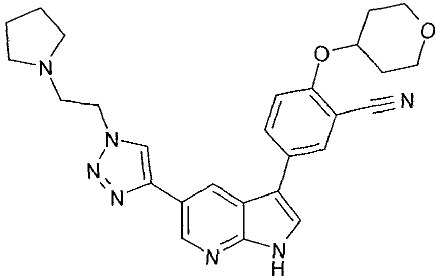 | 13 |
| | 5-[5-[1-(2-Pyrrolidin-1-ylethyl)triazol-4-yl]-1H-pyrrolo[2,3-b]pyridin-3-yl]-2-tetrahydropyran-4-yloxy-benzonitril | |
| | HPLC/MS (C): 1.37 min, [M+H] 484 | |
| "A28" | 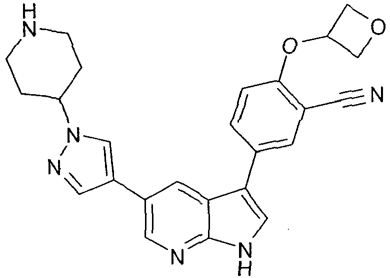 | 6 |
| | 2-(Oxetan-3-yloxy)-5-[5-[1-(4-piperidyl)pyrazol-4-yl]-1H-pyrrolo[2,3-b]pyridin-3-yl]benzonitril | |
| "A29" | 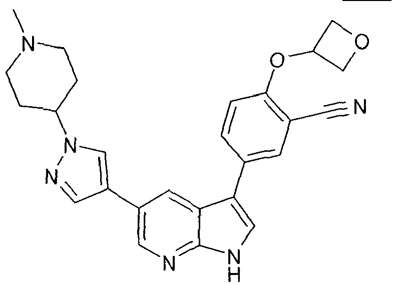 | 9 |
| | 5-[5-[1-(1-Methyl-4-piperidyl)pyrazol-4-yl]-1H-pyrrolo[2,3-b]pyridin-3-yl]-2-(oxetan-3-yloxy)benzonitril | |
| "A30" | 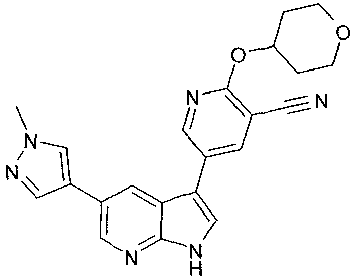 | 11 |
| | 5-[5-(1-Methylpyrazol-4-yl)-1H-pyrrolo[2,3-b]pyridin-3-yl]-2-tetrahydropyran-4-yloxy-pyridine-3-carbonitril | |
| | HPLC/MS (A): 2.29 min, [M+H] 401 | |
| "A33" | 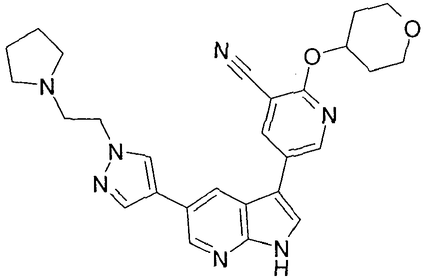 | 11 |
| | 5-{5-[1-(2-Pyrrolidin-1-yl-ethyl)-1H-pyrazol-4-yl]-1H-pyrrolo[2,3-b]pyridin-3-yl}-2-(tetrahydro-pyran-4-yloxy)-nicotinonitril | |
| | HPLC/MS (A): 1.81 min, [M+H] 484 | |
| "A37" | 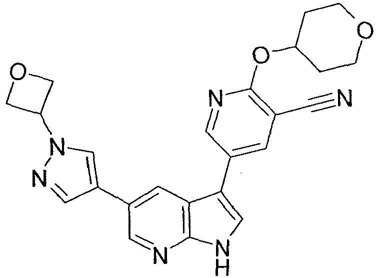 | 11 |
| | 5-[5-(1-Oxetan-3-yl-1H-pyrazol-4-yl)-1H-pyrrolo[2,3-b]pyridin-3-yl]-2-(tetrahydro-pyran-4-yloxy)-nicotinonitril | |
| | HPLC/MS (A): 2.28 min, [M+H] 443 | |
| "A38" | 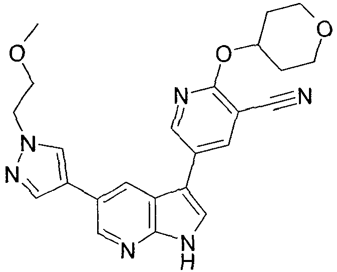 | 11 |
| | 5-{5-[1-(2-Methoxy-ethyl)-1H-pyrazol-4-yl]-1H-pyrrolo[2,3-b]pyridin-3-yl}-2-(tetrahydro-pyran-4-yloxy)-nicotinonitril | |
| | HPLC/MS (C): 1.70 min, [M+H] 445 | |

### Beispiel 14

Die Herstellung von 5-[5-(1-Methyl-1H-pyrazol-4-yl)-1H-pyrrolo[2,3-b]pyridin-3-yl]-2-(oxetan-3-yloxy)-benzonitril ("A31") erfolgt analog nachstehendem Schema:

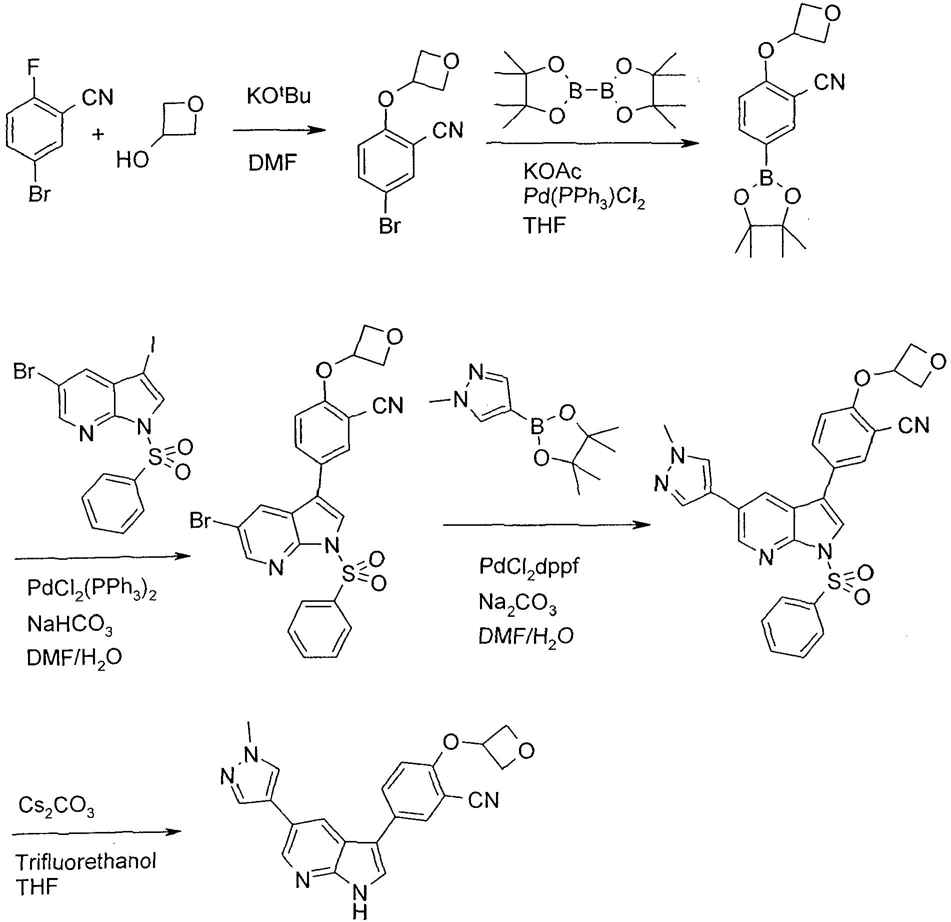

Zu einer unter Stickstoff gehaltenen Lösung von 4.44 g (60.0 mmol) 3-Hydroxyoxetan in 50 ml DMF werden unter externer Eiskühlung 6.73 g (60.0 mmol) Kalium-tert-butanolat gegeben. Dann wird zu der enstandenen gelben Lösung eine Lösung von 10.0 g (50.0 mmol) 5-Brom-2-fluorbenzonitirl in 50 ml DMF zugetropft und das Reaktionsgemisch wird 16 Stunden bei Raumtemperatur gerührt. Das Reaktionsgemisch wird auf 700 ml Wasser gegossen. Der entstandene Niederschlag wird abgesaugt, mit Wasser gewaschen und im Vakuum getrocknet: 5-Brom-2-(oxetan-3-yloxy)-benzonitril als weißes Pulver; HPLC/MS (A): 2.42 min, [M+H] 254/256.

Eine unter Stickstoff gehaltene Lösung von 9.66 g (38.0 mmol) 5-Brom-2-(oxetan-3-yloxy)-benzonitril und 10.1 g (39.9 mmol) Bis(pinacoloto)dibor in 80 ml THF wird mit 7.46 g (76.0 mmol) trockenem Kaliumacetat und 533 mg (0.76 mmol) Bis(triphenylphosphin)-palladium(II)-chlorid versetzt und 16 Stunden bei 80° C gerührt. Das Reaktionsgemisch wird zwischen Wasser und Dichlormethan verteilt. Die organische Phase wird über Natriumsulfat getrocknet und eingedampft. Der Rückstand wird an einer Kieselgelsäule mit Cyclohexan/Ethylacetat als Laufmittel chromatographiert: 2-(Oxetan-3-yloxy)-5-(4,4,5,5-tetramethyl-[1,3,2]dioxaborolan-2-yl)-benzonitril als weiße Kristalle; HPLC/MS (A): 2.83 min, [M+H] 302;
¹H NMR (400 MHz, DMSO-d₆) δ [ppm] 7.91 (d, J=1.6, 1H), 7.86 (dd, J=8.5, 1.5, 1H), 6.90 (d, *J*=8.5, 1H), 5.48 (m, 1H), 4.97 (m, 2H), 4.58 (dd, *J*=7.8, 4.7, 2H), 1.29 (s, 12H).

Eine unter Stickstoff gehaltene Suspension von 4.63 g (10.0 mmol) 1-(Benzolsulfonyl)-5-brom-3-iod-pyrrolo[2,3-b]pyridin, 3.31 g (11.0 mmol) 2-(Oxetan-3-yloxy)-5-(4,4,5,5-tetramethyl-[1,3,2]dioxaborolan-2-yl)-benzonitril und 1.01 g (12.0 mmol) Natriumhydrogencarbonat in 20 ml DMF und 10 ml Wasser wird unter Rühren auf 80° C erwärmt. Dann werden 140 mg (0.20 mmol) Bis(triphenylphosphin)-palladium(II)-chlorid zugegeben. Das Reaktionsgemisch wird 18 Stunden bei 80° C gerührt. Das Reaktionsgemisch wird auf Raumtemperatur gekühlt und mit Wasser versetzt. Der entstandene Niederschlag wird abgesaugt, mit Wasser gewaschen und im Vakuum getrocknet. Das Rohprodukt wird aus Isopropanol kristallisiert: 5-(1-Benzolsulfonyl-5-brom-1H-pyrrolo[2,3-b]pyridin-3-yl)-2-(oxetan-3-yloxy)-benzonitril als hellgraues Pulver; HPLC/MS (A): 3.14 min, [M+H] 510/512.

Eine unter Stickstoff gehaltene Suspension von 510 mg (1.00 mmol) 5-(1-Benzol-sulfonyl-5-brom-1H-pyrrolo[2,3-b]pyridin-3-yl)-2-(oxetan-3-yloxy)-benzonitril, 229 g (1.10 mmol) 1-Methyl-4-(4,4,5,5-tetramethyl-[1,3,2]dioxaborolan-2-yl)-1H-pyrazol und 127 mg (1.20 mmol) Natriumcarbonat in 2 ml DMF und 1 ml Wasser wird unter Rühren auf 80° C erwärmt. Dann werden 16 mg (0.02 mmol) [1,1'-bis(diphenylphosphino)-ferrocen]palladium(II)dichlorid zugegeben. Das Reaktionsgemisch wird 20 Stunden bei 80° C gerührt. Das Reaktionsgemisch wird auf Raumtemperatur gekühlt und mit Wasser versetzt. Der entstandene Niederschlag wird abgesaugt, mit Wasser gewaschen und an einer Kieselgelsäule mit Ethylacetat/Methanol als Laufmittel chromatographiert: 5-[1-Benzolsulfonyl-5-(1-methyl-1H-pyrazol-4-yl)-1H-pyrrolo[2,3-b]pyridin-3-yl]-2-(oxetan-3-yloxy)-benzonitril als hellbraunes Glas; HPLC/MS (C): 1.90 min, [M+H] 512.

Eine Lösung von 338 mg (0.66 mmol) 5-[1-Benzolsulfony)-5-(1-methy)-1H-pyrazol-4-yl)-1H-pyrrolo[2,3-b]pyridin-3-yl]-2-(oxetan-3-yloxy)-benzonitril in 2 ml THF wird mit 2 ml 2,2,2-Trifluorethanol und 642 mg (1.97 mmol) Caesiumcarbonat versetzt und 16 Stunden bei 80° C gerührt. Das Reaktionsgemisch wird eingedampft und der Rückstand wird an einer Kieselgelsäule mit Dichlormethan/Methanol als Laufmittel chromatographiert: 5-[5-(1-Methyl-1H-pyrazol-4-yl)-1H-pyrrolo[2,3-b]pyridin-3-yl]-2-(oxetan-3-yloxy)-benzonitril als gelbliches Pulver; HPLC/MS (C): 1.58 min, [M+H] 372; ¹H NMR (500 MHz, DMSO-d₆) δ [ppm] 11.92 (d, *J*=2.0, 1H), 8.52 (d, *J*=2.0, 1H), 8.35 (d, *J*=1.9, 1H), 8.22 (s, 1H), 8.12 (d, *J*=2.3, 1H), 8.02 (dd, *J*=8.7, 2.3, 1H), 7.97 (s, 1H), 7.91 (d, *J*=2.7, 1H), 6.94 (d, *J*=8.8, 1H), 5.50 (p, *J*=5.7, 1H), 5.00 (t, *J*=6.9, 2H), 4.64 (dd, *J*=7.7, 4.9, 2H), 3.89 (s, 3H).

Analog erhält man 2-(3-Methyl-oxetan-3-ylmethoxy)-5-[5-(1-methyl-1H-pyrazol-4-yl)-1H-pyrrolo[2,3-b]pyridin-3-yl]-benzonitril ("A32"), beiges Pulver; HPLC/MS (*A*): 2.26 min, [M+H] 400;
¹H NMR (500 MHz, DMSO-d₆) δ [ppm] 11.91 (s, 1H), 8.53 (d, *J*=2.0, 1H), 8.36 (d, *J*=2.0, 1H), 8.23 (s, 1H), 8.07 (dq, *J*=4.6, 2.3, 2H), 7.98 (s, 1H), 7.91 (d, *J*=1.2, 1H), 7.38 (d, *J*=9.5, 1H), 4.55 (d, *J*=5.8, 2H), 4.35 (d, *J*=5.9, 2H), 4.29 (s, 2H), 3.89 (s, 3H), 1.43 (s, 3H).

### Beispiel 15

Die Herstellung von 2-(Oxetan-3-yloxy)-5-[5-(1-oxetan-3-yl-1H-pyrazol-4-yl)-1H-pyrrolo[2,3-b]pyridin-3-yl]-benzonitril ("A34") erfolgt analog nachstehendem Schema:

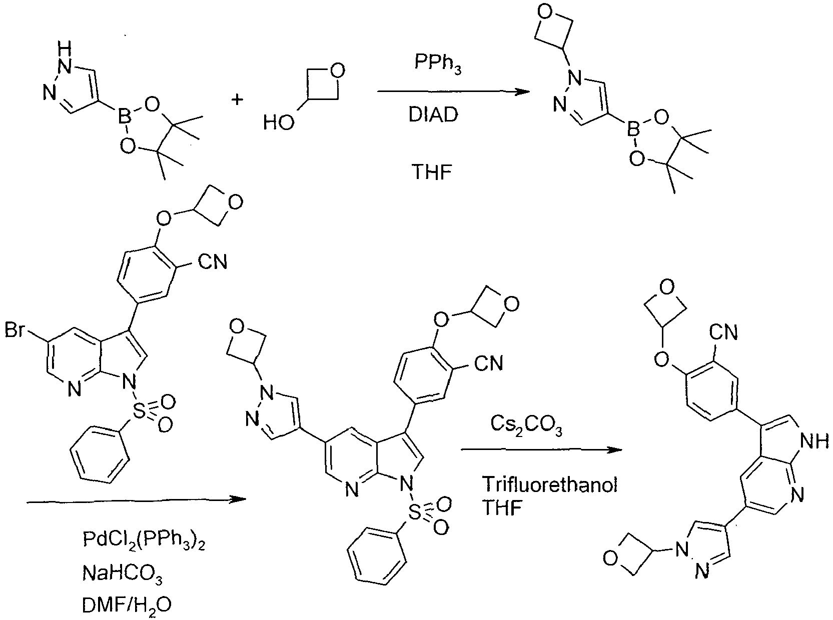

Zu einer Lösung von 3.88 g (20.0 mmol) Pyrazol-4-boronsäure-pinacol-ester, 1.78 g (48.0 mmol) Oxetan-3-ol und 6.29 g (24.0 mmol) Triphenylphosphin in 40 ml THF werden 4.76 ml (24.0 mmol) Diisopropylazodicarboxylat zugetropft. Das Reaktionsgemisch wird 16 Stunden bei Raumtemperatur gerührt. Dann werden nochmals 1.78 g (48.0 mmol) Oxetan-3-ol, 6.29 g (24.0 mmol) Triphenylphosphin und 3.00 ml (15.1 mmol) Diisopropylazodicarboxylat zugegeben und das Reaktionsgemisch wird 3 Tage bei Raumtemperatur gerührt. Das Reaktionsgemisch wird eingedampft und der Rückstand in Cyclohexan aufgenommen. Der entstandene Niederschlag wird abgesaugt und mit Cyclohexan gewaschen. Das Filtrat wird eingedampft und der Rückstand an einer Kieselgelsäule mit Cyclohexan/Ethylacetat als Laufmittel chromatographiert: 1-Oxetan-3-yl-4-(4,4,5,5-tetramethyl-[1,3,2]dioxaborolan-2-yl)-1H-pyrazol als gelbes Öl; HPLC/MS (A): 2.10 min, [M+H] 251; ¹H NMR (400 MHz, DMSO-d₆) δ [ppm] 8.07 (s, 1H), 7.72 (s, 1H), 5.60 (p, *J*=6.9, 1H), 4.89 (m, 4H), 1.25 (s, 12H).

Eine unter Stickstoff gehaltene Suspension von 357 mg (0.70 mmol) 5-(1-Benzol-sulfonyl-5-brom-1H-pyrrolo[2,3-b]pyridin-3-yl)-2-(oxetan-3-yloxy)-benzonitril, 385 mg (1.54 mmol) 1-Oxetan-3-yl-4-(4,4,5,5-tetramethyl-[1,3,2]dioxaborolan-2-yl)-1H-pyrazol und 141 mg (1.68 mmol) Natriumhydrogencarbonat in 2 ml DMF und 1 ml Wasser wird unter Rühren auf 80° C erwärmt. Dann werden 20 mg (0.028 mmol) Bis(triphenylphosphin)-palladium(II)-chlorid zugegeben. Das Reaktionsgemisch wird 44 Stunden bei 80° C gerührt. Das Reaktionsgemisch wird auf Raumtemperatur gekühlt und mit Wasser versetzt. Der entstandene Niederschlag wird abgesaugt, mit Wasser gewaschen und an einer Kieselgelsäule mit Ethylacetat/Methanol als Laufmittel chromatographiert: 5-[1-Benzolsulfonyl-5-(1-oxetan-3-yl-1H-pyrazol-4-yl)-1H-pyrrofo[2,3-bjpyridin-3-yl]-2-(oxetan-3-yloxy)-benzonitril als gelblicher Schaum; HPLC/MS (C): 1.89 min, [M+H] 554.

Eine Lösung von 249 mg (0.45 mmol) 5-[1-Benzolsulfonyl-5-(1-oxetan-3-yl-1H-pyrazol-4-yl)-1H-pyrrolo[2,3-b]pyridin-3-yl]-2-(oxetan-3-yloxy)-benzonitril in 3 ml THF wird mit 2 ml 2,2,2-Trifluorethanol und 441 mg (1.97 mmol) Caesiumcarbonat versetzt und 16 Stunden bei 80° C gerührt. Das Reaktionsgemisch wird eingedampft, in Wasser aufgenommen und filtriert. Der Rückstand wird mit Wasser gewaschen, getrocknet und aus Dimethylsulfoxid umkristallisiert: 2-(Oxetan-3-yloxy)-5-[5-(1-oxetan-3-yl-1H-pyrazol-4-yl)-1H-pyrrolo[2,3-b]pyridin-3-yl]-benzonitril als weiße Kristalle; HPLC/MS (C): 1.59 min, [M+H] 414;
¹H NMR (500 MHz, DMSO-d₆) δ [ppm] 11.95 (s, 1H), 8.57 (d, *J*=1.7, 1H), 8.49 (s, 1H), 8.41 (d, *J*=1.6, 1H), 8.15 (s, 1H), 8.13 (d, *J*=2.1, 1H), 8.03 (dd, *J*=8.7, 2.2, 1H), 7.92 (d, *J*=2.3, 1H), 6.95 (d, *J*=8.8, 1H), 5.61 (p, *J*=7.0, 1H), 5.50 (m, 1H), 5.00 (m, 2H), 4.96 (m, 4H), 4.64 (dd, *J*=7.3, 5.0, 2H).

### Beispiel 16

Die Herstellung von 2-(3-Methyl-oxetan-3-ylmethoxy)-5-[5-(1-oxetan-3-yl-1H-pyrazol-4-yl)-1H-pyrrolo[2,3-b]pyridin-3-yl]-benzonitril ("A35") und 2-(3-Methyl-oxetan-3-ylmethoxy)-5-[5-(1H-pyrazol-4-yl)-1H-pyrrolo[2,3-b]pyridin-3-yl]-benzonitril ("A36") erfolgt analog nachstehendem Schema:

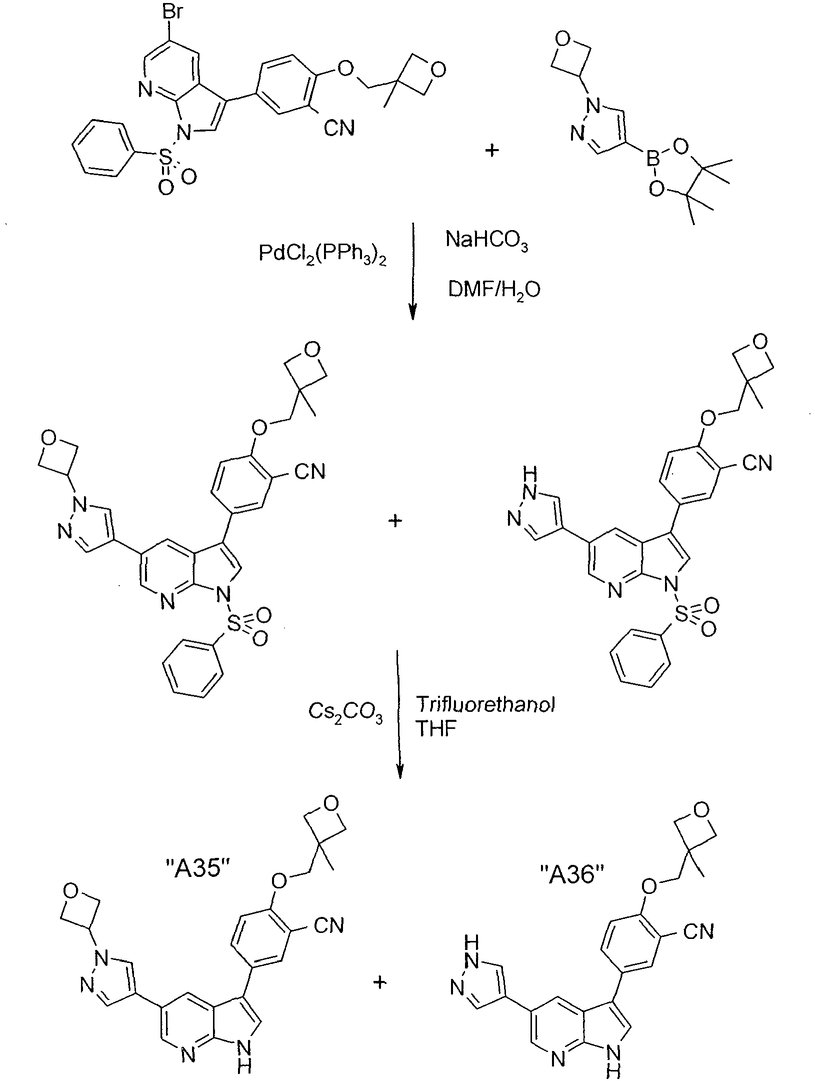

Eine unter Stickstoff gehaltene Suspension von 377 mg (0.70 mmol) 5-(1-Benzol-sulfonyl-5-bromo-1H-pyrrolo[2,3-b]pyridin-3-yl)-2-(3-methyl-oxetan-3-ylmethoxy)-benzonitril, 367 mg (1.47 mmol) 1-Oxetan-3-yl-4-(4,4,5,5-tetramethyl-[1,3,2]dioxa-borolan-2-yl)-1H-pyrazol und 118 mg (1.40 mmol) Natriumhydrogencarbonat in 1.4 ml DMF und 0.7 ml Wasser wird unter Rühren auf 40° C erwärmt. Dann werden 20 mg (0.03 mmol) Bis(triphenylphosphin)-palladium(II)-chlorid zugegeben. Das Reaktionsgemisch wird auf 80° C erhitzt und bei dieser Temperatur 44 Stunden gerührt. Das Reaktionsgemisch wird auf Raumtemperatur gekühlt und mit Wasser versetzt. Der entstandene Niederschlag wird abgesaugt, mit Wasser gewaschen und an einer Kieselgelsäule mit Ethylacetat/Methanol als Laufmittel chromatographiert: Mischung aus 5-[1-Benzolsulfonyl-5-(1-oxetan-3-yl-1H-pyrazol-4-yl)-1H-pyrrolo[2,3-b]pyridin-3-yl]-2-(3-methyl-oxetan-3-ylmethoxy)-benzonitril {HPLC/MS (B): 2.82 min, [M+H] 582} und 5-[1-Benzolsulfonyl-5-(1H-pyrazol-4-yl)-1H-pyrrolo[2,3-b]pyridin-3-yl]-2-(3-methyl-oxetan-3-ylmethoxy)-benzonitril {HPLC/MS (B): 2.71 min, [M+H] 526} als weißer Schaum; Menge 371 mg.

Das so erhaltene Gemisch wird in 2.6 ml THF und 2.6 ml 2,2,2-Trifluorethanol suspendiert und mit 530 mg (1.63 mmol) Caesiumcarbonat versetzt. Die Suspension wird 3 Stunden bei 80° C gerührt. Das Reaktionsgemisch wird eingedampft und der Rückstand wird an einer Kieselgelsäule mit Dichlormethan/Methanol als Laufmittel chromatographiert. Man erhält zwei Produkte:
2-(3-Methyl-oxetan-3-ylmethoxy)-5-[5-(1-oxetan-3-yl-1H-pyrazol-4-yl)-1H-pyrrolo[2,3-b]pyridin-3-yl]-benzonitril als weißes Pulver; HPLC/MS (B): 2.26 min, [M+H] 442;
   ¹H NMR (400 MHz, DMSO-d₆) δ [ppm] 11.94 (s, 1H), 8.57 (d, *J*=2.0, 1H), 8.49 (s, 1H), 8.41 (d, *J*=2.0, 1H), 8.15 (s, 1H), 8.09 (m, 3H), 7.92 (d, *J*=1.3, 1H), 7.38 (d, *J*=9.5, 1H), 5.61 (p, *J*=6.9, 1H), 4.96 (m, 4H), 4.55 (d, *J*=5.8, 2H), 4.35 (d, *J*=5.9, 2H), 4.29 (s, 2H), 1.43 (s, 3H);
2-(3-Methyl-oxetan-3-ylmethoxy)-5-[5-(1H-pyrazol-4-yl)-1H-pyrrolo[2,3-b]pyridin-3-yl]-benzonitril als beiges Pulver; HPLC/MS (B): 2.14 min, [M+H] 386;
   ¹H NMR (400 MHz, DMSO-d₆) δ [ppm] 11.90 (d, *J*=2.1, 1H), 8.57 (d, *J*=1.9, 1H), 8.40 (d, *J*=1.8, 1H), 8.17 (bs, 2H), 8.08 (m, 2H), 7.91 (d, *J*=2.6, 1H), 7.38 (d, *J*=9.5, 1H), 4.55 (d, *J*=5.8, 2H), 4.35 (d, *J*=5.9, 2H), 4.29 (s, 2H), 1.43 (s, 3H).

IC₅₀-Werte von erfindungsgemäßen TBK1- und IKKε-hemmenden Verbindungen

| Verbindung Nr. | TBK1-Enzymassay IC₅₀ [nM] | IKKε-Enzymassay IC₅₀ [nM] | TBK1 + IKK_{ε}-Zellassay IC₅₀ [nM] |
|---|---|---|---|
| "A1" | A | A | C |
| "A2" | B | B | |
| "A3" | C | C | |
| "A4" | A | A | B |
| "A5" | C | C | |
| "A6" | A | B | |
| "A7" | A | A | B |
| "A8" | A | A | C |
| "A9" | A | A | B |
| "A10" | A | A | A |
| "A11" | A | B | |
| "A12" | A | A | B |
| "A13" | A | A | C |
| "A14" | C | C | |
| "A15" | A | A | A |
| "A16" | A | A | A |
| "A17" | B | B | C |
| "A18" | A | A | B |
| "A19" | B | B | |
| "A20" | A | A | B |
| "A21" | A | A | B |
| "A22" | A | A | B |
| "A23" | A | A | B |
| "A24" | A | A | B |
| "A26" | A | A | A |
| "A27" | A | A | B |
| "A30" | A | A | B |
| "A31" | A | A | C |
| "A32" | A | A | B |
| "A33" | A | A | B |
| "A34" | A | A | C |
| "A35" | A | A | B |
| "A36" | A | A | C |
| "A37" | A | A | C |
| "A38" | A | A | B |

| | | | |
|---|---|---|---|
| IC₅₀: < 0.3 µM = A 0.3-3µM=B 3-50 µM= C | | | |

Die nachfolgenden Beispiele betreffen Arzneimittel:

### Beispiel A: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2H₂O, 28,48g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2kg Wirkstoff werden in üblicher Weise in Hartgelatinekapseln gefüllt, so daß jede Kapsel 20 mg des Wirkstoffs enthält.

### Beispiel H: Ampullen

Eine Lösung von 1 kg eines erfindungsgemäßen Wirkstoffes in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Verbindung Nr. | Name |
|---|---|
| "A4" | 5-[5-(1-Methylpyrazol-4-yl)-1H-pyrrolo[2,3-b]pyridin-3-yl]-2-tetrahydropyran-4-yloxy-benzonitril |
| "A6" | 5-[5-(4-Cyanphenyl)-1H-pyrrolo[2,3-b]pyridin-3-yl]-2-tetrahydropyran-4-yloxy-benzonitril |
| "A7" | 5-[5-[1-(2-Morpholinoethyl)pyrazol-4-yl]-1H-pyrrolo[2,3-b]pyridin-3-yl]-2-tetrahydropyran-4-yloxy-benzonitril |
| "A8" | 5-[5-(4-Morpholinophenyl)-1H-pyrrolo[2,3-b]pyridin-3-yl]-2-tetrahydropyran-4-yloxy-benzonitril |
| "A9" | 5-[5-[1-(2-Pyrrolidin-1-ylethyl)pyrazol-4-yl]-1H-pyrrolo[2,3-b]pyrid in-3-yl]-2-tetrahyd ropyran-4-yloxy-benzon itril |
| "A10" | 5-[5-[1-(2-Methoxyethyl)pyrazol-4-yl]-1H-pyrrolo[2,3-b]pyridin-3-yl]-2-tetrahydropyran-4-yloxy-benzonitril |
| "A11" | 5-[2-Methyl-5-(1-methylpyrazol-4-yl)-1H-pyrrolo[2,3-b]pyridin-3-yl]-2-tetrahydropyran-4-yloxy-benzonitril |
| "A12" | 5-[5-(1-Piperidin-4-yl-1H-pyrazol-4-yl)-1H-pyrrolo[2,3-b]pyridin-3-yl]-2-(tetrahydro-pyran-4-yloxy)-benzonitril |
| "A13" | 2-(1-Methyl-piperidin-4-yloxy)-5-[5-(1-methyl-1H-pyrazol-4-yl)-1H-pyrrolo[2,3-b]pyridin-3-yl]-benzonitril |
| "A14" | 5-(5-Brom-1H-pyrrolo[2,3-b]pyridin-3-yl)-2-(cyclopropylmethoxy)benzonitril |
| "A15" | 5-[5-[1-(2-Hydroxyethyl)pyrazol-4-yl]-1H-pyrrolo[2,3-b]pyridin-3-yl]-2-tetrahydropyran-4-yloxy-benzonitril |
| "A16" | 5-[5-[1-(1-Methyl-4-piperidyl)pyrazol-4-yl]-1H-pyrrolo[2,3-b]pyridin-3-yl]-2-tetrahydropyran-4-yloxy-benzonitril |
| "A17" | 5-[5-(4-Methylpiperazin-1-yl)-1H-pyrrolo[2,3-b]pyridin-3-yl]-2-tetrahydropyran-4-yloxy-benzonitril |
| "A18" | 5-[5-[1-(4-Piperidyl)pyrazol-4-yl]-1H-pyrrolo[2,3-b]pyridin-3-yl]-2-tetrahydropyran-4-yloxy-pyridin-3-carbonitril |
| "A19" | 5-[5-[1-[1-(Benzolsulfonyl)-4-piperidyl]pyrazol-4-yl]-1H-pyrrolo[2,3-b]pyridin-3-yl]-2-tetrahydropyran-4-yloxy-pyridin-3-carbonitril |
| "A20" | 5-[5-[1-(1-Methyl-4-piperidyl)pyrazol-4-yl]-1H-pyrrolo[2,3-b]pyridin-3-yl]-2-tetrahydropyran-4-yloxy-benzonitril |
| "A21" | 5-[5-(2-Morpholinoethoxy)-1H-pyrrolo[2,3-b]pyridin-3-yl]-2-tetrahydropyran-4-yloxy-benzonitril |
| "A22" | 5-[5-[1-(4-Piperidyl)triazol-4-yl]-1H-pyrrolo[2,3-b]pyridin-3-yl]-2-tetrahydropyran-4-yloxy-benzonitril |
| "A23" | 5-[5-[1-(1-Methyl-4-piperidyl)triazol-4-yl]-1H-pyrrolo[2,3-b]pyridin-3-yl]-2-tetrahydropyran-4-yloxy-benzonitril |
| "A24" | 2-[(3-Methyloxetan-3-yl)methoxy]-5-[5-[1-(4-piperidyl)pyrazol-4-yl]-1H-pyrrolo[2,3-b]pyridin-3-yl]benzonitril |
| "A25" | 2-[(3-Methyloxetan-3-yl)methoxy]-5-[5-[1-(1-methyl-4-piperidyl)pyrazol-4-yl]-1H-pyrrolo[2,3-b]pyridin-3-yl]benzonitril |
| "A26" | 5-[5-[1-(Oxetan-3-yl)pyrazol-4-yl]-1H-pyrrolo[2,3-b]pyridin-3-yl]-2-tetrahydropyran-4-yloxy-benzonitril |
| "A27" | 5-[5-[1-(2-Pyrrolidin-1-ylethyl)triazol-4-yl]-1H-pyrrolo[2,3-b)pyridin-3-yl]-2-tetrahydropyran-4-yloxy-benzonitril |
| "A28" | 2-(Oxetan-3-yloxy)-5-[5-[1-(4-piperidyl)pyrazol-4-yl]-1H-pyrrolo[2,3-b]pyridin-3-yl]benzonitril |
| "A29" | 5-[5-[1-(1-Methyl-4-piperidyl)pyrazol-4-yl]-1H-pyrrolo[2,3-b]pyridin-3-yl]-2-(oxetan-3-yloxy)benzonitril |
| "A30" | 5-[5-(1-Methylpyrazol-4-yl)-1H-pyrrolo[2,3-b]pyridin-3-yl]-2-tetrahydropyran-4-yloxy-pyridine-3-carbonitril |
| "A31" | 5-[5-(1-Methyl-1H-pyrazol-4-yl)-1H-pyrrolo[2,3-b]pyridin-3-yl]-2-(oxetan-3-yloxy)-benzonitril |
| "A32" | 2-(3-Methyl-oxetan-3-ylmethoxy)-5-[5-(1-methyl-1H-pyrazol-4-yl)-1H-pyrrolo[2,3-b]pyridin-3-yl]-benzonitril |
| "A33" | 5-{5-[1-(2-Pyrrolidin-1-yl-ethyl)-1H-pyrazol-4-yl]-1H-pyrrolo[2,3-b]pyridin-3-yl}-2-(tetrahydro-pyran-4-yloxy)-nicotinonitril |
| "A34" | 2-(Oxetan-3-yloxy)-5-[5-(1-oxetan-3-yl-1H-pyrazol-4-yl)-1H-pyrrolo[2,3-b]pyridin-3-yl]-benzonitril |
| "A35" | 2-(3-Methyl-oxetan-3-ylmethoxy)-5-[5-(1-oxetan-3-yl-1H-pyrazol-4-yl)-1H-pyrrolo[2,3-b]pyridin-3-yl]-benzonitril |
| "A36" | 2-(3-Methyl-oxetan-3-ylmethoxy)-5-[5-(1H-pyrazol-4-yl)-1H-pyrrolo[2,3-b]pyridin-3-yl]-benzonitril |
| "A37" | 5-[5-(1-Oxetan-3-yl-1H-pyrazol-4-yl)-1H-pyrrolo[2,3-b]pyridin-3-yl]-2-(tetrahydro-pyran-4-yloxy)-nicotinonitril |
| "A38" | 5-{5-[1-(2-Methoxy-ethyl)-1H-pyrazol-4-yl]-1H-pyrrolo[2,3-b]pyridin-3-yl}-2-(tetrahydro-pyran-4-yloxy)-nicotinonitril |
sowie ihre pharmazeutisch verwendbaren Salze, Tautomere und Stereoisomere, einschließlich deren Mischungen in allen Verhältnissen.

2. Arzneimittel enthaltend mindestens eine Verbindung gemäß Anspruch 1 und/oder ihre pharmazeutisch verwendbaren Salze, Tautomere und Stereoisomere, einschließlich deren Mischungen in allen Verhältnissen, sowie gegebenenfalls Träger- und/oder Hilfsstoffe.

3. Verbindungen gemäß Anspruch 1 sowie ihre pharmazeutisch verwendbaren Salze, Tautomere und Stereoisomere, einschließlich deren Mischungen in allen Verhältnissen, zur Verwendung für die Behandlung von Krebs, septischem Schock, primärem Offenwinkelglaukom (POAG), Hyperplasie, Atherosklerose, Retinopathie, Osteoarthritis, Endometriose, chronischer Entzündung, neurodegenerativen Erkrankungen, rheumatoide Arthritis (RA), systemischer Lupus erythematosus (SLE), Sjörgrens Syndrom, Aicardi-Goutieres Syndrom Lupus Chilblain, retinale Vasculopathie, cerebrale Leukodystrophie (RVCL), systemische Sklerosis, Myositis, Psoriasis, chronisch obstruktive pulmonare Krankheit (CPD), endzündliche Darmkrankheit (IBD), Fettsucht, Insulinresistenz, Typ 2 Diabetes (NIDDM) und/oder metabolisches Syndrom.

4. Verbindungen gemäß Anspruch 1 und/oder ihre physiologisch unbedenklichen Salze, Tautomere und Stereoisomere zur Verwendung für die Behandlung von Tumoren, wobei eine therapeutisch wirksame Menge einer Verbindung gemäß Anspruch 1 in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weitere Angiogenese-Hemmer verabreicht wird.

5. Verbindungen gemäß Anspruch 1 und/oder ihre physiologisch unbedenklichen Salze, Tautomere und Stereoisomere zur Verwendung für die Behandlung von Tumoren, wobei eine therapeutisch wirksame Menge einer Verbindung gemäß Anspruch 1 in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weitere Angiogenese-Hemmer verabreicht wird.

## Claims

1. Compounds selected from the group
| Compound No. | Name |
|---|---|
| "A4" | 5-[5-(1-Methylpyrazol-4-yl)-1H-pyrrolo[2,3-b]pyridin-3-yl]-2-tetrahydropyran-4-yloxybenzonitrile |
| "A6" | 5-[5-(4-Cyanophenyl)-1H-pyrrolo[2,3-b]pyridin-3-yl]-2-tetrahydropyran-4-yloxybenzonitrile |
| "A7" | 5-[5-[1-(2-Morpholinoethyl)pyrazol-4-yl]-1H-pyrrolo[2,3-b]pyridin-3-yl]-2-tetrahydropyran-4-yloxybenzonitrile |
| "A8" | 5-[5-(4-Morpholinophenyl)-1H-pyrrolo[2,3-b]pyridin-3-yl]-2-tetrahydropyran-4-yloxybenzonitrile |
| "A9" | 5-[5-[1-(2-Pyrrolidin-1-ylethyl)pyrazol-4-yl]-1H-pyrrolo[2,3-b]pyridin-3-yl]-2-tetrahydropyran-4-yloxybenzonitrile |
| "A10" | 5-[5-[1-(2-Methoxyethyl)pyrazol-4-yl]-1H-pyrrolo[2,3-b]pyridin-3-yl]-2-tetrahydropyran-4-yloxybenzonitrile |
| "A11" | 5-[2-Methyl-5-(1-methylpyrazol-4-yl)-1H-pyrrolo[2,3-b]pyridin-3-yl]-2-tetrahydropyran-4-yloxybenzonitrile |
| "A12" | 5-[5-(1-Piperidin-4-yl-1H-pyrazol-4-yl)-1H-pyrrolo[2,3-b]pyridin-3-yl]-2-(tetrahydropyran-4-yloxy)benzonitrile |
| "A 13" | 2-(1-Methy)piperidin-4-y)oxy)-5-[5-(1-methyl-1H-pyrazol-4-yl)-1H-pyrrolo[2,3-b]pyridin-3-yl]benzonitrile |
| "A14" | 5-(5-Bromo-1H-pyrrolo[2,3-b]pyridin-3-yl)-2-(cyclopropylmethoxy)benzonitrile |
| "A 15" | 5-[5-[1-(2-Hydroxyethyl)pyrazol-4-yl]-1H-pyrrolo[2,3-b]pyridin-3-yl]-2-tetrahydropyran-4-yloxybenzonitrile |
| "A16" | 5-[5-[1-(1-Methyl-4-piperidyl)pyrazol-4-yl]-1H-pyrrolo[2,3-b]pyridin-3-yl]-2-tetrahydropyran-4-yloxybenzonitrile |
| "A17" | 5-[5-(4-Methylpiperazin-1-yl)-1H-pyrrolo[2,3-b]pyridin-3-yl]-2-tetrahydropyran-4-yloxybenzonitrile |
| "A18" | 5-[5-[1-(4-Piperidyl)pyrazol-4-yl]-1H-pyrrolo[2,3-b]pyridin-3-yl]-2-tetrahydropyran-4-yloxypyridine-3-carbonitrile |
| "A19" | 5-[5-[1-[1-(Benzenesulfonyl)-4-piperidyl]pyrazol-4-yl]-1H-pyrrolo[2,3-b]pyridin-3-yl]-2-tetrahydropyran-4-yloxypyridine-3-carbonitrile |
| "A20" | 5-[5-[1-(1-Methyl-4-piperidyl)pyrazol-4-yl]-1H-pyrrolo[2,3-b]pyridin-3-yl]-2-tetrahydropyran-4-yloxybenzonitrile |
| "A21" | 5-[5-(2-Morpholinoethoxy)-1H-pyrrolo[2,3-b]pyridin-3-yl]-2-tetrahydropyran-4-yloxybenzonitrile |
| "A22" | 5-[5-[1-(4-Piperidyl)triazol-4-yl]-1H-pyrrolo[2,3-b]pyridin-3-yl]-2-tetrahydropyran-4-yloxybenzonitrile |
| "A23" | 5-[5-[1-(1-Methyl-4-piperidyl)triazol-4-yl]-1H-pyrrolo[2,3-b]pyridin-3-yl]-2-tetrahydropyran-4-yloxybenzonitrile |
| "A24" | 2-[(3-Methyloxetan-3-yl)methoxy]-5-[5-[1-(4-piperidyl)pyrazol-4-yl]-1H-pyrrolo[2,3-b]pyridin-3-yl]benzonitrile |
| "A25" | 2-[(3-Methyloxetan-3-yl)methoxy]-5-[5-[1-(1-methyl-4-piperidyl)pyrazol-4-yl]-1H-pyrrolo[2,3-b]pyridin-3-yl]benzonitrile |
| "A26" | 5-[5-[1-(Oxetan-3-yl)pyrazol-4-yl]-1H-pyrrolo[2,3-b]pyridin-3-yl]-2-tetrahydropyran-4-yloxybenzonitrile |
| "A27" | 5-[5-[1-(2-Pyrrolidin-1-ylethyl)triazol-4-yl]-1H-pyrrolo[2,3-b]pyridin-3-yl]-2-tetrahydropyran-4-yloxybenzonitrile |
| "A28" | 2-(Oxetan-3-yloxy)-5-[5-[1-(4-piperidyl)pyrazol-4-yl]-1H-pyrrolo[2,3-b]pyridin-3-yl]benzonitrile |
| "A29" | 5-[5-[1-(1-Methyl-4-piperidyl)pyrazol-4-yl]-1H-pyrrolo[2,3-b]pyridin-3-yl]-2-(oxetan-3-yloxy)benzonitrile |
| "A30" | 5-[5-(1-Methylpyrazol-4-yl)-1H-pyrrolo[2,3-b]pyrid in-3-yl]-2-tetrahydropyran-4-yloxypyridine-3-carbonitrile |
| "A31" | 5-[5-(1-Methyl-1H-pyrazol-4-yl)-1H-pyrrolo[2,3-b]pyridin-3-yl]-2-(oxetan-3-yloxy)benzonitrile |
| "A32" | 2-(3-Methyloxetan-3-ylmethoxy)-5-[5-(1-methyl-1H-pyrazol-4-yl)-1H-pyrrolo[2,3-b]pyridin-3-yl]benzonitrile |
| "A33" | 5-{5-[1-(2-Pyrrolidin-1-ylethyl)-1H-pyrazol-4-yl]-1H-pyrrolo-[2,3-b]pyridin-3-yl}-2-(tetrahydropyran-4-yloxy)nicotinonitrile |
| "A34" | 2-(Oxetan-3-yloxy)-5-[5-(1-oxetan-3-yl-1H-pyrazol-4-yl)-1H-pyrrolo[2,3-b]pyridin-3-yl]benzonitrile |
| "A35" | 2-(3-Methyloxetan-3-ylmethoxy)-5-[5-(1-oxetan-3-yl-1H-pyrazol-4-yl)-1H-pyrrolo[2,3-b]pyridin-3-yl]benzonitrile |
| "A36" | 2-(3-Methyloxetan-3-ylmethoxy)-5-[5-(1H-pyrazol-4-yl)-1H-pyrrolo[2,3-b]pyridin-3-yl]benzonitrile |
| "A37" | 5-[5-(1-Oxetan-3-yl-1H-pyrazol-4-yl)-1H-pyrrolo[2,3-b]pyridin-3-yl]-2-(tetrahydropyran-4-yloxy)nicotinonitrile |
| "A38" | 5-{5-[1-(2-Methoxyethyl)-1H-pyrazol-4-yl]-1H-pyrrolo-[2,3-b]pyridin-3-yl}-2-(tetrahydropyran-4-yloxy)nicotinonitrile |
and pharmaceutically usable salts, tautomers and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Compounds according to Claim 1 and pharmaceutically usable salts, tautomers and stereoisomers thereof, including mixtures thereof in all ratios, for use for the treatment of cancer, septic shock, primary open angle glaucoma (POAG), hyperplasia, atherosclerosis, retinopathy, osteoarthritis, endometriosis, chronic inflammation, neurodegenerative diseases, rheumatoid arthritis (RA), systemic lupus erythematosus (SLE), Sjörgren's syndrome, Aicardi-Goutières syndrome, chilblain lupus, retinal vasculopathy with cerebral leukodystrophy (RVCL), systemic sclerosis, myositis, psoriasis, chronic obstructive pulmonary disease (CPD), inflammatory bowel disease (IBD), obesity, insulin resistance, type 2 diabetes (NIDDM) and/or metabolic syndrome.

4. Compounds according to Claim 1 and/or physiologically acceptable salts, tautomers and stereoisomers thereof for use for the treatment of tumours, where a therapeutically effective amount of a compound according to Claim 1 is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

5. Compounds according to Claim 1 and/or physiologically acceptable salts, tautomers and stereoisomers thereof for use for the treatment of tumours, where a therapeutically effective amount of a compound according to Claim 1 is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

1. Composés choisis dans le groupe constitué par
| Composé n° | Nom |
|---|---|
| « A4 » | 5-[5-(1-Méthylpyrazol-4-yl)-1H-pyrrolo[2,3-b]pyridin-3-yl]-2-tétrahydropyran-4-yloxybenzonitrile |
| « A6 » | 5-[5-(4-Cyanophényl)-1H-pyrrolo[2,3-b]pyridin-3-yl]-2-tétrahydropyran-4-yloxybenzonitrile |
| « A7 » | 5-[5-[1-(2-Morpholinoéthyl)pyrazol-4-yl]-1H-pyrrolo[2,3-b]pyridin-3-yl]-2-tétrahydropyran-4-yloxybenzonitrile |
| « A8 » | 5-[5-(4-Morpholinophényl)-1H-pyrrolo[2,3-b]pyridin-3-yl]-2-tétrahydropyran-4-yloxybenzonitrile |
| « A9 » | 5-[5-[1-(2-Pyrrolidin-1-yléthyl)pyrazol-4-yl]-1H-pyrrolo-[2,3-b]pyridin-3-yl]-2-tétrahydropyran-4-yloxybenzonitrile |
| « A10 » | 5-[5-[1-(2-Méthoxyéthyl)pyrazol-4-yl]-1H-pyrrolo[2,3-b]pyridin-3-yl]-2-tétrahydropyran-4-yloxybenzonitrile |
| « A11 » | 5-[2-Méthyl-5-(1-méthylpyrazol-4-yl)-1H-pyrrolo[2,3-b]pyridin-3-yl]-2-tétrahydropyran-4-yloxybenzonitrile |
| « A12 » | 5-[5-(1-Pipéridin-4-yl-1H-pyrazol-4-yl)-1H-pyrrolo[2,3-b]pyridin-3-yl]-2-(tétrahydropyran-4-yloxy)benzonitrile |
| « A13 » | 2-(1-Méthylpipéridin-4-yloxy)-5-[5-(1-méthyl-1H-pyrazol-4-yl)-1H-pyrrolo[2,3-b]pyridin-3-yl]benzonitrile |
| « A14 » | 5-(5-Bromo-1H-pyrrolo[2,3-b]pyridin-3-yl)-2-(cyclopropyl-méthoxy)benzonitrile |
| « A15 » | 5-[5-[1-(2-Hydroxyéthyl)pyrazol-4-yl]-1H-pyrrolo[2,3-b]pyridin-3-yl]-2-tétrahydropyran-4-yloxybenzonitrile |
| « A16 » | 5-[5-[1-(1-Méthyl-4-pipéridyl)pyrazol-4-yl]-1H-pyrrolo-[2,3-b]pyridin-3-yl]-2-tétrahydropyran-4-yloxybenzonitrile |
| « A17 » | 5-[5-(4-Méthylpipérazin-1-yl)-1H-pyrrolo[2,3-b]pyridin-3-yl]-2-tétrahydropyran-4-yloxybenzonitrile |
| « A18 » | 5-[5-[1-(4-Pipéridyl)pyrazol-4-yl]-1H-pyrrolo[2,3-b]pyridin-3-yl]-2-tétrahydropyran-4-yloxypyridine-3-carbonitrile |
| « A19 » | 5-[5-[1-[1-(Benzènesulfonyl)-4-pipéridyl]pyrazol-4-yl]-1H-pyrrolo[2,3-b]pyridin-3-yl]-2-tétrahydropyran-4-yloxypyridine-3-carbonitrile |
| « A20 » | 5-[5-[1-(1-Méthyl-4-pipéridyl)pyrazol-4-yl]-1H-pyrrolo-[2,3-b]pyridin-3-yl]-2-tétrahydropyran-4-yloxybenzonitrile |
| « A21 » | 5-[5-(2-Morpholinoéthoxy)-1H-pyrrolo[2,3-b]pyridin-3-yl]-2-tétrahydropyran-4-yloxybenzonitrile |
| « A22 » | 5-[5-[1-(4-Pipéridyl)triazol-4-yl]-1H-pyrrolo[2,3-b]pyridin-3-yl]-2-tétrahydropyran-4-yloxybenzonitrile |
| « A23 » | 5-[5-[1-(1-Méthyl-4-pipéridyl)triazol-4-yl]-1H-pyrrolo-[2,3-b]pyridin-3-yl]-2-tétrahydropyran-4-yloxybenzonitrile |
| « A24 » | 2-[(3-Méthyloxétan-3-yl)méthoxy]-5-[5-[1-(4-pipéridyl)pyrazol-4-yl]-1H-pyrrolo[2,3-b]pyridin-3-yl]benzonitrile |
| « A25 » | 2-[(3-Méthyloxétan-3-yl)méthoxy]-5-[5-[1-(1-méthyl-4-pipéridyl)pyrazol-4-yl]-1H-pyrrolo[2,3-b]pyridin-3-yl]benzonitrile |
| « A26 » | 5-[5-[1-(Oxétan-3-yl)pyrazol-4-yl]-1H-pyrrolo[2,3-b]pyridin-3-yl]-2-tétrahydropyran-4-yloxybenzonitrile |
| « A27 » | 5-[5-[1-(2-Pyrrolidin-1-yléthyl)triazol-4-yl]-1H-pyrrolo-[2,3-b]pyridin-3-yl]-2-tétrahydropyran-4-yloxybenzonitrile |
| « A28 » | 2-(Oxétan-3-yloxy)-5-[5-[1-(4-pipéridyl)pyrazol-4-yl]-1H-pyrrolo[2,3-b]pyridin-3-yl]benzonitrile |
| « A29 » | 5-[5-[1-(1-Méthyl-4-pipéridyl)pyrazol-4-yl]-1H-pyrrolo-[2,3-b]pyridin-3-yl]-2-(oxétan-3-yloxy)benzonitrile |
| « A30 » | 5-[5-(1-Méthylpyrazol-4-yl)-1H-pyrrolo[2,3-b]pyridin-3-yl]-2-tétrahydropyran-4-yloxypyridine-3-carbonitrile |
| « A31 » | 5-[5-(1-Méthyl-1H-pyrazol-4-yl)-1H-pyrrolo[2,3-b]pyridin-3-yl]-2-(oxétan-3-yloxy)benzonitrile |
| « A32 » | 2-(3-Méthyloxétan-3-ylméthoxy)-5-[5-(1-méthyl-1H-pyrazol-4-yl)-1H-pyrrolo[2,3-b]pyridin-3-yl]benzonitrile |
| « A33 » | 5-{5-[1-(2-Pyrrolidin-1-yléthyl)-1H-pyrazol-4-yl]-1H-pyrrolo-[2,3-b]pyridin-3-yl}-2-(tétrahydropyran-4-yloxy)nicotinonitrile |
| « A34 » | 2-(Oxétan-3-yloxy)-5-[5-(1-oxétan-3-yl-1H-pyrazol-4-yl)-1H-pyrrolo[2,3-b]pyridin-3-yl]benzonitrile |
| « A35 » | 2-(3-Méthyloxétan-3-ylméthoxy)-5-[5-(1-oxétan-3-yl-1H-pyrazol-4-yl)-1H-pyrrolo[2,3-b]pyridin-3-yl]benzonitrile |
| « A36 » | 2-(3-Méthyloxétan-3-ylméthoxy)-5-[5-(1H-pyrazol-4-yl)-1H-pyrrolo[2,3-b]pyridin-3-yl]benzonitrile |
| « A37 » | 5-[5-(1-Oxétan-3-yl-1H-pyrazol-4-yl)-1H-pyrrolo[2,3-b]pyridin-3-yl]-2-(tétrahydropyran-4-yloxy)nicotinonitrile |
| « A38 » | 5-{5-[1-(2-Méthoxyéthyl)-1H-pyrazol-4-yl]-1H-pyrrolo-[2,3-b]pyridin-3-yl}-2-(tétrahydropyran-4-yloxy)nicotinonitrile |
ainsi que les sels, tautomères et stéréoisomères pharmaceutiquement utilisables de ceux-ci, y compris des mélanges de ceux-ci en toutes proportions.

2. Médicaments comprenant au moins un composé selon la revendication 1 et/ou des sels, tautomères et stéréoisomères pharmaceutiquement utilisables de celui-ci, y compris des mélanges de ceux-ci en toutes proportions, et éventuellement des excipients et/ou des adjuvants.

3. Composés selon la revendication 1 et des sels, tautomères et stéréoisomères pharmaceutiquement utilisables de ceux-ci, y compris des mélanges de ceux-ci en toutes proportions, pour une utilisation dans le traitement du cancer, du choc septique, du glaucome primitif à angle ouvert (POAG), de l'hyperplasie, de l'athérosclérose, de la rétinopathie, de l'arthrose, de l'endométriose, des inflammations chroniques, des maladies neurodégénératives, de la polyarthrite rhumatoïde (« RA »), du lupus érythémateux disséminé (« SLE »), du syndrome de Sjögren, du syndrome d'Aicardi-Goutières, du lupus engelure, de la vasculo-pathie rétinienne avec leucodystrophie cérébrale (« RVCL »), de la sclérodermie généralisée, de la myosite, du psoriasis, de la bronchopneumopathie chronique obstructive (BPCO), de l'affection abdominale inflammatoire (« IBD »), de l'obésité, de l'insulinorésistance, du diabète de type 2 (DNID) et/ou du syndrome métabolique.

4. Composés selon la revendication 1 et/ou des sels, tautomères et stéréoisomères physiologiquement acceptables de ceux-ci pour une utilisation dans le traitement de tumeurs, où une quantité thérapeutiquement efficace d'un composé selon la revendication 1 est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

5. Composés selon la revendication 1 et/ou des sels, tautomères et stéréoisomères physiologiquement acceptables de ceux-ci pour une utilisation dans le traitement de tumeurs, où une quantité thérapeutiquement efficace d'un composé selon la revendication 1 est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.


### SEQUENCE LISTING

<110> Merck Patent GmbH
<120> 3-cyanaryl-1H-pyrrolo[2,3-b]pyridin-Derivate
<130> P 11/176-ve/ik
<140> 1020111191279
   <141> 2011-11-22
<160> 2
<170> PatentIn version 3.5
<210> 1
   <211> 23
   <212> PRT
   <213> Artificial Sequence
<220>
   <223> Biotin-Ah-Ah-
<400> 1
   Ala Lys Pro Lys Gly Asn Lys Asp Tyr His Leu Gln Thr Cys Cys Gly
   1               5                   10                  15
   Ser Leu Ala Tyr Arg Arg Arg
               20
<210> 2
   <211> 23
   <212> PRT
   <213> Artificial Sequence
<220>
   <223> Biotin-C6-C6-
<400> 2
   Gly Leu Lys Lys Glu Arg Leu Leu Asp Asp Arg His Asp Ser Gly Leu
   1               5                   10                  15
   Asp Ser Met Lys Asp Glu Glu
               20